(19) Europäisches Patentamt
European Patent Office
Office européen des brevets

(11) **EP 4 401 759 B1**

(12) **EUROPEAN PATENT SPECIFICATION**

(45) Date of publication and mention
of the grant of the patent:
**18.02.2026   Bulletin 2026/08**

(21) Application number: **22777691.1**

(22) Date of filing: **16.09.2022**

(51) International Patent Classification (IPC):
*A61K 38/48* (2006.01)     *C07K 14/33* (2006.01)
*A61P 7/02* (2006.01)

(52) Cooperative Patent Classification (CPC):
**A61K 38/48; A61P 7/02; C07K 14/33**

(86) International application number:
**PCT/GB2022/052361**

(87) International publication number:
**WO 2023/041934 (23.03.2023 Gazette 2023/12)**

(54) **MODIFIED BONT/A FOR USE IN THE TREATMENT OF CERVICAL DYSTONIA**

MODIFIZIERTES BONT/A ZUR VERWENDUNG BEI DER BEHANDLUNG VON ZERVIXDYSTONIE

BONT/A MODIFIÉE DESTINÉE À ÊTRE UTILISÉE DANS LE TRAITEMENT DE LA DYSTONIE CERVICALE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **16.09.2021   GB 202113262**
**29.04.2022   GB 202206357**

(43) Date of publication of application:
**24.07.2024   Bulletin 2024/30**

(73) Proprietor: **Ipsen Biopharm Limited**
**Wrexham LL13 9UF (GB)**

(72) Inventors:
• **GRIGORE, Nicolae**
**Wrexham Industrial Estate,**
**Wrexham LL13 9UF (GB)**
• **PONS, Laurent**
**Wrexham Industrial Estate,**
**Wrexham LL13 9UF (GB)**

(74) Representative: **Mathys & Squire**
**The Shard**
**32 London Bridge Street**
**London SE1 9SG (GB)**

(56) References cited:
**WO-A1-2015/004461     WO-A1-2017/191315**

• **CHOUDHURY SUPRIYO ET AL: "Botulinum Toxin: An Update on Pharmacology and Newer Products in Development", vol. 13, no. 1, 14 January 2021 (2021-01-14), pages 58, XP055916832, Retrieved from the Internet <URL:https://www.ncbi.nlm.nih.gov/pmc/articles/PMC7828686/pdf/toxins-13-00058.pdf> DOI: 10.3390/toxins13010058**

Remarks:
The complete document including Reference Table(s) and the Sequence Listing(s) can be downloaded from the EPO website

EP 4 401 759 B1

## Description

### FIELD OF THE INVENTION

The present invention relates to treatment of cervical dystonia as claimed.

### BACKGROUND

Cervical dystonia (also known as spasmodic torticollis) is a chronic neurological movement disorder typically associated with extreme pain. The disorder causes the neck of an affected subject to involuntarily turn to the left, right, upwards, and/or downwards. Both agonist and antagonist muscles may contract simultaneously during dystonic movement.

The disorder typically presents with relatively mild symptoms, such as an invisible tremor of the head for a few months at onset. Other early/progressive symptoms may include the head turning, pulling, and/or tilting in sudden movements. Yet further early/progressive symptoms typically include sustained/prolonged involuntary head positioning. Involuntary neck muscle spasms tend to increase in frequency and strength over time prior to reaching a plateau. Subjects with cervical dystonia may also experience muscle hypertrophy, neck pain, dysarthria, and/or tremor.

The symptoms of cervical dystonia may involve any neck muscles of a subject and the head posture can vary. Typically the most common abnormal posture associated with cervical dystonia is the twisting of the chin toward a shoulder so that the head rotates sideways (torticollis). Other abnormal postures associated with cervical dystonia may include anterocollis, where the head is tipped forward, retrocollis, where the head is tilted backwards, or laterocollis, where the head is tilted toward one side. There can also be shifting of the head on the shoulders in an anterior sagittal shift (a forward shift) or posterior sagittal shift (a backwards shift). However, most commonly, cervical dystonia presents with complex symptoms in which a subject exhibits several angles of head movement.

Current treatment options include oral medications (e.g. dopamine blocking agents), deep brain stimulation, botulinum neurotoxins, and selective surgical denervation of nerves triggering muscle contractions. Conventional oral medications are associated with a number of severe side-effects, while deep brain stimulation and surgical denervation are invasive, have associated risks of complications, and/or can be ineffective.

An example of a conventional botulinum neurotoxin serotype A (BoNT/A) treatment for cervical dystonia is Dysport®, which is a medicinal product containing drug substance BoNT/A haemagglutinin complex (BTX-A-HAC) isolated and purified from *Clostridium botulinum* type A strain. Several other medicinal BoNT/A products naturally produced by *Clostridium botulinum* are also on the market (e.g. BOTOX® and XEOMIN®).

By paralysing a dystonic antagonist muscle, BoNT/A may allow the agonist muscle to move freely. In more detail, BoNT/A selectively inhibits the release of acetylcholine from the presynaptic nerve terminals and thus blocks cholinergic transmission at the neuromuscular junction inducing a reduction in the muscle contraction and muscle tone, causing the injected muscles to relax. However, the duration of action of the currently available BoNT/A products is about 12 to 14 weeks, which is when the new nerve endings sprout allowing the nerve function to return to normal, and the original symptoms reappear. Consequently, for the effect to be maintained, injections need to be repeated periodically. Thus, the frequency of BoNT/A injections is an important consideration for the treatment of cervical dystonia, considering the chronicity of the condition and long-term nature of the treatment required. Indeed, it has an impact on the direct and indirect health costs involved for the patients and caregivers, the logistics for injections within the hospitals/clinics, and, most importantly, the quality of life of patients.

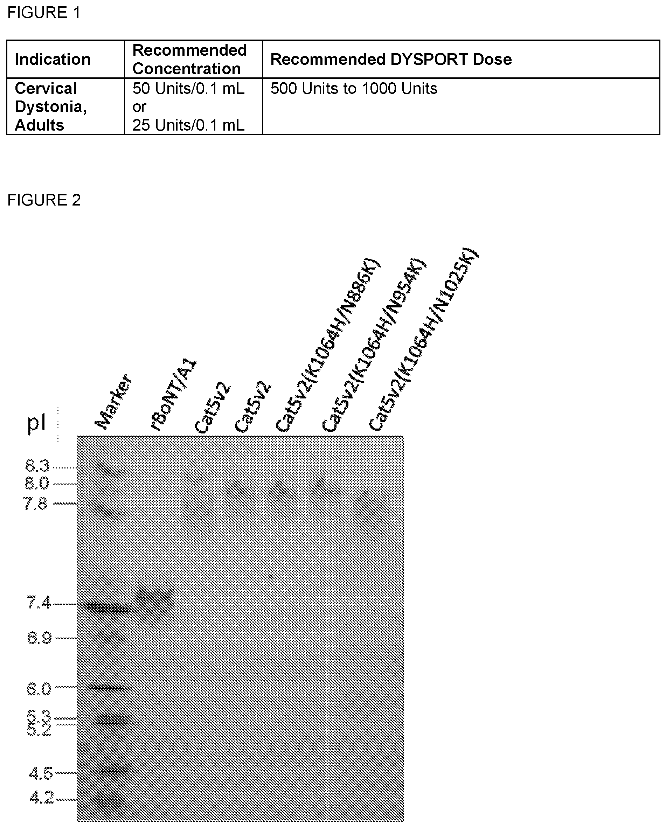

Dysport® is approved for the treatment of cervical dystonia with a maximum total dose per treatment session of 1,000 Units (see Figure 1). A clinician is required to administer Dysport® to neck muscles of the subject up to the upper threshold of 1,000 Units total per treatment session. The clinician is forced to make difficult choices during treatment of a patient. In other words, in conventional treatment regimens, a clinician must find a balance between the relatively low total amount of BoNT/A that can be administered (1,000 Units - necessitated by the highly toxic nature of BoNT/A) and the effective amount at a plurality of different muscles. Hence, certain muscles are neglected while others receive a suboptimal amount of BoNT/A, resulting in suboptimal therapy.

Moreover, the conventional cervical dystonia treatment regimens are complicated and result in clinicians under-dosing in an effort to avoid toxicity to the patient. There is thus a need for a convenient, safe, and effective single dose unit and a corresponding guide to the number of units that can be administered to an affected neck muscle (e.g. including the number of injection sites per muscle) in a treatment session without resultant patient toxicity.

WO 2017/191315 describes chimeric neurotoxins with enhanced properties and their use in therapy.

WO2015/004461 describes engineered clostridial toxins comprising at least one amino acid modification, and the use of such engineered clostridial toxins in medicine and therapy.

In conclusion, there is a need for an improved treatment for cervical dystonia that would allow an individualised patient-centric approach to tailor the treatment according to the targeted clinical pattern permitting different combinations of affected neck muscles to be injected depending on the distribution, extent and severity of the cervical dystonia, while avoiding toxicity and providing a longer-lasting treatment (resulting in less frequent administration).

The present invention overcomes one or more of the above-mentioned problems.

## SUMMARY OF THE INVENTION

The present invention is defined by the claims.

Accordingly, in one aspect the invention provides a modified botulinum neurotoxin A (BoNT/A) for use in treating cervical dystonia, wherein the modified BoNT/A is administered by intramuscular injection to an affected neck muscle of a subject,

wherein the modified BoNT/A is administered by way of a unit dose of 750 pg to 17,000 pg of modified BoNT/A,
wherein at least a single unit dose is administered to the affected neck muscle,
wherein the total dose administered during the treatment is up to 170,000 pg of modified BoNT/A, and
wherein the modified BoNT/A comprises a BoNT/A light-chain and translocation domain, and a BoNT/B receptor binding domain ($H_C$ domain).

In another aspect, the invention provides a unit dosage form of modified BoNT/A for use in treating cervical dystonia, the unit dosage form comprising:

(a) 750 pg to 17,000 pg of modified BoNT/A; and
(b) optionally a pharmaceutically acceptable carrier, excipient, adjuvant, and/or salt,

wherein the modified BoNT/A comprises a BoNT/A light-chain and translocation domain, and a BoNT/B receptor binding domain ($H_C$ domain).

The present inventors have surprisingly found that a modified BoNT/A finds particular utility in treating cervical dystonia. The modified BoNT/A may comprise a BoNT/A light-chain and translocation domain and a BoNT/B receptor binding domain ($H_c$ domain), which results in a modified BoNT/A that exhibits increased retention at (reduced diffusion away from) a site of administration and/or increased duration of action (e.g. 6-9 months). Alternatively, the modified BoNT/A may comprise one or more modifications of surface exposed amino acid residues resulting in an increased net positive charge. The increased charge promotes electrostatic interactions between the polypeptide and anionic extracellular components, thereby promoting binding between the polypeptide and cell surface. In turn this also increases retention at (reduces diffusion away from) a site of administration and/or results in an increased duration of action (e.g. 6-9 months).

Advantageously, modified BoNT/A has a safety profile that is improved when compared to unmodified BoNT/A (e.g. Dysport®). This improved safety profile may be expressed by the high Safety Ratio described herein for the modified BoNT/A.

Based on the pre-clinical and clinical data herein (see Examples) it has been shown that a higher total amount of modified BoNT/A can be administered to a subject while achieving a similar safety profile to unmodified BoNT/A (e.g. Dysport®) while at such high doses. Thus, more modified BoNT/A can be injected and/or can be injected at a greater number of neck muscles/sites in the treatment of cervical dystonia before reaching the maximum total dose. This is a significant and advantageous finding, and yields an improved treatment of cervical dystonia while providing clinicians with a greater range of treatment options. The treatment may be improved in that it provides for longer-lasting treatment (resulting in less frequent administration) and/or is capable of being tailored for the subject and/or results in an improved quality of life of a subject when compared to treatment with unmodified BoNT/A (e.g. Dysport®). Hence, the treatment of the invention is improved compared to conventional treatment regimens.

Moreover, the present invention provides a convenient, safe, and effective single unit dose as well as a total (maximum) dosage that can be safely administered in a single treatment. The present invention also provides a corresponding guide to the number of times at which said unit dose can be administered to a neck muscle (e.g. including the number of injection sites per muscle) without resultant patient toxicity. Treatment of cervical dystonia in accordance with the present invention is thus much less complicated for the clinician and helps avoid under-dosing and/or over-dosing. Furthermore, treatment according to the invention is much more satisfactory to the patient, as it is better tailored to the patient's needs, when compared to conventional cervical dystonia treatments.

## DETAILED DESCRIPTION

In one aspect, the invention provides a modified botulinum neurotoxin A (BoNT/A) for use in treating cervical dystonia, wherein the modified BoNT/A is administered by intramuscular injection to an affected neck muscle of a subject,

wherein the modified BoNT/A is administered by way of a unit dose of 750 pg to 17,000 pg of modified BoNT/A, wherein at least a single unit dose is administered to the affected neck muscle, wherein the total dose administered during the treatment is up to 170,000 pg of modified BoNT/A, and wherein the modified BoNT/A comprises a BoNT/A light-chain and translocation domain, and a BoNT/B receptor binding domain ($H_c$ domain).

In a related aspect, the invention provides a modified BoNT/A for use in treating cervical dystonia of a subject for a longer duration than that treated by an unmodified BoNT/A (e.g. SEQ ID NO: 2), wherein the modified BoNT/A is administered by intramuscular injection to an affected neck muscle of the subject,

wherein the modified BoNT/A is administered by way of a unit dose of 750 pg to 17,000 pg of modified BoNT/A, wherein at least a single unit dose is administered to the affected neck muscle, wherein the total dose administered during the treatment is up to 170,000 pg of modified BoNT/A, wherein the modified BoNT/A comprises a BoNT/A light-chain and translocation domain, and a BoNT/B receptor binding domain ($H_c$ domain).

The term "treating cervical dystonia of a subject for a longer duration than that treated by an unmodified BoNT/A" may mean that one or more symptoms of cervical dystonia of the subject are reduced for a longer time period following administration of a modified BoNT/A of the invention, when compared to administration of an unmodified BoNT/A. Said duration of action may be at least 1.25x, 1.5x, 1.75x, 2.0x, or 2.25x greater. The duration of action of modified BoNT/A may be between 6 and 9 months. For example, a duration of action may be at least: 4.5 months (from onset), 5.0 months, 5.5 months, 6 months, 6.5 months, 7.0 months, 7.5 months, 8.0 months, 8.5 months or 9.0 months. In particular embodiments, a duration of action may be greater than 9.0 months. Said reduction may be determined by comparison to an equivalent control subject exhibiting equivalent symptoms that has been treated with an unmodified BoNT/A. At a time period where the severity of one or more symptoms of the control subject are substantially the same (e.g. the same) as before unmodified BoNT/A treatment, a subject treated with a modified BoNT/A according to the invention may exhibit an improvement in the equivalent one or more symptoms of at least 5%, 10%, 25%, or 50% when compared to the severity of the one or more symptoms before treatment with the modified BoNT/A. The unmodified BoNT/A is preferably SEQ ID NO: 2 present in a di-chain form.

The unit dose may be 750 pg to 17,000 pg of modified BoNT/A, wherein the modified BoNT/A comprises a BoNT/A light-chain and translocation domain, and a BoNT/B receptor binding domain ($H_c$ domain). An upper limit of the unit dose range may be 16,500, 15,500, 14,500, 13,500, 12,500, 11,500, 10,500, 9,500, 8,500, 7,500, 6,500, 5,500, 4,500, 3,500, 2,500, 2,250, 2,000, 1,500, 1,250, 1,000, 750 or 500 pg of modified BoNT/A, preferably the upper limit is 16,000 pg. A lower limit of the unit dose range may be 800, 850, 950, 1,000, 1,500, 1,750, 2,000, 2,500, 3,000, 3,500, 4,000, 4,500 or 5,000 pg of modified BoNT/A, preferably the lower limit is 1,000 pg. Preferably, the unit dose of modified BoNT/A is 1,000 pg to 16,000 pg of modified BoNT/A, e.g. 950 pg to 1,250 pg, 1,750 pg to 2,250 pg, or 8,000 pg to 12,000 pg. Preferably, a unit dose of modified BoNT/A may be 1,000, 2,000, 3,000, 8,000 or 16,000 pg.

A total dose administered when carrying out the treatment regimen of the present invention may be up to 170,000 pg of modified BoNT/A, wherein the modified BoNT/A comprises a BoNT/A light-chain and translocation domain, and a BoNT/B receptor binding domain ($H_C$ domain). In other words, the total amount of modified BoNT/A administered at a given treatment session may be up to 170,000 pg. The total dose may be up to 165,000, 160,000, 140,000, 110,000, 100,000, 90,000, 80,000, 70,000, 60,000, 50,000, 40,000, 30,000, 28,000, 25,000, 20,000, 15,000, 14,000, 10,000, 8,000, 7,000, or 5,000 pg. Preferably, the total dose may be up to 160,000 pg of modified BoNT/A, e.g. the total dose may be up to 7,000, 10,000, 14,000, 20,000, 28,000, 30,000, 80,000 or 160,000 pg. The total dose may be at least 900, 1,000, 2,000, 3,000, 4,000, 5,000, 6,000, 7,000, 7,500, 10,000, 12,500, 13,000, 14,000, 15,000, 20,000, 25,000, 30,000, 40,000, 50,000, 60,000, 70,000, 80,000, 90,000, 100,000, 120,000 or 150,000 pg. Preferably, the total dose may be at least 1,500 pg, more preferably at least 2,000 pg of modified BoNT/A, e.g. at least 5,000 pg. The total dose may be 5,250 pg to 170,000 pg, preferably 7,000 pg to 160,000 pg. For example, the total dose administered may be 6,000-30,000 pg, such as 6,000-15,000 pg or 13,000-29,000 pg. More preferably, the total dose administered is 10,000 pg to 160,000 pg. Preferably, the total dose administered may be 7,000, 10,000, 14,000, 20,000, 28,000, 30,000, 80,000 or 160,000 pg.

Accordingly, the unit dose may be 750 pg to 17,000 pg of modified BoNT/A, wherein the modified BoNT/A comprises a BoNT/A light-chain and translocation domain, and a BoNT/B receptor binding domain ($H_c$ domain), and the total dose administered when carrying out the treatment regimen of the present invention may be up to 170,000 pg. The unit dose may be 1,000 pg and the total dose may be up to 7,000 pg. The unit dose may be 1,000 pg and the total dose may be up to 10,000 pg. The unit dose may be 1,000 pg and the total dose may be up to 14,000 pg. The unit dose may be 2,000 pg and the total dose may be up to 14,000 pg. The unit dose may be 2,000 pg and the total dose may be up to 20,000 pg. The unit dose may be 2,000 pg and the total dose may be up to 28,000 pg. The unit dose may be 3,000 pg and the total dose may be up to 30,000 pg. The unit dose may be 8,000 pg and the total dose may be up to 80,000 pg. The unit dose may be 16,000 pg and the total dose may be up to 160,000 pg.

Accordingly, the unit dose may be 750 pg to 17,000 pg of modified BoNT/A, wherein the modified BoNT/A comprises a BoNT/A light-chain and translocation domain, and a BoNT/B **receptor** binding domain ($H_C$ domain), and the total dose administered when carrying out the treatment regimen of the present invention may be up to 170,000 pg. The unit dose may be 1,000 pg and the total dose may be 7,000 pg. The unit dose may be 1,000 pg and the total dose may be 10,000 pg. The unit dose may be 1,000 pg and the total dose may be 14,000 pg. The unit dose may be 2,000 pg and the total dose may be 14,000 pg. The unit dose may be 2,000 pg and the total dose may be 20,000 pg. The unit dose may be 2,000 pg and the total dose may be 28,000 pg. The unit dose may be 3,000 pg and the total dose may be 30,000 pg. The unit dose may be 8,000 pg and the total dose may be 80,000 pg. The unit dose may be 16,000 pg and the total dose may be 160,000 pg.

Described herein is a modified botulinum neurotoxin A (BoNT/A) for use in treating cervical dystonia, wherein the modified BoNT/A is administered by intramuscular injection to an affected neck muscle of a subject,

wherein the modified BoNT/A is administered by way of a unit dose of 31 Units (U) to 707 U of modified BoNT/A, wherein 1 Unit is an amount of the modified BoNT/A that corresponds to the calculated median lethal dose ($LD_{50}$) in mice,
wherein at least a single unit dose is administered to the affected neck muscle,
wherein the total dose administered during the treatment is up to 7,070 U of modified BoNT/A, and
wherein the modified BoNT/A comprises a BoNT/A light-chain and translocation domain, and a BoNT/B receptor binding domain ($H_C$ domain).

Described herein is a modified BoNT/A for use in treating cervical dystonia of a subject for a longer duration than that treated by an unmodified BoNT/A (e.g. SEQ ID NO: 2), wherein the modified BoNT/A is administered by intramuscular injection to an affected neck muscle of the subject,

wherein the modified BoNT/A is administered by way of a unit dose of 31 Units (U) to 707 U of modified BoNT/A, wherein 1 Unit is an amount of the modified BoNT/A that corresponds to the calculated median lethal dose ($LD_{50}$) in mice,
wherein at least a single unit dose is administered to the affected neck muscle,
wherein the total dose administered during the treatment is up to 7,070 U of modified BoNT/A, and
wherein the modified BoNT/A comprises a BoNT/A light-chain and translocation domain, and a BoNT/B receptor binding domain ($H_c$ domain).

The unit dose may be 31 Units to 707 Units of modified BoNT/A wherein the modified BoNT/A comprises a BoNT/A light-chain and translocation domain, and a BoNT/B receptor binding domain ($H_c$ domain). An upper limit of the unit dose range may be 700, 650, 600, 550, 500, 450, 400, 350, 300, 250, 200, 150 100, 95, 90, 85, 65, 60, 55, 50, or 31 Units of modified BoNT/A, preferably the upper limit is 666 Units. A lower limit of the unit dose range may be 35, 40, 45, 50, 60, 65, 70, 75, 80, 85, 90, 100, 150, 200, 250, 300, 350, 400, 450, 500, 550, 600, 650, or 700 Units of modified BoNT/A, preferably the lower limit is 42 Units. Preferably, the unit dose of modified BoNT/A is 42 Units to 666 Units of modified BoNT/A, for example 40 Units to 50 Units, 70 Units to 95 Units, or 333 Units to 499 Units. Preferably, a unit dose of modified BoNT/A may be 41.6 Units, 83.2 Units, 124.8 Units, 332.8 Units or 665.6 Units.

A total dose administered when carrying out the treatment regimen of the present disclosure may be up to 7,070 Units of modified BoNT/A, wherein the modified BoNT/A comprises a BoNT/A light-chain and translocation domain, and a BoNT/B receptor binding domain ($H_c$ domain). In other words, the total amount of modified BoNT/A administered at a given treatment session may be up to 7,070 Units. The total dose may be up to 7,000, 6,000, 5,000, 4,000, 3,500, 3,350, 3,000, 2,000, 1,500, 1,250, 1,175, 1,000, 900, 800, 700, 650, 625, 600, 575, 550, 500, 450, 425, 400, 350, 330, 300, 290, 250, or 200 Units. Preferably, the total dose may be up to 6,660 Units of modified BoNT/A, e.g. the total dose may be up to 290, 425, 575, 600, 800, 1,000, 1,175, 1,250, 3,000, 3,350, 3,500, or 7,000 Units. The total dose may be at least 35, 40, 50, 100, 150, 200, 250, 290, 300, 350, 400, 450, 500, 540, 550, 580, 600, 650, 700, 750, 800, 850, 900, 950, 1,000, 1,050, 1,500, 2,000, 3,000, 4,000, 5,000, 6,000 or 7,000 Units. Preferably, the total dose may be at least 62 Units, more preferably at least 83 Units of modified BoNT/A, e.g. at least 208 Units. The total dose may be 217 Units to 7,070 Units, preferably 294 Units to 6,660 Units. For example, the total dose administered may be 250-1,250 Units, such as 250-625 Units, or 540-1,200 Units. More preferably, the total dose administered is 420 Units to 6,660 Units. Preferably, the total dose administered may be 291 Units, 416 Units, 582 Units, 832 Units, 1,165 Units, 1,248 Units, 3,328 Units or 6,656 Units.

Accordingly, the unit dose may be 31 Units to 707 Units of modified BoNT/A, wherein the modified BoNT/A comprises a BoNT/A light-chain and translocation domain, and a BoNT/B receptor binding domain ($H_c$ domain), and the total dose administered when carrying out the treatment regimen of the present disclosure may be up to 7,070 Units. The unit dose may be 41.6 Units and the total dose may be up to 291 Units. The unit dose may be 41.6 Units and the total dose may be up to 416 Units. The unit dose may be 41.6 Units and the total dose may be up to 582 Units. The unit dose may be 83.2 Units and the total dose may be up to 582 Units. The unit dose may be 83.2 Units and the total dose may be up to 832 Units. The unit dose may be 83.2 Units and the total dose may be up to 1,165 Units. The unit dose may be 124.8 Units and the total dose may be up to 1,248 Units. The unit dose may be 332.8 Units and the total dose may be up to 3,328 Units. The unit dose may be 665.5 Units and the total dose may be up to 6,656 Units.

Accordingly, the unit dose may be 31 Units to 707 Units of modified BoNT/A, wherein the modified BoNT/A comprises a BoNT/A light-chain and translocation domain, and a BoNT/B receptor binding domain (H$_c$ domain), and the total dose administered when carrying out the treatment regimen of the present disclosure may be up to 7,070 Units. The unit dose may be 41.6 Units and the total dose may be 291 Units. The unit dose may be 41.6 Units and the total dose may be 416 Units. The unit dose may be 41.6 Units and the total dose may be 582 Units. The unit dose may be 83.2 Units and the total dose may be 582 Units. The unit dose may be 83.2 Units and the total dose may be 832 Units. The unit dose may be 83.2 Units and the total dose may be 1,165 Units. The unit dose may be 124.8 Units and the total dose may be 1,248 Units. The unit dose may be 332.8 Units and the total dose may be 3,328 Units. The unit dose may be 665.5 Units and the total dose may be 6,656 Units.

Described herein is a modified botulinum neurotoxin A (BoNT/A) for use in treating cervical dystonia, wherein the modified BoNT/A is administered by intramuscular injection to an affected neck muscle of a subject,

> wherein the modified BoNT/A is administered by way of a unit dose of 450 pg to 8,000 pg of modified BoNT/A,
> wherein at least a single unit dose is administered to the affected neck muscle,
> wherein the total dose administered during the treatment is up to 80,000 pg of modified BoNT/A, and
> wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from:
>
>> (i) substitution of an acidic surface exposed amino acid residue with a basic amino acid residue;
>> (ii) substitution of an acidic surface exposed amino acid residue with an uncharged amino acid residue;
>> (iii) substitution of an uncharged surface exposed amino acid residue with a basic amino acid residue;
>> (iv) insertion of a basic amino acid residue; and
>> (v) deletion of an acidic surface exposed amino acid residue.

Described herein is a modified BoNT/A for use in treating cervical dystonia of a subject for a longer duration than that treated by an unmodified BoNT/A (e.g. SEQ ID NO: 2), wherein the modified BoNT/A is administered by intramuscular injection to an affected neck muscle of the subject,

> wherein the modified BoNT/A is administered by way of a unit dose of 450 pg to 8,000 pg of modified BoNT/A,
> wherein at least a single unit dose is administered to the affected neck muscle,
> wherein the total dose administered during the treatment is up to 80,000 pg of modified BoNT/A, and
> wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from:
>
>> (i) substitution of an acidic surface exposed amino acid residue with a basic amino acid residue;
>> (ii) substitution of an acidic surface exposed amino acid residue with an uncharged amino acid residue;
>> (iii) substitution of an uncharged surface exposed amino acid residue with a basic amino acid residue;
>> (iv) insertion of a basic amino acid residue; and
>> (v) deletion of an acidic surface exposed amino acid residue.

The unit dose may be 450 pg to 8,000 pg of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. An upper limit of the unit dose range may be 7,750, 7,500, 7,000, 6,000, 5,000, 4,000, 3,000, 2,000 or 1,000, pg of modified BoNT/A, preferably the upper limit is 7,500 pg. A lower limit of the unit dose range may be 475, 500, 600, 700, 800, 900, 1,000, 1,500, 2,000, 3,000, 4,000, 5,000, 6,000 or 7,000 pg of modified BoNT/A, preferably the lower limit is 500 pg. Preferably, the unit dose of modified BoNT/A is 500 pg to 7,500 pg of modified BoNT/A, e.g. 4,000 pg to 6,000 pg. Most preferably a unit dose of modified BoNT/A is 2,000 pg to 3,000 pg, such as 2,400 pg to 2,600 pg.

The unit dose may be greater than 1,000 or greater than 5,000 pg of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. The unit dose of modified BoNT/A may be greater than 1,000 pg up to 7,500 pg of modified BoNT/A, e.g. greater than 5,000 pg up to 7,500 pg of modified BoNT/A.

A total dose administered when carrying out the treatment regimen of the present disclosure may be up to 80,000 pg of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. In other words, the total amount of modified BoNT/A administered at a given treatment session may be up to 80,000 pg. The total dose may be up to 75,000, 70,000, 60,000, 50,000, 40,000, 30,000, 20,000, 10,000 or 5,000 pg. Preferably, the total dose may be up to 75,000 pg of modified BoNT/A. The total dose may be at least 700, 800, 900, 1,000, 2,000, 3,000, 4,000, 5,000, 7,500, 10,000, 12,500, 15,000, 20,000, 30,000, 40,000, 50,000, 60,000, or 70,000 pg. Preferably, the total dose may be at least 900 pg, more preferably at least 1,000 pg of modified BoNT/A, e.g. at least 3,000 pg. The total dose may be 3,150 pg to 80,000 pg, preferably 3,500 pg to 75,000 pg. More preferably, the total dose administered is 7,500-75,000 pg.

The total dose administered may be greater than 1,000 or greater than 5,000 pg of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. Preferably, the total dose may be greater than 1,000 pg of modified BoNT/A, e.g. greater than 5,000 pg of modified BoNT/A.

Described herein is a modified botulinum neurotoxin A (BoNT/A) for use in treating cervical dystonia, wherein the modified BoNT/A is administered by intramuscular injection to an affected neck muscle of a subject,

wherein the modified BoNT/A is administered by way of a unit dose of 53 U to 948 U of modified BoNT/A, wherein 1 Unit is an amount of the modified BoNT/A that corresponds to the calculated median lethal dose ($LD_{50}$) in mice,
wherein at least a single unit dose is administered to the affected neck muscle,
wherein the total dose administered during the treatment is up to 9,480 U of modified BoNT/A, and
wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from:

(i) substitution of an acidic surface exposed amino acid residue with a basic amino acid residue;
(ii) substitution of an acidic surface exposed amino acid residue with an uncharged amino acid residue;
(iii) substitution of an uncharged surface exposed amino acid residue with a basic amino acid residue;

(iv) insertion of a basic amino acid residue; and

(v) deletion of an acidic surface exposed amino acid residue.

Described herein is a modified BoNT/A for use in treating cervical dystonia of a subject for a longer duration than that treated by an unmodified BoNT/A (e.g. SEQ ID NO: 2), wherein the modified BoNT/A is administered by intramuscular injection to an affected neck muscle of the subject,

wherein the modified BoNT/A is administered by way of a unit dose of 53 U to 948 U of modified BoNT/A, wherein 1 Unit is an amount of the modified BoNT/A that corresponds to the calculated median lethal dose ($LD_{50}$) in mice,

wherein at least a single unit dose is administered to the affected neck muscle,

wherein the total dose administered during the treatment is up to 9,480 U of modified BoNT/A, and

wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from:

(i) substitution of an acidic surface exposed amino acid residue with a basic amino acid residue;

(ii) substitution of an acidic surface exposed amino acid residue with an uncharged amino acid residue;

(iii) substitution of an uncharged surface exposed amino acid residue with a basic amino acid residue;

(iv) insertion of a basic amino acid residue; and

(v) deletion of an acidic surface exposed amino acid residue.

The unit dose may be 53 Units to 948 Units of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. An upper limit of the unit dose range may be 925, 900, 850, 800, 750, 700, 650, 600, 550, 500, 450, 400, 350, 300, 250, 200, 150 or 100 Units of modified BoNT/A, preferably the upper limit is 889 Units. A lower limit of the unit dose range may be 55, 60, 65, 70, 75, 80, 85, 90, 100, 150, 200, 250, 300, 350, 400, 450, 500, 550, 600, 650, 700, 750, 800, 850 or 900 Units of modified BoNT/A, preferably the lower limit is 59 Units. Preferably, the unit dose of modified BoNT/A is 59 Units to 889 Units of modified BoNT/A, for example 200 Units to 600 Units. Most preferably a unit dose of modified BoNT/A is 237 Units to 355 Units, such as 284 Units to 308 Units.

The unit dose may be greater than 118.5 Units or greater than 592.5 Units of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. The unit dose of modified BoNT/A may be greater than 118.5 Units up to 888 Units of modified BoNT/A, e.g. greater than 592.5 Units up to 888 Units of modified BoNT/A.

A total dose administered when carrying out the treatment regimen of the present disclosure may be up to 9,480 Units of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. In other words, the total amount of modified BoNT/A administered at a given treatment session may be up to 9,480 Units. The total dose may be up to 9,000, 8,000, 7,000, 6,000, 5,000, 4,000, 3,000, 2,000 or 1,000 Units. Preferably, the total dose may be up to 8,890 Units of modified BoNT/A. The total dose may be at least 83, 95, 106, 150, 200, 250, 300, 350, 400, 450, 500, 550, 600, 650, 700, 750, 800, 850, 900, 950, 1,000, 1,500, 2,000, 3,000, 4,000, 5,000, 6,000, 7,000, 8,000 or 9,000 Units. Preferably, the total dose may be at least 106 Units, more preferably at least 118 Units of modified BoNT/A, e.g. at least 355 Units. The total dose may be 371 Units to 9,480 Units, preferably 413 Units to 8,890 Units. More preferably, the total dose administered is 889 Units to 8,890 Units.

The total dose administered may be greater than 118.5 Units or greater than 592.5 Units of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. Preferably, the total dose may be greater than 118.5 Units of modified BoNT/A, e.g. greater than 592.5 Units of modified BoNT/A.

An "affected neck muscle" may be a neck muscle contributing to (e.g. causing) cervical dystonia and/or a symptom thereof in a subject or that contributes to (e.g. causes) cervical dystonia and/or a symptom thereof in a subject. It is not intended that the "affected neck muscle" necessarily has to be contributing to (e.g. causing) cervical dystonia and/or a symptom thereof at the time of treatment, although this is preferred. For example, the neck muscle may be one that in the past has contributed to (e.g. caused) cervical dystonia and/or a symptom thereof in the subject or that is expected to contribute to (e.g. cause) cervical dystonia and/or a symptom thereof in the subject in the future. In one embodiment two or more neck muscles (e.g. an agonist and antagonist pair of neck muscles) may contribute to (e.g. cause) cervical dystonia and/or a symptom thereof in a subject. In such cases, modified BoNT/A may be administered to the two or more neck muscles (e.g. administered to the agonist neck muscle and the antagonist neck muscle of the pair of neck muscles).

An affected neck muscle preferably contributes to (e.g. causes) cervical dystonia and/or a symptom thereof in a subject by contracting. Thus, an affected neck muscle is preferably a neck muscle of the subject that is contracted or that contracts resulting in cervical dystonia and/or a symptom thereof in the subject. Said neck muscle is preferably a neck muscle that involuntarily contracts or that has involuntarily contracted, e.g. at the time of treatment. A neck muscle may be any muscle (e.g. skeletal muscle) that is operably connected to the neck and/or head of a subject, for example any muscle that is capable of altering the head positioning of a subject (e.g. when contracted). An affected neck muscle may be one that is capable of: causing twisting of the chin of a subject towards a shoulder of the subject resulting in sideways head rotation (torticollis); causing tipping forward of the head of a subject (anterocollis); causing tipping backwards of the head of a subject (retrocollis); causing sideways tilting of the head of a subject (laterocollis); causing an anterior sagittal shift (a forward shift) of the head of a subject; and/or causing a posterior sagittal shift (a backwards shift) of the head of a subject.

An affected neck muscle may comprise the sternocleidomastoid, the sternocleidomastoideus, the splenius capitis, the splenius cervicis, the scalene complex (e.g. the scalenus anterior and/or the scalenus medius), the trapezius (e.g. the upper trapezius and/or the trapezius pars descendens), the levator scapulae, the semispinalis capitis, or the longissimus (e.g. longissimus capitis and/or longissimus cervicis). An affected neck muscle may comprise the sternocleidomastoid, the splenius capitis, the splenius cervicis, the scalene complex (e.g. the scalenus anterior and/or the scalenus medius), the trapezius (e.g. the upper trapezius and/or the trapezius pars descendens), the levator scapulae, the semispinalis capitis, the longissimus (e.g. longissimus capitis and/or longissimus cervicis), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, or the processus transversus C2-C5-atlas (anterior tubercle). An affected neck muscle may comprise the right levator scapulae, the left levator scapulae, the right trapezius, the left trapezius, the right sternocleidomastoid, the left sternocleidomastoid, the right splenius capitis, the left splenius capitis, the scalenus medius, the scalenus anterior, the right semispinalis capitis, the left semispinalis capitis, the right longissimus capitis, or the left longissimus capitis. An affected neck muscle may comprise the sternocleidomastoideus (e.g. the left or right sternocleidomastoid), the left or right splenius capitis, the scalenus anterior or the scalenus medius, the left or right trapezius (e.g. the left or right upper trapezius), the left or right levator scapulae, the left or right semispinalis capitis, the longissimus (e.g. the left or right longissimus capitis and/or longissimus cervicis), the splenius cervicis, the scalene complex (e.g. the scalenus anterior and/or the scalenus medius), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the obliquus capitis superior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, the semispinalis cervicis, the rectus capitis posterior major, the rectus capitis posterior minor, the rectus capitis anterior, the multifudus, or the processus transversus C2-C5-atlas (anterior tubercle).

An affected neck muscle may comprise: M. semispinalis cervicis, M. levator scapulae, M. splenius cervicis, M. longissimus cervicis, M. trapezius pars descendens, M. sternocleidomastoideus, M. semispinalis capitis, M. obliquus capitis inferior, M. longissimus capitis, M. splenius capitis, M. semispinalis cervicis, M. scalenus medius, M. longissimus capitis, M. longus colli, or M. longus capitis.

A plurality of affected neck muscles treated in accordance with the invention may comprise at least one (e.g. at least two) of any of the muscles described herein.

In one embodiment, a modified BoNT/A may be administered to one or more affected neck muscle(s) selected from: the sternocleidomastoid, the splenius capitis, the splenius cervicis, the scalene complex (e.g. the scalenus anterior and/or the scalenus medius), the trapezius (e.g. the upper trapezius and/or the trapezius pars descendens), the levator scapulae, the semispinalis capitis, and the longissimus (e.g. longissimus capitis and/or longissimus cervicis). Preferably, a modified BoNT/A is administered to a plurality of affected neck muscles. For example, a modified BoNT/A may be administered to at least two (e.g. at least three, four, five, six or seven, preferably eight) affected neck muscles selected from: the sternocleidomastoid, the splenius capitis, the splenius cervicis, the scalene complex (e.g. the scalenus anterior and/or the scalenus medius), the trapezius (e.g. the upper trapezius and/or the trapezius pars descendens), the levator scapulae, the semispinalis capitis, and the longissimus (e.g. longissimus capitis and/or longissimus cervicis).

In one embodiment, a modified BoNT/A may be administered to one or more affected neck muscle(s) selected from: the right levator scapulae, the left levator scapulae, the right trapezius, the left trapezius, the right sternocleidomastoid, the left sternocleidomastoid, the right splenius capitis, the left splenius capitis, the scalenus medius, the scalenus anterior, the right semispinalis capitis, the left semispinalis capitis, the right longissimus capitis, and the left longissimus capitis.

In one embodiment, a modified BoNT/A may be administered to one or more affected neck muscle(s) selected from: the sternocleidomastoid, the sternocleidomastoideus, the splenius capitis, the splenius cervicis, the scalene complex (e.g. the scalenus anterior and/or the scalenus medius), the trapezius (e.g. the upper trapezius and/or the trapezius pars descendens), the levator scapulae, the semispinalis capitis, the longissimus (e.g. longissimus capitis and/or longissimus cervicis), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, and the processus transversus C2-C5-atlas (anterior tubercle). Preferably, a modified BoNT/A is administered to a plurality of affected neck muscles. For example, a modified BoNT/A may be administered to at least two (e.g. at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35) affected neck muscles selected from: the sternocleidomastoid, the sternocleidomastoideus, the splenius capitis, the splenius cervicis, the scalene complex (e.g. the scalenus anterior and/or the scalenus medius), the trapezius (e.g. the upper trapezius, and/or the trapezius pars descendens), the levator scapulae, the semispinalis capitis, the longissimus (e.g. longissimus capitis and/or longissimus cervicis), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, and the processus transversus C2-C5-atlas (anterior tubercle).

In one embodiment, a modified BoNT/A may be administered to one or more affected neck muscle(s) comprising: the sternocleidomastoideus (e.g. the left or right sternocleidomastoid), the left or right splenius capitis, the scalenus anterior or the scalenus medius, the left or right trapezius (e.g. the left or right upper trapezius), the left or right levator scapulae, the left or right semispinalis capitis, the semispinalis capitis pars med, the longissimus (e.g. the left or right longissimus capitis and/or longissimus cervicis), the splenius cervicis, the scalene complex (e.g. the scalenus anterior and/or the scalenus medius), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the obliquus capitis superior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, the semispinalis cervicis, the rectus capitis posterior major, the rectus capitis posterior minor, the rectus capitis anterior, the multifudus, or the processus transversus C2-C5-atlas (anterior tubercle). Preferably, a modified BoNT/A is administered to a plurality of affected neck muscles. For example, a modified BoNT/A may be administered to at least two (e.g. at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35) affected neck muscles selected from: the sternocleidomastoideus (e.g. the left or right sternocleidomastoid), the left or right splenius capitis, the scalenus anterior or the scalenus medius, the left or right trapezius (e.g. the left or right upper trapezius), the left or right levator scapulae, the left or right semispinalis capitis, the semispinalis capitis pars med, the longissimus (e.g. the left or right longissimus capitis and/or longissimus cervicis), the splenius cervicis, the scalene complex (e.g. the scalenus anterior and/or the scalenus medius), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the obliquus capitis superior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, the semispinalis cervicis, the rectus capitis posterior major, the rectus capitis posterior minor, the rectus capitis anterior, the multifudus, and/or the processus transversus C2-C5-atlas (anterior tubercle).

In one embodiment, a modified BoNT/A may be administered to one or more affected neck muscle(s) selected from: the sternocleidomastoideus (e.g. the left or right sternocleidomastoid), the left or right splenius capitis, the scalenus anterior or the scalenus medius, the left or right trapezius (e.g. the left or right upper trapezius), the left or right levator scapulae, the left or right semispinalis capitis, the longissimus (e.g. the left or right longissimus capitis and/or longissimus cervicis), the splenius cervicis, the scalene complex (e.g. the scalenus anterior and/or the scalenus medius), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the obliquus capitis superior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, the semispinalis cervicis, the rectus capitis posterior major, the rectus capitis posterior minor, the rectus capitis anterior, the multifudus, and the processus transversus C2-C5-atlas (anterior tubercle). Preferably, a modified BoNT/A is administered to a plurality of affected neck muscles. For example, a modified BoNT/A may be administered to at least two (e.g. at least 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34 or 35) affected neck muscles selected from: the sternocleidomastoideus (e.g. the left or right sternocleidomastoid), the left or right splenius capitis, the scalenus anterior or the scalenus medius, the left or right trapezius (e.g. the left or right upper trapezius), the left or right levator scapulae, the left or right semispinalis capitis, the longissimus (e.g. the left or right longissimus capitis and/or longissimus cervicis), the splenius cervicis, the scalene complex (e.g. the scalenus anterior and/or the scalenus medius), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior-Clavicula (lateral part), the processus spinosus C3-Th3-processus mastoideus, the processus spinosus Th3-Th5-processus transversus C1-C2, the processus transversus C3-Th6, the processus spinosus C3-Th1-linea nuchalis superior, the processus transversus Th1-Th6-processus spinosus C2-C7, the processus transversus C3-Th3-processus mastoideus, the processus transversus Th1-Th6-processus transversus C2-C6, the obliquus capitis inferior, the obliquus capitis superior, the processus spinosus C2-processus transversus C1, the suprasternal notch and clavicula (medial part)-processus mastoideus and linea nuchalis superior, the processus transversus C1-C4-scapula (angulus superior), the processus transversus C2-C7-first rib, the processus transversus C3-C6-first rib, the longus capitis, the processus transversus C3-C6-occipital bone (basilar part), the longus colli, the semispinalis cervicis, the rectus capitis posterior major, the rectus capitis posterior minor, the rectus capitis anterior, the multifudus, and the processus transversus C2-C5-atlas (anterior tubercle).

In one embodiment, a modified BoNT/A may be administered to one or more affected neck muscle(s) comprising: M. semispinalis cervicis, M. levator scapulae, M. splenius cervicis, M. longissimus cervicis, M. trapezius pars descendens, M. sternocleidomastoideus, M. semispinalis capitis, M. obliquus capitis inferior, M. longissimus capitis, M. splenius capitis, M. semispinalis cervicis, M. scalenus medius, M. longissimus capitis, M. longus colli, or M. longus capitis. In one embodiment, a modified BoNT/A may be administered to one or more affected neck muscle(s) selected from: M. semispinalis cervicis, M. levator scapulae, M. splenius cervicis, M. longissimus cervicis, M. trapezius pars descendens, M. sternocleidomastoideus, M. semispinalis capitis, M. obliquus capitis inferior, M. longissimus capitis, M. splenius capitis, M. semispinalis cervicis, M. scalenus medius, M. longissimus capitis, M. longus colli, and M. longus capitis. For example, a modified BoNT/A may be administered to at least two (e.g. at least 3, 4, 5, 6, 7, 8, 9, 10, 11, 12, 13, 14, or 15) of said affected neck muscles.

In one embodiment, the modified BoNT/A is administered by intramuscular injection to a plurality of affected neck muscles of the subject, and wherein at least a single unit dose is administered to each affected neck muscle, preferably wherein:

the plurality of affected neck muscles are selected from: the sternocleidomastoid, the splenius capitis, the splenius cervicis, the scalene complex (e.g. the scalenus anterior and/or the scalenus medius), the trapezius (e.g. the upper trapezius), the levator scapulae, the semispinalis capitis, and the longissimus (e.g. longissimus capitis and/or longissimus cervicis);

the plurality of affected neck muscles are selected from: the right levator scapulae, the left levator scapulae, the right trapezius, the left trapezius, the right sternocleidomastoid, the left sternocleidomastoid, the right splenius capitis, the left splenius capitis, the scalenus medius, the scalenus anterior, the right semispinalis capitis, the left semispinalis capitis, the right longissimus capitis, and the left longissimus capitis;

the plurality of affected neck muscles are selected from: the sternocleidomastoid, the sternocleidomastoideus, the splenius capitis, the splenius cervicis, the scalene complex (e.g. the scalenus anterior and/or the scalenus medius), the trapezius (e.g. the upper trapezius), the levator scapulae, the semispinalis capitis, the longissimus (e.g. longissimus capitis and/or longissimus cervicis), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior -Clavicula (lateral part), the processus spinosus C3-Th3-processus mastoideus, the processus spinosus Th3-Th5 -processus transversus C1-C2, the processus transversus C3-Th6, the processus spinosus C3-Th1 -linea nuchalis superior, the processus transversus Th1-Th6 -processus spinosus C2-C7, the processus transversus C3-Th3 -processus mastoideus, the processus transversus Thl-Th6 -processus transversus C2-C6, the obliquus capitis inferior, the processus spinosus C2 --processus transversus C1, the suprasternal notch and clavicula (medial part) -processus mastoideus and linea nuchalis superior, the processus transversus C1-C4 - scapula (angulus superior), the processus transversus C2-C7 -first rib, the processus transversus C3-C6 -first rib, the longus capitis, the processus transversus C3-C6 -occipital bone (basilar part), the longus colli, and the processus transversus C2-C5 -atlas (anterior tubercle); or

the plurality of affected neck muscles are selected from: the sternocleidomastoideus (e.g. the left or right sternocleidomastoid), the left or right splenius capitis, the scalenus anterior or the scalenus medius, the left or right trapezius (e.g. the left or right upper trapezius), the left or right levator scapulae, the left or right semispinalis capitis, the semispinalis capitis pars med, the longissimus (e.g. the left or right longissimus capitis and/or longissimus cervicis), the splenius cervicis, the scalene complex (e.g. the scalenus anterior and/ or the scalenus medius), the posterior paravertebrals (e.g. the scalenus posterior, scalenus medius and/or scalenus anterior, preferably the scalenus posterior), the submental complex (e.g. the digastric muscle, the geniohyoid muscle, the mylohyoid muscle, the mylohyoid boutonniere and/or the stylohyoid muscle), the trapezius pars descendens, the linea nuchalis superior -Clavicula (lateral part), the processus spinosus C3-Th3-processus mastoideus, the processus spinosus Th3-Th5 -processus transversus C1-C2, the processus transversus C3-Th6, the processus spinosus C3-Th1 -linea nuchalis superior, the processus transversus Th1-Th6 -processus spinosus C2-C7, the processus transversus C3-Th3 -processus mastoideus, the processus transversus Th1-Th6 -processus transversus C2-C6, the obliquus capitis inferior, the obliquus capitis superior, the processus spinosus C2 -processus transversus C1, the suprasternal notch and clavicula (medial part) - processus mastoideus and linea nuchalis superior, the processus transversus C1-C4 -scapula (angulus superior), the processus transversus C2-C7 -first rib, the processus transversus C3-C6 -first rib, the longus capitis, the processus transversus C3-C6 -occipital bone (basilar part), the longus colli, the semispinalis cervicis, the rectus capitis posterior major, the rectus capitis posterior minor, the rectus capitis anterior, the multifudus, and the processus transversus C2-C5 -atlas (anterior tubercle).

Where there are two equivalent neck muscles either side of the neck (e.g. the sternocleidomastoid muscles, such as the left and right sternocleidomastoid muscles) the modified BoNT/A may be administered unilaterally (e.g. to one of the muscles where only one is contracted) or bilaterally (e.g. to both of the muscles where both muscles are contracted). When treating a plurality of affected muscles, it is preferred, that this is a plurality of different types of affected muscles. For example, where bilateral administration to two affected sternocleidomastoid muscles is carried out, it is preferred that a further muscle is treated when treating a plurality of affected muscles as described herein.

Where there are two equivalent neck muscles either side of the neck, unilateral administration may be to either of said equivalent neck muscles. For example, administration may be to a contracted muscle or a non-contracted equivalent muscle. In one embodiment, unilateral administration is to a muscle on a side of a subject's neck, wherein the side of the subject's neck exhibits a symptom of cervical dystonia or to an equivalent muscle on a contralateral side of the subject's neck, wherein the contralateral side of the subject's neck does not exhibit a symptom of cervical dystonia.

The affected neck muscles selected for treatment according to the invention may depend on the particular presentation of cervical dystonia of the subject to be treated (e.g. torticollis, laterocollis, anterocollis, retrocollis, or combinations thereof).

The affected neck muscles selected for treatment according to the invention may depend on the particular presentation of cervical dystonia of the subject to be treated (e.g. torticollis, laterocollis, anterocollis, retrocollis, laterocaput, torticaput, antecaput, retrocaput, lateral shift, sagittal shift or combinations thereof).

In one embodiment, when treating torticollis, a modified BoNT/A may be administered to one or more affected neck muscles selected from: the sternocleidomastoid, the trapezius (e.g. upper trapezius), the scalenus anterior, the splenius capitis, the splenius cervicis, the levator scapulae, and the longissimus (e.g. the longissimus capitis and/or longissimus cervicis). Preferably, when treating torticollis, modified BoNT/A may be administered contralaterally to one or more affected neck muscles selected from: the sternocleidomastoid, the trapezius (e.g. upper trapezius), and the scalenus anterior; and/or administered ipsilaterally to one or more affected neck muscles selected from: the splenius capitis, the splenius cervicis, the levator scapulae, and the longissimus (e.g. the longissimus capitis and/or longissimus cervicis).

In one embodiment, when treating torticollis, a modified BoNT/A may be administered to one or more affected neck muscles selected from: the sternocleidomastoid, the trapezius (e.g. upper trapezius), the scalenus anterior, the splenius capitis, the splenius cervicis, the levator scapulae, the longissimus (e.g. the longissimus capitis and/or longissimus cervicis) and the semispinalis cervicis. In one embodiment, when treating torticollis, modified BoNT/A may be administered contralaterally to one or more affected neck muscles selected from: the sternocleidomastoid, the trapezius (e.g. upper trapezius), and the scalenus anterior; and/or administered ipsilaterally to one or more affected neck muscles selected from: the splenius capitis, the splenius cervicis, the levator scapulae, the longissimus (e.g. the longissimus capitis and/or longissimus cervicis), and the semispinalis cervicis.

In one embodiment, when treating torticollis, a modified BoNT/A may be administered to one or more affected neck muscles selected from: the sternocleidomastoid, the trapezius (e.g. upper trapezius), the scalenus anterior, the splenius capitis, the splenius cervicis, the levator scapulae, the longissimus (e.g. the longissimus capitis and/or longissimus cervicis), the semispinalis cervicis, the rectus capitis posterior major, the multifidus and the obliquus capitis inferior. In one embodiment, when treating torticollis, modified BoNT/A may be administered contralaterally to one or more affected neck muscles selected from: the sternocleidomastoid, the trapezius (e.g. upper trapezius), the scalenus anterior, the semispinalis cervicis, and the multifudus; and/or administered ipsilaterally to one or more affected neck muscles selected from: the splenius capitis, the splenius cervicis, the levator scapulae, the longissimus (e.g. the longissimus capitis and/or longissimus cervicis), the rectus capitis posterior major, and the obliquus capitis inferior.

In one embodiment, when treating torticollis, a modified BoNT/A may be administered to one or more affected neck muscle(s) comprising: the sternocleidomastoid, the trapezius (e.g. upper trapezius), the scalenus anterior, the splenius capitis, the splenius cervicis, the levator scapulae, the longissimus (e.g. the longissimus capitis and/or longissimus cervicis), the semispinalis cervicis, the rectus capitis posterior major, the multifidus or the obliquus capitis inferior. In one embodiment, when treating torticollis, modified BoNT/A may be administered contralaterally to one or more affected neck muscle(s) comprising: the sternocleidomastoid, the trapezius (e.g. upper trapezius), the scalenus anterior, the semispinalis cervicis, or the multifudus; and/or administered ipsilaterally to one or more affected neck muscle(s) comprising: the splenius capitis, the splenius cervicis, the levator scapulae, the longissimus (e.g. the longissimus capitis and/or longissimus cervicis), the rectus capitis posterior major, or the obliquus capitis inferior.

In one embodiment, when treating laterocollis, a modified BoNT/A may be administered to one or more affected neck muscles selected from: the levator scapulae, the trapezius (e.g. upper trapezius), the scalene complex (e.g. the scalenus anterior and/or the scalenus medius), the sternocleidomastoid, the splenius capitis, the splenius cervicis, and the longissimus (e.g. the longissimus capitis and/or longissimus cervicis). Preferably, when treating laterocollis, modified BoNT/A may be administered ipsilaterally to one or more of said affected neck muscles.

In one embodiment, when treating laterocollis, a modified BoNT/A may be administered to one or more affected neck muscles selected from: the levator scapulae, the trapezius (e.g. upper trapezius), the scalene complex (e.g. the scalenus anterior and/or the scalenus medius), the sternocleidomastoid, the splenius capitis, the splenius cervicis, the longissimus (e.g. the longissimus capitis and/or longissimus cervicis) and the semispinalis cervicis. In one embodiment, when treating laterocollis, modified BoNT/A may be administered ipsilaterally to one or more of said affected neck muscles.

In one embodiment, when treating laterocollis, a modified BoNT/A may be administered to one or more affected neck muscles selected from: the levator scapulae, the trapezius (e.g. upper trapezius), the scalene complex (e.g. the scalenus anterior and/or the scalenus medius), the sternocleidomastoid, the splenius capitis, the splenius cervicis, the longissimus (e.g. the longissimus capitis and/or longissimus cervicis), and the multifidus. In one embodiment, when treating laterocollis, modified BoNT/A may be administered ipsilaterally to one or more of said affected neck muscles.

In one embodiment, when treating laterocollis, a modified BoNT/A may be administered to one or more affected neck muscles selected from: the levator scapulae, the trapezius (e.g. upper trapezius), the scalene complex (e.g. the scalenus anterior and/or the scalenus medius), the sternocleidomastoid, the splenius capitis, the splenius cervicis, the longissimus (e.g. the longissimus capitis and/or longissimus cervicis), the semispinalis cervicis, and the multifidus. In one embodiment, when treating laterocollis, modified BoNT/A may be administered ipsilaterally to one or more of said affected neck muscles.

In one embodiment, when treating laterocollis, a modified BoNT/A may be administered to one or more affected neck muscle(s) comprising: the levator scapulae, the trapezius (e.g. upper trapezius), the scalene complex (e.g. the scalenus anterior and/or the scalenus medius), the sternocleidomastoid, the splenius capitis, the splenius cervicis, the longissimus (e.g. the longissimus capitis and/or longissimus cervicis), the semispinalis cervicis, or the multifidus. In one embodiment, when treating laterocollis, modified BoNT/A may be administered ipsilaterally to one or more of said affected neck muscles.

In one embodiment, when treating anterocollis, a modified BoNT/A may be administered to one or more affected neck muscles selected from: the sternocleidomastoid, the scalenus anterior, and the scalenus medius. Preferably, when treating anterocollis, modified BoNT/A may be administered bilaterally.

In one embodiment, when treating anterocollis, a modified BoNT/A may be administered to one or more affected neck muscles selected from: the sternocleidomastoid, the scalenus anterior, the scalenus medius, the levator scapulae, the longus colli, and the submental complex (e.g. the digastric muscle, the geniohyoid muscle, the mylohyoid muscle, the mylohyoid boutonniere and/or the stylohyoid muscle). Preferably, when treating anterocollis, modified BoNT/A may be administered bilaterally.

In one embodiment, when treating anterocollis, a modified BoNT/A may be administered to one or more affected neck muscles selected from: the sternocleidomastoid, the scalenus anterior, the scalenus medius, the longus capitis, the longus colli, and the rectus capitis anterior. In one embodiment, when treating anterocollis, modified BoNT/A may be administered bilaterally.

In one embodiment, when treating anterocollis, a modified BoNT/A may be administered to one or more affected neck muscles selected from: the sternocleidomastoid, the scalenus anterior, the scalenus medius, the levator scapulae, the longus colli, the submental complex (e.g. the digastric muscle, the geniohyoid muscle, the mylohyoid muscle, the mylohyoid boutonniere and/or the stylohyoid muscle), the longus capitis, and the rectus capitis anterior. In one embodiment, when treating anterocollis, modified BoNT/A may be administered bilaterally.

In one embodiment, when treating anterocollis, a modified BoNT/A may be administered to one or more affected neck muscle(s) comprising: the sternocleidomastoid, the scalenus anterior, the scalenus medius, the levator scapulae, the longus colli, the submental complex (e.g. the digastric muscle, the geniohyoid muscle, the mylohyoid muscle, the mylohyoid boutonniere and/or the stylohyoid muscle), the longus capitis, or the rectus capitis anterior. In one embodiment, when treating anterocollis, modified BoNT/A may be administered bilaterally.

In one embodiment, when treating retrocollis, a modified BoNT/A may be administered to one or more affected neck muscles selected from: the levator scapulae, the trapezius (e.g. upper trapezius), the longissimus (e.g. the longissimus capitis and/or longissimus cervicis), the splenius capitis, the splenius cervicis, and the semispinalis capitis. Preferably, when treating retrocollis, modified BoNT/A may be administered bilaterally.

In one embodiment, when treating retrocollis, a modified BoNT/A may be administered to one or more affected neck muscles selected from: the levator scapulae, the trapezius (e.g. upper trapezius), the longissimus (e.g. the longissimus capitis and/or longissimus cervicis), the splenius capitis, the splenius cervicis, the semispinalis capitis, the semispinalis cervicis, and the posterior paravertebrals (e.g. the scalenus posterior, scalenus medius and/or scalenus anterior, preferably the scalenus posterior). Preferably, when treating retrocollis, modified BoNT/A may be administered bilaterally.

In one embodiment, when treating retrocollis, a modified BoNT/A may be administered to one or more affected neck muscles selected from: the levator scapulae, the trapezius (e.g. upper trapezius), the longissimus (e.g. the longissimus capitis and/or longissimus cervicis), the splenius capitis, the splenius cervicis, the semispinalis capitis, the semispinalis cervicis, the spinalis capitis, the rectus capitis posterior major, the rectus capitis posterior minor, the obliquus capitis superior. Preferably, when treating retrocollis, modified BoNT/A may be administered bilaterally.

In one embodiment, when treating retrocollis, a modified BoNT/A may be administered to one or more affected neck muscles selected from: the levator scapulae, the trapezius (e.g. upper trapezius), the longissimus (e.g. the longissimus capitis and/or longissimus cervicis), the splenius capitis, the splenius cervicis, the semispinalis capitis, the semispinalis cervicis, the posterior paravertebrals (e.g. the scalenus posterior, scalenus medius and/or scalenus anterior, preferably the scalenus posterior), the spinalis capitis, the rectus capitis posterior major, the rectus capitis posterior minor, and the obliquus capitis superior. Preferably, when treating retrocollis, modified BoNT/A may be administered bilaterally.

In one embodiment, when treating retrocollis, a modified BoNT/A may be administered to one or more affected neck muscle(s) comprising: the levator scapulae, the trapezius (e.g. upper trapezius), the longissimus (e.g. the longissimus capitis and/or longissimus cervicis), the splenius capitis, the splenius cervicis, the semispinalis capitis, the semispinalis cervicis, the posterior paravertebrals (e.g. the scalenus posterior, scalenus medius and/or scalenus anterior, preferably the scalenus posterior), the spinalis capitis, the rectus capitis posterior major, the rectus capitis posterior minor, or the obliquus capitis superior. Preferably, when treating retrocollis, modified BoNT/A may be administered bilaterally.

In one embodiment, when treating lateral shift, a modified BoNT/A may be administered to one or more affected neck muscles selected from: the levator scapulae, the trapezius (e.g. upper trapezius or trapezius pars descendens), the scalene complex (e.g. the scalenus anterior and/or the scalenus medius), the sternocleidomastoid, the splenius capitis, the splenius cervicis, the longissimus (e.g. the longissimus capitis and/or longissimus cervicis), and the semispinalis cervicis. Preferably, when treating lateral shift, modified BoNT/A may be administered to an affected neck muscle selected from: the levator scapulae, the semispinalis cervicis, the scalenus medius and the longissimus cervicis on a first side (e.g. left side) of the neck and the modified BoNT/A may be administered to an affected muscle selected from the sternocleidomastoid, the trapezius pars descendens, the splenius capitis, the semispinalis capitis, the longissimus capitis and the levator scapulae on a second side (e.g. right side) of the neck.

In one embodiment, when treating lateral shift, a modified BoNT/A may be administered to one or more affected neck muscle(s) comprising: the levator scapulae, the trapezius (e.g. upper trapezius or trapezius pars descendens), the scalene complex (e.g. the scalenus anterior and/or the scalenus medius), the sternocleidomastoid, the splenius capitis, the splenius cervicis, the longissimus (e.g. the longissimus capitis and/or longissimus cervicis), or the semispinalis cervicis. Preferably, when treating lateral shift, modified BoNT/A may be administered to an affected neck muscle comprising: the levator scapulae, the semispinalis cervicis, the scalenus medius or the longissimus cervicis on a first side (e.g. left side) of the neck and the modified BoNT/A may be administered to an affected muscle comprising: the sternocleidomastoid, the trapezius pars descendens, the splenius capitis, the semispinalis capitis, the longissimus capitis or the levator scapulae on a second side (e.g. right side) of the neck.

In one embodiment, when treating laterocaput, a modified BoNT/A may be administered to one or more affected neck muscles selected from: the trapezius pars descendens, the sternocleidomastoideus, the longissimus capitis, the splenius capitis, the semispinalis capitis, the levator scapulae, and the posterior paravertebrals (e.g. the scalenus posterior, scalenus medius and/or scalenus anterior, preferably the scalenus posterior). Preferably, when treating laterocaput, modified BoNT/A may be administered ipsilaterally.

In one embodiment, when treating laterocaput, a modified BoNT/A may be administered to one or more affected neck muscle(s) comprising: the trapezius pars descendens, the sternocleidomastoideus, the longissimus capitis, the splenius capitis, the semispinalis capitis, the levator scapulae, or the posterior paravertebrals (e.g. the scalenus posterior, scalenus medius and/or scalenus anterior, preferably the scalenus posterior). Preferably, when treating laterocaput, modified BoNT/A may be administered ipsilaterally.

In one embodiment, when treating torticaput, a modified BoNT/A may be administered to one or more affected neck muscles selected from: the trapezius pars descendens, the sternocleidomastoideus, the longissimus capitis, the splenius capitis, the semispinalis capitis pars med., and the obliquus capitis inferior. Preferably, when treating torticaput, modified BoNT/A may be administered ipsilaterally or contralaterally. For example, a modified BoNT/A may be administered contralaterally to one or more affected neck muscles selected from: the trapezius pars descendens, the sternocleidomastoideus and the semispinalis capitis pars med; and/or the modified BoNT/A may be administered ipsilaterally to one or more neck muscles selected from: the obliquus capitis inferior, the longissimus capitis, and the splenius capitis.

In one embodiment, when treating torticaput, a modified BoNT/A may be administered to one or more affected neck muscle(s) comprising: the trapezius pars descendens, the sternocleidomastoideus, the longissimus capitis, the splenius capitis, the semispinalis capitis pars med., or the obliquus capitis inferior. Preferably, when treating torticaput, modified BoNT/A may be administered ipsilaterally or contralaterally. For example, a modified BoNT/A may be administered contralaterally to one or more affected neck muscle(s) comprising: the trapezius pars descendens, the sternocleidomastoideus or the semispinalis capitis pars med; and/or the modified BoNT/A may be administered ipsilaterally to one or more neck muscle(s) comprising: the obliquus capitis inferior, the longissimus capitis, or the splenius capitis.

In one embodiment, when treating antecaput, a modified BoNT/A may be administered to one or more affected neck muscles selected from: the longus capitis, the levator scapulae and the sternocleidomastoideus. Preferably, when treating antecaput, modified BoNT/A may be administered bilaterally.

In one embodiment, when treating antecaput, a modified BoNT/A may be administered to one or more affected neck muscle(s) comprising: the longus capitis, the levator scapulae or the sternocleidomastoideus. Preferably, when treating antecaput, modified BoNT/A may be administered bilaterally.

In one embodiment, when treating retrocaput, a modified BoNT/A may be administered to one or more affected neck muscles selected from: the obliquus capitis inferior, the semispinalis capitis, the trapezius pars descendens and the splenius capitis. Preferably, when treating retrocaput, modified BoNT/A may be administered bilaterally.

In one embodiment, when treating retrocaput, a modified BoNT/A may be administered to one or more affected neck muscle(s) comprising: the obliquus capitis inferior, the semispinalis capitis, the trapezius pars descendens or the splenius capitis. Preferably, when treating retrocaput, modified BoNT/A may be administered bilaterally.

In one embodiment, when treating sagittal shift, a modified BoNT/A may be administered to one or more affected neck muscles selected from: the sternocleidomastoideus, the scalenus anterior, the scalenus medius, the levator scapulae, the longus colli, the submental complex (e.g. the digastric muscle, the geniohyoid muscle, the mylohyoid muscle, the mylohyoid boutonniere and/or the stylohyoid muscle), the obliquus capitis inferior, the semispinalis capitis, the trapezius pars descendens and the splenius capitis. Preferably, when treating sagittal shift, modified BoNT/A may be administered to an affected neck muscle selected from: the sternocleidomastoideus, the scalenus anterior, the scalenus medius, the levator scapulae, the longus colli, and the submental complex (e.g. the digastric muscle, the geniohyoid muscle, the mylohyoid muscle, the mylohyoid boutonniere and/or the stylohyoid muscle) on a first side (e.g. left side) of the neck and modified BoNT/A may be administered to an affected neck muscle selected from: the obliquus capitis inferior, the semispinalis capitis, the trapezius pars descendens and the splenius capitis on a second side (e.g. right side) of the neck.

In one embodiment, when treating sagittal shift, a modified BoNT/A may be administered to one or more affected neck muscle(s) comprising: the sternocleidomastoideus, the scalenus anterior, the scalenus medius, the levator scapulae, the longus colli, the submental complex (e.g. the digastric muscle, the geniohyoid muscle, the mylohyoid muscle, the mylohyoid boutonniere and/or the stylohyoid muscle), the obliquus capitis inferior, the semispinalis capitis, the trapezius pars descendens or the splenius capitis. Preferably, when treating sagittal shift, modified BoNT/A may be administered to an affected neck muscle comprising: the sternocleidomastoideus, the scalenus anterior, the scalenus medius, the levator scapulae, the longus colli, or the submental complex (e.g. the digastric muscle, the geniohyoid muscle, the mylohyoid muscle, the mylohyoid boutonniere and/or the stylohyoid muscle) on a first side (e.g. left side) of the neck and modified BoNT/A may be administered to an affected neck muscle comprising: the obliquus capitis inferior, the semispinalis capitis, the trapezius pars descendens or the splenius capitis on a second side (e.g. right side) of the neck.

A modified BoNT/A is administered by intramuscular injection at an affected neck muscle. One or more unit doses (e.g. at least two unit doses) of modified BoNT/A may be administered to an affected neck muscle. However, it is preferred that a single unit dose only is administered per affected neck muscle. Where the neck muscle is a sternocleidomastoideus two unit doses may be administered. For example, two unit doses may be administered to the left sternocleidomastoid and/or two unit doses may be administered to the right sternocleidomastoid. Where the neck muscle is a trapezius (e.g. trapezius pars descendens) two unit doses may be administered. For example, two unit doses may be administered to the left trapezius (e.g. the left upper trapezius) and/or two unit doses may be administered to the right trapezius (e.g. the right upper trapezius).

A unit dose may be administered to an affected neck muscle at a single injection site. Accordingly, in some embodiments, the modified BoNT/A is administered by way of a unit dose per injection site at an affected neck muscle.

However, it is preferred that less than a unit dose is administered at a single injection site, in which case the unit dose may be divided (equally or unequally) between two or more injection sites of the affected neck muscle. Thus, the modified BoNT/A may be administered to an affected neck muscle at two or more injection sites. In preferred embodiments, the modified BoNT/A is administered by way of less than a unit dose per injection site at an affected neck muscle. Advantageously, this may allow a clinician to contour the muscle and/or treat particularly affected regions of the muscle by administering more modified BoNT/A at said regions when compared to less affected regions.

Most preferably, a unit dose is administered per injection site.

The modified BoNT/A may be administered at a dose of 750-4,000 pg per injection site, wherein the modified BoNT/A comprises a BoNT/A light-chain and translocation domain, and a BoNT/B receptor binding domain ($H_c$ domain). Preferably, the modified BoNT/A is administered at a dose of 1,000 pg or 2,000 pg per injection site, wherein the modified BoNT/A comprises a BoNT/A light-chain and translocation domain, and a BoNT/B receptor binding domain ($H_c$ domain).

The modified BoNT/A may be administered at a dose of 31-166.4 Units per injection site, wherein the modified BoNT/A comprises a BoNT/A light-chain and translocation domain, and a BoNT/B receptor binding domain ($H_c$ domain). Preferably, the modified BoNT/A is administered at a dose of 41.6 Units or 83.2 Units per injection site, wherein the modified BoNT/A comprises a BoNT/A light-chain and translocation domain, and a BoNT/B receptor binding domain ($H_c$ domain).

The term "at least a single unit dose is administered" means at least substantially all of a single unit dose is administered. For example, a residual amount (e.g. up to 1%, 0.1% or 0.01%) of the unit dose may remain in a vial in which the modified BoNT/A has been reconstituted. However, preferably all of at least a single unit dose is administered (e.g. at one or more injection sites).

Potency of a modified BoNT/A for use according to the invention may be determined by a mouse $LD_{50}$ assay according to standard techniques. In said assay, 1 Unit is defined as an amount of the modified BoNT/A that corresponds to the calculated median lethal dose ($LD_{50}$) in mice. Preferably, the calculated median lethal intraperitoneal dose in mice.

Where a modified BoNT/A for use in the invention is modified BoNT/A comprising a BoNT/A light-chain and translocation domain, and a BoNT/B receptor binding domain ($H_c$ domain), an amount of a modified BoNT/A that corresponds to 1 Unit in said assay is preferably 24.04 pg.

Where a modified BoNT/A for use in the disclosure is a modified BoNT/A comprising a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue, an amount of a modified BoNT/A that corresponds to 1 Unit in said assay is preferably 8.44 pg.

The term "up to" when used in reference to a value (e.g. up to 170,000 pg) means up to and including the value recited. Thus, as an example, reference to administering "up to 170,000 pg" of modified BoNT/A encompasses administration of 170,000 pg of modified BoNT/A as well as administration of less than 170,000 pg of modified BoNT/A.

A unit dose may be expressed in terms of an amount of modified BoNT/A, in Units of modified BoNT/A, or a combination thereof.

In one embodiment, modified BoNT/A may be administered to one or more of the following neck muscles as follows at the following dosages:

| Neck Muscle | Dosage (Unit Dose) |
|---|---|
| Sternocleidomastoid | 1 x UD |
| Splenius capitis | 1 x UD |
| Splenius cervicis | 1 x UD |
| Trapezius | 1 x UD |
| Levator scapulae | 1 x UD |
| Scalenus medius | 1 x UD |
| Scalenus anterior | 1 x UD |
| Semispinalis capitis | 1 x UD |
| Longissimus | 1 x UD |

In one embodiment, modified BoNT/A may be administered to one or more of the following neck muscles as follows at the following dosages:

| Neck Muscle | Dosage (Unit Dose) |
|---|---|
| Sternocleidomastoid | 1 x UD |
| Splenius capitis | 1 x UD |
| Splenius cervicis | 1 x UD |
| Trapezius | 1 x UD |
| Levator scapulae | 1 x UD |
| Scalenus medius | 1 x UD |
| Scalenus anterior | 1 x UD |
| Semispinalis capitis | 1 x UD |
| Longissimus | 1 x UD |
| Posterior paravertebrals | 1 x UD |
| Submental complex | 1 x UD (e.g. per muscle) |

Modified BoNT/A may be administered to one or more of the following neck muscles as follows at the following dosages (as long as the total dose administered during the treatment does not exceed the upper limit of 170,000 pg or 7,070 Units when the modified BoNT/A comprises a BoNT/A light-chain and translocation domain, and a BoNT/B receptor binding domain ($H_c$ domain); or 80,000 pg or 9,480 Units when the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue):

| Neck Muscle | Dosage (Unit Dose) |
|---|---|
| Right Levator scapulae | 1 x UD |
| Left Levator scapulae | 1 x UD |
| Right Trapezius | 1 x UD |
| Left Trapezius | 1 x UD |
| Right Sternocleidomastoid | 1 x UD |
| Left Sternocleidomastoid | 1 x UD |
| Right Splenius capitis | 1 x UD |
| Left Splenius capitis | 1 x UD |
| Scalenus medius | 1 x UD |
| Scalenus anterior | 1 x UD |
| Right Semispinalis capitis | 1 x UD |
| Left Semispinalis capitis | 1 x UD |
| Right Longissimus capitis | 1 x UD |
| Left Longissimus capitis | 1 x UD |

Preferably, modified BoNT/A may be administered to the following neck muscles as follows at the following dosages (as long as the total dose administered during the treatment does not exceed the upper limit of 170,000 pg or 7,070 Units when the modified BoNT/A comprises a BoNT/A light-chain and translocation domain, and a BoNT/B receptor binding domain ($H_c$ domain); or 80,000 pg or 9,480 Units when the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue):

| Neck Muscle | Dosage (Unit Dose) |
| --- | --- |
| Right Levator scapulae | 1 x UD |
| Left Levator scapulae | 1 x UD |
| Right Trapezius | 1 x UD |
| Left Trapezius | 1 x UD |
| Right Sternocleidomastoid | 1 x UD |
| Left Sternocleidomastoid | 1 x UD |
| Right Splenius capitis | 1 x UD |
| Left Splenius capitis | 1 x UD |
| Scalenus medius | 1 x UD |
| Scalenus anterior | 1 x UD |
| Right Semispinalis capitis | 1 x UD |
| Left Semispinalis capitis | 1 x UD |
| Right Longissimus capitis | 1 x UD |
| Left Longissimus capitis | 1 x UD |

Preferably, modified BoNT/A may be administered to one or more of the following neck muscles as follows at the following dosages:

| Neck Muscle | Dosage (Unit Dose) |
| --- | --- |
| Either the right Levator scapulae or the left Levator scapulae | 1 x UD |
| Either the right Trapezius or the left Trapezius | 1 x UD |
| Either the right Sternocleidomastoid or the left Sternocleidomastoid | 1 x UD |
| Either the right Splenius capitis or the left Splenius capitis | 1 x UD |
| Either the Scalenus medius or the Scalenus anterior | 1 x UD |
| Either the right Semispinalis capitis or the left Semispinalis capitis | 1 x UD |
| Either the right Longissimus capitis or the left Longissimus capitis | 1 x UD |

Preferably, modified BoNT/A may be administered to the following neck muscles as follows at the following dosages:

| Neck Muscle | Dosage (Unit Dose) |
| --- | --- |
| Either the right Levator scapulae or the left Levator scapulae | 1 x UD |
| Either the right Trapezius or the left Trapezius | 1 x UD |

(continued)

| Neck Muscle | Dosage (Unit Dose) |
|---|---|
| Either the right Sternocleidomastoid or the left Sternocleidomastoid | 1 x UD |
| Either the right Splenius capitis or the left Splenius capitis | 1 x UD |
| Either the Scalenus medius or the Scalenus anterior | 1 x UD |
| Either the right Semispinalis capitis or the left Semispinalis capitis | 1 x UD |
| Either the right Longissimus capitis or the left Longissimus capitis | 1 x UD |

As used herein, the terms "right" and "left" take on their normal meaning. For example, a subject's right levator scapulae will be the levator scapulae that is on the subject's right hand side, while the subject's left levator scapulae will be the levator scapulae that is on the subject's left hand side.

The total number of unit doses administered in a given treatment may be up to 10x the unit dose or up to 7x the unit dose. The total number of unit doses may be divided according to the affected neck muscles treated, for example, in one embodiment, when the number of doses to be delivered during treatment is 1x the unit dose, then only one affected neck muscle may be treated, however, if the total is 2x unit doses then two affected neck muscles may be treated. The total number of unit doses administered may be up to 9x, 8x, 7x or 6x. The total number of unit doses administered may be at least 2x, 3x, 4x, 5x, 6x, 7x the unit dose, preferably at least 2x. The total number of unit doses administered may be 1x to 10x, or 5x to 10x, preferably 7x to 10x.

The total number of unit doses administered in a given treatment may be up to 20x or 15x (preferably up to 14x or up to 7x) the unit dose as long as the total dose administered during the treatment does not exceed the upper limit of 170,000 pg or 7,070 Units when the modified BoNT/A comprises a BoNT/A light-chain and translocation domain, and a BoNT/B receptor binding domain ($H_c$ domain); or 80,000 pg or 9,480 Units when the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue. Accordingly, the total number of unit doses administered may be up to 13x, 12x, 10x, 9x, 8x, 7x or 6x as long as the total dose administered during the treatment does not exceed the upper limit of 170,000 pg or 7,070 Units when the modified BoNT/A comprises a BoNT/A light-chain and translocation domain, and a BoNT/B receptor binding domain ($H_c$ domain); or 80,000 pg or 9,480 Units when the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue. The total number of unit doses administered may be at least 2x, 3x, 4x, 5x, 6x, 7x, 8x, 9x, 10x the unit dose, preferably at least 2x (e.g. 7x or 14x) as long as the total dose administered during the treatment does not exceed the upper limit of 170,000 pg or 7,070 Units when the modified BoNT/A comprises a BoNT/A light-chain and translocation domain, and a BoNT/B receptor binding domain ($H_c$ domain); or 80,000 pg or 9,480 Units when the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue. The total number of unit doses administered may be 1x to 20x (e.g. 12x to 16x), 1x to 15x, 1x to 14x, or 5x to 14x, preferably 7x to 14x as long as the total dose administered during the treatment does not exceed the upper limit of 170,000 pg or 7,070 Units when the modified BoNT/A comprises a BoNT/A light-chain and translocation domain, and a BoNT/B receptor binding domain ($H_c$ domain); or 80,000 pg or 9,480 Units when the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: substitution of an acidic surface exposed amino acid residue with a basic amino acid residue; substitution of an acidic surface exposed amino acid residue with an uncharged amino acid residue; substitution of an uncharged surface exposed amino acid residue with a basic amino acid residue; insertion of a basic amino acid residue; and deletion of an acidic surface exposed amino acid residue.

The skilled person will take into consideration when a subject has recently had (or is subsequently having) additional treatment with a clostridial neurotoxin (e.g. BoNT), e.g. as part of a cosmetic treatment or treatment for a different indication. Using techniques routine in the art, the skilled person will adapt the present treatment regimen accordingly.

A modified BoNT/A of the invention preferably has a longer duration of action (e.g. an improvement in one or more symptoms of at least 5%, 10%, 25%, or 50%) when compared to unmodified BoNT/A (e.g. Dysport®). Said duration of action may be at least 1.25x, 1.5x, 1.75x, 2.0x, or 2.25x greater. The duration of action of modified BoNT/A may be between 6 and 9 months. For example, a duration of action may be at least: 4.5 months (from onset), 5.0 months, 5.5 months, 6 months, 6.5 months, 7.0 months, 7.5 months, 8.0 months, 8.5 months or 9.0 months. In particular embodiments, a duration of action may be greater than 9.0 months.

Where administration is to a plurality of affected neck muscles, preferably said administration occurs in the same treatment session.

Treatment may be repeated at an appropriate time period following administration of modified BoNT/A. Given that the duration of action is approximately twice that of unmodified BoNT/A (e.g. Dysport®) there are suitably longer periods between subsequent administrations than when a subject is treated with unmodified BoNT/A (e.g. Dysport®). A subject may be readministered a modified BoNT/A in accordance with the present invention at least 18, 20, 25 or 30 weeks following a previous administration. For example, a subject may be readministered a modified BoNT/A in accordance with the present invention at least 18-45 weeks, preferably 20-35 weeks following a previous administration.

The efficacy of the treatment (including severity of a subject's symptom(s)) may be assessed using the Toronto Western Spasmodic Torticollis Rating Scale (TWSTRS) reviewed in Jost et al. 2013 J Neural Transm (Vienna) 120(3):487-496. The TWSTRS is a composite scale consisting of the TWSTRS-Severity scale, the TWSTRS-Disability scale and the TWSTRS-Pain scale. A higher score on the TWSTRS indicates more severe disease. The TWSTRS-Severity scale includes the following items: A. maximal excursion (rotation, tilt, anterocollis or retrocollis, lateral shift, sagittal shift), B. duration factor, C. effect of sensory tricks, D. shoulder elevation/anterior displacement, E. range of motion (without the aid of sensory tricks), F. time (up to 60 s that the patient is able to maintain the head within 10° of the neutral position without the use of sensory tricks). The sum of A to F amounts to a maximum score of 35 with the duration factor weighted twice. The TWSTRS-Disability scale is a six-item scale that comprises an assessment of performances of daily activities which may be possibly affected by CD: work performance (job or domestic), activities of daily living (feeding, dressing, hygiene), driving, reading, watching television, and leisure activities outside the home. Each item is rated on a 6-point scale (0 = no difficulty, 5 = highest degree of disability) and the sum of each item amounts to a maximum score of 30. The TWSTRS-Pain scale consists of a severity score for the patient's usual, worst, and best pain in the last week, as well as a duration component and an assessment of the contribution of pain to disability. The score range is between 0 and 20, with 20 assigned to the highest possible experienced pain.

A "subject" as used herein may be a mammal, such as a human or other mammal. Preferably "subject" means a human subject. A "subject" is preferably an adult subject, i.e. a subject at least 18 years old. The terms "subject" and "patient" are used synonymously herein.

The term "treat" or "treating" as used herein encompasses prophylactic treatment (e.g. to prevent onset of a disorder) as well as corrective treatment (treatment of a subject already suffering from a disorder). Preferably "treat" or "treating" as used herein means corrective treatment. The term "treat" or "treating" as used herein refers to the disorder and/or a symptom thereof.

Suitable modified BoNT/A polypeptides (and nucleotide sequences encoding the same, where present) are described in WO 2015/004461 A1 and WO 2017/191315.

BoNT/A is one example of a clostridial neurotoxin produced by bacteria in the genus *Clostridia*. Other examples of such clostridial neurotoxins include those produced by *C. tetani* (TeNT) and by *C. botulinum* (BoNT) serotypes B-G, as well as those produced by *C. baratii* and C. *butyricum.* Said neurotoxins are highly potent and specific and can poison neurons and other cells to which they are delivered. The clostridial toxins are some of the most potent toxins known. By way of example, botulinum neurotoxins have median lethal dose ($LD_{50}$) values for mice ranging from 0.5 to 5 ng/kg, depending on the serotype. Both tetanus and botulinum toxins act by inhibiting the function of affected neurons, specifically the release of neurotransmitters. While botulinum toxin acts at the neuromuscular junction and inhibits cholinergic transmission in the peripheral nervous system, tetanus toxin acts in the central nervous system.

In nature, clostridial neurotoxins (including BoNT/A) are synthesised as a single-chain polypeptide that is modified post-translationally by a proteolytic cleavage event to form two polypeptide chains joined together by a disulphide bond. Cleavage occurs at a specific cleavage site, often referred to as the activation site, that is located between the cysteine residues that provide the inter-chain disulphide bond. It is this di-chain form that is the active form of the toxin. The two chains are termed the heavy chain (H-chain), which has a molecular mass of approximately 100 kDa, and the light chain (L-chain), which has a molecular mass of approximately 50 kDa. The H-chain comprises an N-terminal translocation component ($H_N$ domain) and a C-terminal targeting component ($H_C$ domain). The cleavage site is located between the L-chain and the translocation domain components. Following binding of the $H_C$ domain to its target neuron and internalisation of the bound toxin into the cell via an endosome, the $H_N$ domain translocates the L-chain across the endosomal membrane and into the cytosol, and the L-chain provides a protease function (also known as a non-cytotoxic protease).

Non-cytotoxic proteases act by proteolytically cleaving intracellular transport proteins known as SNARE proteins (e.g. SNAP-25, VAMP, or Syntaxin) - see Gerald K (2002) "Cell and Molecular Biology" (4th edition) John Wiley & Sons, Inc. The acronym SNARE derives from the term Soluble NSF Attachment Receptor, where NSF means N-ethylmaleimide-Sensitive Factor. SNARE proteins are integral to intracellular vesicle fusion, and thus to secretion of molecules via vesicle transport from a cell. The protease function is a zinc-dependent endopeptidase activity and exhibits a high substrate specificity for SNARE proteins. Accordingly, once delivered to a desired target cell, the non-cytotoxic protease is capable of inhibiting cellular secretion from the target cell. The L-chain proteases of clostridial toxins are non-cytotoxic proteases that cleave SNARE proteins.

In view of the ubiquitous nature of SNARE proteins, clostridial neurotoxins such as botulinum toxin have been successfully employed in a wide range of therapies.

For further details on the genetic basis of toxin production in *Clostridium botulinum* and C. *tetani, see* Henderson et al (1997) in The Clostridia: Molecular Biology and Pathogenesis, Academic press.

As discussed above, clostridial neurotoxins are formed from two polypeptide chains, the heavy chain (H-chain), which has a molecular mass of approximately 100 kDa, and the light chain (L-chain), which has a molecular mass of approximately 50 kDa. The H-chain comprises a C-terminal targeting component (receptor binding domain or $H_C$ domain) and an N-terminal translocation component ($H_N$ domain).

Clostridial neurotoxin domains are described in more detail below.

Examples of L-chain reference sequences include:

Botulinum type A neurotoxin: amino acid residues 1-448
Botulinum type B neurotoxin: amino acid residues 1-440

The above-identified reference sequences should be considered a guide, as slight variations may occur according to sub-serotypes. By way of example, US 2007/0166332 cites slightly different clostridial sequences:

Botulinum type A neurotoxin: amino acid residues M1-K448
Botulinum type B neurotoxin: amino acid residues M1-K441

The translocation domain is a fragment of the H-chain of a clostridial neurotoxin approximately equivalent to the amino-terminal half of the H-chain, or the domain corresponding to that fragment in the intact H-chain.

Examples of reference translocation domains include:

Botulinum type A neurotoxin - amino acid residues (449-871)
Botulinum type B neurotoxin - amino acid residues (441-858)

The above-identified reference sequence should be considered a guide as slight variations may occur according to sub-serotypes. By way of example, US 2007/0166332 cites slightly different clostridial sequences:

Botulinum type A neurotoxin - amino acid residues (A449-K871)
Botulinum type B neurotoxin - amino acid residues (A442-S858)

In the context of the present invention, a variety of BoNT/A $H_N$ regions comprising a translocation domain can be useful in aspects of the present invention. The $H_N$ regions from the heavy-chain of BoNT/A are approximately 410-430 amino acids in length and comprise a translocation domain. Research has shown that the entire length of a $H_N$ region from a clostridial neurotoxin heavy-chain is not necessary for the translocating activity of the translocation domain. Thus, aspects of this embodiment can include BoNT/A $H_N$ regions comprising a translocation domain having a length of, for example, at least 350 amino acids, at least 375 amino acids, at least 400 amino acids or at least 425 amino acids. Other aspects of this embodiment can include BoNT/A $H_N$ regions comprising a translocation domain having a length of, for example, at most 350 amino acids, at most 375 amino acids, at most 400 amino acids or at most 425 amino acids.

The term $H_N$ embraces naturally-occurring BoNT/A $H_N$ portions, and modified BoNT/A $H_N$ portions having amino acid sequences that do not occur in nature and/or synthetic amino acid residues. Preferably, said modified BoNT/A $H_N$ portions still demonstrate the above-mentioned translocation function.

Examples of clostridial neurotoxin receptor binding domain ($H_C$) reference sequences include:

BoNT/A - N872-L1296

BoNT/B - E859-E1291

The ~50 kDa $H_C$ domain of a clostridial neurotoxin (such as a BoNT) comprises two distinct structural features that are referred to as the $H_{CC}$ and $H_{CN}$ domains, each typically of ~25 kDa. Amino acid residues involved in receptor binding are believed to be primarily located in the $H_{CC}$ domain. The $H_C$ domain of a native clostridial neurotoxin may comprise approximately 400-440 amino acid residues. This fact is confirmed by the following publications: Umland TC (1997) Nat. Struct. Biol. 4: 788-792; Herreros J (2000) Biochem. J. 347: 199-204; Halpern J (1993) J. Biol. Chem. 268: 15, pp. 11188-11192; Rummel A (2007) PNAS 104: 359-364; Lacey DB (1998) Nat. Struct. Biol. 5: 898-902; Knapp (1998) Am. Cryst. Assoc. Abstract Papers 25: 90; Swaminathan and Eswaramoorthy (2000) Nat. Struct. Biol. 7: 1751-1759; and Rummel A (2004) Mol. Microbiol. 51(3), 631-643.

Examples of (reference) $H_{CN}$ domains include:

Botulinum type A neurotoxin - amino acid residues (872-1110)
Botulinum type B neurotoxin - amino acid residues (859-1097)

The above sequence positions may vary a little according to serotype/ sub-type, and further examples of (reference) $H_{CN}$ domains include:

Botulinum type A neurotoxin - amino acid residues (874-1110)
Botulinum type B neurotoxin - amino acid residues (861-1097)

Examples of (reference) $H_{CC}$ domains include:

Botulinum type A neurotoxin - amino acid residues (Y1111-L1296)
Botulinum type B neurotoxin - amino acid residues (Y1098-E1291)

The L-chain and $H_N$ domain (optionally including a complete or partial activation loop, e.g. a complete activation loop when the modified BoNT/A is in a single-chain form and a cleaved/partial activation loop when in a di-chain form) may be collectively referred to as an $LH_N$ domain. The $LH_N$ domain thus does not further comprise an $H_C$ domain.

A modified BoNT/A for use in the present disclosure may be one that comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277. Such modified BoNT/A demonstrates a reduction in, or absence of, side effects compared to the use of known BoNT/A. The increased tissue retention properties of the modified BoNT/A also provides increased potency and/or duration of action and can allow for reduced dosages to be used compared to known clostridial toxin therapeutics (or increased dosages without any additional adverse effects), thus providing further advantages.

The modification may be a modification when compared to unmodified BoNT/A shown as SEQ ID NO: 2, wherein the amino acid residue numbering is determined by alignment with SEQ ID NO: 2. As the presence of a methionine residue at position 1 of SEQ ID NO: 2 (as well as the SEQ ID NOs corresponding to modified BoNT/A polypeptides described herein) is optional, the skilled person will take the presence/absence of the methionine residue into account when determining amino acid residue numbering. For example, where SEQ ID NO: 2 includes a methionine, the position numbering will be as defined above (e.g. ASN 886 will be ASN 886 of SEQ ID NO: 2). Alternatively, where the methionine is absent from SEQ ID NO: 2 the amino acid residue numbering should be modified by -1 (e.g. ASN 886 will be ASN 885 of SEQ ID NO: 2). Similar considerations apply when the methionine at position 1 of the other polypeptide sequences described herein is present/absent, and the skilled person will readily determine the correct amino acid residue numbering using techniques routine in the art.

An alignment described herein for determining amino acid residue numbering may be carried out using any of the methods described herein for determining sequence homology and/or % sequence identity.

The amino acid residue(s) indicated for modification are surface exposed amino acid residue(s).

A modified BoNT/A may comprise a modification at one or more amino acid residue(s) selected from: ASN 886, ASN 930, ASN 954, SER 955, GLN 991, ASN 1025, ASN 1026, ASN 1052, ASN 1188, ASP 1213, GLY 1215, ASN 1216, GLN 1229, ASN 1242, ASN 1243, SER 1274 and THR 1277. The modified BoNT/A may be encoded by a nucleic acid sequence having at least 70% sequence identity to a nucleic acid sequence selected from SEQ ID NOs: 3, 5, 7, and 9. For example, a nucleic acid sequence having at least 80%, 90%, 95% or 99.9% sequence identity to a nucleic acid sequence selected from SEQ ID NOs: 3, 5, 7, and 9. Preferably, a modified BoNT/A for use in the disclosure may be encoded by a nucleic acid comprising (or consisting of) SEQ ID NO: 3, 5, 7 or 9. The modified BoNT/A may comprise a polypeptide sequence having at least 70% sequence identity to a polypeptide sequence selected from SEQ ID NOs: 4, 6, 8, and 10. For example, a polypeptide sequence having at least 80%, 90%, 95% or 99.9% sequence identity to a polypeptide sequence selected from SEQ ID NOs: 4, 6, 8, and 10. Preferably, a modified BoNT/A for use in the disclosure may comprise (more preferably consist of) a polypeptide sequence selected from SEQ ID NOs: 4, 6, 8, and 10.

The term "one or more amino acid residue(s)" when used in the context of modified BoNT/A preferably means at least 2, 3, 4, 5, 6 or 7 of the indicated amino acid residue(s). Thus, a modified BoNT/A may comprise at least 2, 3, 4, 5, 6 or 7 (preferably 7) modifications at the indicated amino acid residue(s). A modified BoNT/A may comprise 1-30, 3-20, or 5-10 amino acid modifications. More preferably, the term "one or more amino acid residue(s)" when used in the context of modified BoNT/A means all of the indicated amino acid residue(s).

Preferably, beyond the one or more amino acid modification(s) at the indicated amino acid residue(s), the modified BoNT/A does not contain any further amino acid modifications when compared to SEQ ID NO: 2.

Most preferably, a modified BoNT/A comprises (more preferably consists of) a modification at one or more amino acid residue(s) selected from: ASN 886, ASN 930, SER 955, GLN 991, ASN 1026, ASN 1052, and GLN 1229. The modified BoNT/A may be encoded by a nucleic acid sequence having at least 70% sequence identity to SEQ ID NO: 3. For example, a nucleic acid sequence having at least 80%, 90%, 95% or 99.9% sequence identity to SEQ ID NO: 3. Preferably, a modified BoNT/A for use in the disclosure may be encoded by a nucleic acid comprising (or consisting of) SEQ ID NO: 3. The modified BoNT/A may comprise a polypeptide sequence having at least 70% sequence identity to SEQ ID NO: 4. For example, a polypeptide sequence having at least 80%, 90%, 95% or 99.9% sequence identity to SEQ ID NO: 4.

Preferably, a modified BoNT/A for use in the disclosure may comprise (more preferably consist of) SEQ ID NO: 4.

The modification may be selected from:

    i. substitution of an acidic surface exposed amino acid residue with a basic amino acid residue;
    ii. substitution of an acidic surface exposed amino acid residue with an uncharged amino acid residue;
    iii. substitution of an uncharged surface exposed amino acid residue with a basic amino acid residue;
    iv. insertion of a basic amino acid residue; and
    v. deletion of an acidic surface exposed amino acid residue.

A modification as indicated above results in a modified BoNT/A that has an increased positive surface charge and increased isoelectric point when compared to the corresponding unmodified BoNT/A.

The isoelectric point (pI) is a specific property of a given protein. As is well known in the art, proteins are made from a specific sequence of amino acids (also referred to when in a protein as amino acid residues). Each amino acid of the standard set of twenty has a different side chain (or R group), meaning that each amino acid residue in a protein displays different chemical properties such as charge and hydrophobicity. These properties may be influenced by the surrounding chemical environment, such as the temperature and pH. The overall chemical characteristics of a protein will depend on the sum of these various factors.

Certain amino acid residues (detailed below) possess ionisable side chains that may display an electric charge depending on the surrounding pH. Whether such a side chain is charged or not at a given pH depends on the pKa of the relevant ionisable moiety, wherein pKa is the negative logarithm of the acid dissociation constant (Ka) for a specified proton from a conjugate base.

For example, acidic residues such as aspartic acid and glutamic acid have side chain carboxylic acid groups with pKa values of approximately 4.1 (precise pKa values may depend on temperature, ionic strength and the microenvironment of the ionisable group). Thus, these side chains exhibit a negative charge at a pH of 7.4 (often referred to as "physiological pH"). At low pH values, these side chains will become protonated and lose their charge.

Conversely, basic residues such as lysine and arginine have nitrogen-containing side chain groups with pKa values of approximately 10-12. These side chains therefore exhibit a positive charge at a pH of 7.4. These side chains will become de-protonated and lose their charge at high pH values.

The overall (net) charge of a protein molecule therefore depends on the number of acidic and basic residues present in the protein (and their degree of surface exposure) and on the surrounding pH. Changing the surrounding pH changes the overall charge on the protein. Accordingly, for every protein there is a given pH at which the number of positive and negative charges is equal and the protein displays no overall net charge. This point is known as the isoelectric point (pI). The isoelectric point is a standard concept in protein biochemistry with which the skilled person would be familiar.

The isoelectric point (pI) is therefore defined as the pH value at which a protein displays a net charge of zero. An increase in pI means that a higher pH value is required for the protein to display a net charge of zero. Thus, an increase in pI represents an increase in the net positive charge of a protein at a given pH. Conversely, a decrease in pI means that a lower pH value is required for the protein to display a net charge of zero. Thus, a decrease in pI represents a decrease in the net positive charge of a protein at a given pH.

Methods of determining the pI of a protein are known in the art and would be familiar to a skilled person. By way of example, the pI of a protein can be calculated from the average pKa values of each amino acid present in the protein ("calculated pI"). Such calculations can be performed using computer programs known in the art, such as the Compute pI/MW Tool from ExPASy (.expasy.org/compute_pi/), which is the preferred method for calculating pI in accordance with the present invention. Comparisons of pI values between different molecules should be made using the same calculation technique/program.

Where appropriate, the calculated pI of a protein can be confirmed experimentally using the technique of isoelectric focusing ("observed pI"). This technique uses electrophoresis to separate proteins according to their pI. Isoelectric focusing is typically performed using a gel that has an immobilised pH gradient. When an electric field is applied, the protein migrates through the pH gradient until it reaches the pH at which it has zero net charge, this point being the pI of the protein. Results provided by isoelectric focusing are typically relatively low-resolution in nature, and thus the present inventors believe that results provided by calculated pI (as described above) are more appropriate to use.

Throughout the present specification, "pI" means "calculated pI" unless otherwise stated.

The pI of a protein may be increased or decreased by altering the number of basic and/or acidic groups displayed on its surface. This can be achieved by modifying one or more amino acids of the protein. For example, an increase in pI may be provided by reducing the number of acidic residues, or by increasing the number of basic residues.

A modified BoNT/A of the invention may have a pI value that is at least 0.2, 0.4, 0.5 or 1 pI units higher than that of an unmodified BoNT/A (e.g. SEQ ID NO: 2). Preferably, a modified BoNT/A may have a pI of at least 6.6, e.g. at least 6.8.

The properties of the 20 standard amino acids are indicated in the table below:

| Amino Acid | | | Side Chain |
| --- | --- | --- | --- |
| Aspartic acid | Asp | D | Charged (acidic) |
| Glutamic acid | Glu | E | Charged (acidic) |
| Arginine | Arg | R | Charged (basic) |
| Lysine | Lys | K | Charged (basic) |
| Histidine | His | H | Uncharged (polar) |
| Asparagine | Asn | N | Uncharged (polar) |
| Glutamine | Gln | Q | Uncharged (polar) |
| Serine | Ser | S | Uncharged (polar) |
| Threonine | Thr | T | Uncharged (polar) |
| Tyrosine | Tyr | Y | Uncharged (polar) |
| Methionine | Met | M | Uncharged (polar) |
| Tryptophan | Trp | W | Uncharged (polar) |
| Cysteine | Cys | C | Uncharged (polar) |
| Alanine | Ala | A | Uncharged (hydrophobic) |
| Glycine | Gly | G | Uncharged (hydrophobic) |
| Valine | Val | V | Uncharged (hydrophobic) |
| Leucine | Leu | L | Uncharged (hydrophobic) |
| Isoleucine | Ile | I | Uncharged (hydrophobic) |
| Proline | Pro | P | Uncharged (hydrophobic) |
| Phenylalanine | Phe | F | Uncharged (hydrophobic) |

The following amino acids are considered charged amino acids: aspartic acid (negative), glutamic acid (negative), arginine (positive), and lysine (positive).

At a pH of 7.4, the side chains of aspartic acid (pKa 3.1) and glutamic acid (pKa 4.1) have a negative charge, while the side chains of arginine (pKa 12.5) and lysine (pKa 10.8) have a positive charge. Aspartic acid and glutamic acid are referred to as acidic amino acid residues. Arginine and lysine are referred to as basic amino acid residues.

The following amino acids are considered uncharged, polar (meaning they can participate in hydrogen bonding) amino acids: asparagine, glutamine, histidine, serine, threonine, tyrosine, cysteine, methionine, and tryptophan.

The following amino acids are considered uncharged, hydrophobic amino acids: alanine, valine, leucine, isoleucine, phenylalanine, proline, and glycine.

In an amino acid insertion, an additional amino acid residue (one that is not normally present) is incorporated into the BoNT/A polypeptide sequence, thus increasing the total number of amino acid residues in said sequence. In an amino acid deletion, an amino acid residue is removed from the clostridial toxin amino acid sequence, thus reducing the total number of amino acid residues in said sequence.

Preferably, the modification is a substitution, which advantageously maintains the same number of amino acid residues in the modified BoNT/A. In an amino acid substitution, an amino acid residue that forms part of the BoNT/A polypeptide sequence is replaced with a different amino acid residue. The replacement amino acid residue may be one of the 20 standard amino acids, as described above. Alternatively, the replacement amino acid in an amino acid substitution may be a non-standard amino acid (an amino acid that is not part of the standard set of 20 described above). By way of example, the replacement amino acid may be a basic non-standard amino acid, e.g. L-Ornithine, L-2-amino-3-guanidinopropionic acid, or D-isomers of Lysine, Arginine and Ornithine). Methods for introducing non-standard amino acids into proteins are known in the art and include recombinant protein synthesis using *E. coli* auxotrophic expression hosts.

In one embodiment, the substitution is selected from: substitution of an acidic amino acid residue with a basic amino acid residue, substitution of an acidic amino acid residue with an uncharged amino acid residue, and substitution of an uncharged amino acid residue with a basic amino acid residue. In one embodiment, wherein the substitution is a substitution of an acidic amino acid residue with an uncharged amino acid residue, the acidic amino acid residue is replaced with its corresponding uncharged amide amino acid residue (i.e. aspartic acid is replaced with asparagine, and glutamic acid is replaced with glutamine).

Preferably, the basic amino acid residue is a lysine residue or an arginine residue. In other words, the substitution is substitution with lysine or arginine. Most preferably, the modification is substitution with lysine.

Following modification in accordance with the invention, the modified BoNT/A is capable of binding to the target cell receptors that unmodified BoNT/A (e.g. SEQ ID NO: 2) binds.

A modified BoNT/A for use in the disclosure may comprise between 4 and 40 amino acid modifications located in the clostridial toxin $H_{CN}$ domain. Said modified BoNT/A preferably also has pI of at least 6.6. Said modified BoNT/A preferably comprises modifications of at least 4 amino acids selected from: ASN 886, ASN 930, ASN 954, SER 955, GLN 991, ASN 1025, ASN 1026, and ASN 1052, wherein said modification comprises substitution of the amino acids with a lysine residue or an arginine residue. For example, said modified BoNT/A or fragment thereof may comprise modifications of at least 5 amino acids selected from: ASN 886, ASN 930, ASN 954, SER 955, GLN 991, ASN 1025, ASN 1026, ASN 1052, and GLN 1229, wherein said modification comprises substitution of the amino acids with a lysine residue or an arginine residue.

The term "modified BoNT/A" or "chimeric neurotoxin" as used herein preferably means a neurotoxin comprising (preferably consisting of) a clostridial neurotoxin light-chain and translocation domain ($H_N$ domain) from a first clostridial neurotoxin serotype and a receptor binding domain ($H_C$ domain) originating from a second different clostridial neurotoxin serotype. Specifically, a modified BoNT/A for use in the invention comprises a botulinum neurotoxin A (BoNT/A) light-chain and translocation domain ($H_N$ domain), and a BoNT/B receptor binding domain ($H_C$ domain). The BoNT/A $LH_N$ domain of the modified BoNT/A is covalently linked to the BoNT/B $H_C$ domain. The modified BoNT/A of the invention may be referred to as a chimeric botulinum neurotoxin. Said modified BoNT/A is also referred to herein as "BoNT/AB", "mrBoNT/AB" or a "BoNT/AB chimera".

A modified BoNT/A for use in the invention comprises a BoNT/A light-chain and translocation domain (a BoNT/A $LH_N$ domain), and a BoNT/B $H_C$ domain. The BoNT/A $LH_N$ domain is covalently linked to the BoNT/B $H_C$ domain. Said modified BoNT/A is also referred to herein as "BoNT/AB" or a "BoNT/AB chimera".

The C-terminal amino acid residue of the $LH_N$ domain may correspond to the first amino acid residue of the $3_{10}$ helix separating the $LH_N$ and $H_C$ domains of BoNT/A, and the N-terminal amino acid residue of the $H_C$ domain may correspond to the second amino acid residue of the $3_{10}$ helix separating the $LH_N$ and $H_C$ domains in BoNT/B.

An example of a BoNT/B polypeptide sequence is provided as SEQ ID NO: 16 (UniProt accession number B1INP5).

Reference herein to the "first amino acid residue of the $3_{10}$ helix separating the $LH_N$ and $H_C$ domains of BoNT/A" means the N-terminal residue of the $3_{10}$ helix separating the $LH_N$ and $H_C$ domains.

Reference herein to the "second amino acid residue of the $3_{10}$ helix separating the $LH_N$ and $H_C$ domains of BoNT/B" means the amino acid residue following the N-terminal residue of the $3_{10}$ helix separating the $LH_N$ and $H_C$ domains.

A "$3_{10}$ helix" is a type of secondary structure found in proteins and polypeptides, along with $\alpha$-helices, $\beta$-sheets and reverse turns. The amino acids in a $3_{10}$ helix are arranged in a right-handed helical structure where each full turn is completed by three residues and ten atoms that separate the intramolecular hydrogen bond between them. Each amino acid corresponds to a 120° turn in the helix (i.e., the helix has three residues per turn), and a translation of 2.0 Å (= 0.2 nm) along the helical axis, and has 10 atoms in the ring formed by making the hydrogen bond. Most importantly, the N-H group of an amino acid forms a hydrogen bond with the C = O group of the amino acid three residues earlier; this repeated $i + 3 \rightarrow i$ hydrogen bonding defines a $3_{10}$ helix. A $3_{10}$ helix is a standard concept in structural biology with which the skilled person is familiar.

This $3_{10}$ helix corresponds to four residues which form the actual helix and two cap (or transitional) residues, one at each end of these four residues. The term "$3_{10}$ helix separating the $LH_N$ and $H_C$ domains" as used herein consists of those 6 residues.

Through carrying out structural analyses and sequence alignments, a $3_{10}$ helix separating the $LH_N$ and $H_C$ domains was identified. This $3_{10}$ helix is surrounded by an $\alpha$-helix at its N-terminus (i.e. at the C-terminal part of the $LH_N$ domain) and by a $\beta$-strand at its C-terminus (i.e. at the N-terminal part of the $H_C$ domain). The first (N-terminal) residue (cap or transitional residue) of the $3_{10}$ helix also corresponds to the C-terminal residue of this $\alpha$-helix.

The $3_{10}$ helix separating the $LH_N$ and $H_C$ domains can be for example determined from publicly available crystal structures of botulinum neurotoxins, for example 3BTA w.rcsb.org/pdb/explore/explore.do?structureId=3BTA) and 1EPW w.rcsb.org/pdb/explore/explore.do?structureId=1EPW) for botulinum neurotoxins A1 and B1 respectively.

*In silico* modelling and alignment tools which are publicly available can also be used to determine the location of the $3_{10}$ helix separating the $LH_N$ and $H_C$ domains in other neurotoxins, for example the homology modelling servers LOOPP (Learning, Observing and Outputting Protein Patterns,p.org), PHYRE (Protein Homology/analogY Recognition Engine,w.sbg.bio.ic.ac.uk/phyre2/) and Rosetta w.rosettacommons.org/), the protein superposition server SuperPose t.biology.ualberta.ca/superpose/), the alignment program Clustal Omega w.clustal.org/omega/), and a number of other tools/services listed at the Internet Resources for Molecular and Cell Biologists I-tools.ca/). In particular, the region around the "$H_N/H_{CN}$" junction may be structurally highly conserved which renders it an ideal region to superimpose different serotypes.

For example, the following methodology may be used to determine the sequence of this $3_{10}$ helix in other neurotoxins:

1. The structural homology modelling tool LOOP p.org) may be used to obtain a predicted structure of other BoNT serotypes based on the BoNT/A1 crystal structure (3BTA.pdb);

2. The structural (pdb) files thus obtained may be edited to include only the N-terminal end of the $H_{CN}$ domain and about 80 residues before it (which are part of the $H_N$ domain), thereby retaining the "$H_N/H_{CN}$" region which is structurally highly conserved;

3. The protein superposition server SuperPose t.biology.ualberta.ca/superpose/) may be used to superpose each serotype onto the 3BTA.pdb structure;

4. The superposed pdb files may be inspected to locate the $3_{10}$ helix at the start of the $H_C$ domain of BoNT/A1, and corresponding residues in the other serotype may then identified.

5. The other BoNT serotype sequences may be aligned with Clustal Omega in order to check that corresponding residues were correct.

Examples of $LH_N$, $H_C$ and $3_{10}$ helix domains determined by this method are presented below:

| Neurotoxin | Accession Number (Plus Sequence Version after Decimal) | $LH_N$ | $H_C$ | $3_{10}$ helix |
|---|---|---|---|---|
| BoNT/A1 (SEQ ID NO: 2) | A5HZZ9.1 | 1-872 | 873-1296 | ${}^{872}$NIINTS${}^{877}$ |
| BoNT/A2 | X73423.3 | 1-872 | 873-1296 | ${}^{872}$NIVNTS${}^{877}$ |
| BoNT/A3 | DQ185900.1 (aka Q3LRX9.1) | 1-872 | 873-1292 | ${}^{872}$NIVNTS${}^{877}$ |
| BoNT/A4 | EU341307.1 (aka Q3LRX8.1) | 1-872 | 873-1296 | ${}^{872}$NITNAS${}^{877}$ |
| BoNT/A5 | EU679004.1 (aka C1IPK2.1) | 1-872 | 873-1296 | ${}^{872}$NIINTS${}^{877}$ |
| BoNT/A6 | FJ981696.1 | 1-872 | 873-1296 | ${}^{872}$NIINTS${}^{877}$ |
| BoNT/A7 | JQ954969.1 (aka K4LN57.1) | 1-872 | 873-1296 | ${}^{872}$NIINTS${}^{877}$ |
| BoNT/A8 | KM233166.1 | 1-872 | 873-1297 | ${}^{872}$NITNTS${}^{877}$ |
| BoNT/B1 (SEQ ID NO: 16) | B1INP5.1 | 1-859 | 860-1291 | ${}^{859}$EILNNI${}^{864}$ |

(continued)

| Neurotoxin | Accession Number (Plus Sequence Version after Decimal) | $LH_N$ | $H_C$ | $3_{10}$ helix |
|---|---|---|---|---|
| BoNT/B2 | AB084152.1 (aka Q8GR96.1) | 1-859 | 860-1291 | [859]EILNNI[864] |
| BoNT/B3 | EF028400.1 (aka A2I2S2.1) | 1-859 | 860-1291 | [859]EILNNI[864] |
| BoNT/B4 | EF051570.1 (aka A2I2WO.1) | 1-859 | 860-1291 | [859]EILNNI[864] |
| BoNT/B5 | EF033130.1 (aka A2I2U6.1) | 1-859 | 860-1291 | [859]DILNNI[864] |
| BoNT/B6 | AB302852.1 (aka A8R089.1) | 1-859 | 860-1291 | [859]EILNNI[864] |
| BoNT/B7 | JQ354985.1 (aka H9CNK9.1) | 1-859 | 860-1291 | [859]EILNNI[864] |
| BoNT/B8 | JQ964806.1 (aka I6Z8G9.1) | 1-859 | 860-1292 | [859]EILNNI[864] |

Using structural analysis and sequence alignments, it was found that the β-strand following the $3_{10}$ helix separating the $LH_N$ and $H_C$ domains is a conserved structure in all botulinum and tetanus neurotoxins and starts at the 8th residue when starting from the first residue of the $3_{10}$ helix separating the $LH_N$ and $H_C$ domains (e.g., at residue 879 for BoNT/A1).

A BoNT/AB chimera may comprise an $LH_N$ domain from BoNT/A covalently linked to a $H_C$ domain from BoNT/B, wherein the C-terminal amino acid residue of the $LH_N$ domain corresponds to the eighth amino acid residue N-terminally to the β-strand located at the beginning (N-term) of the $H_C$ domain of BoNT/A, and wherein the N-terminal amino acid residue of the $H_C$ domain corresponds to the seventh amino acid residue N-terminally to the β-strand located at the beginning (N-term) of the $H_C$ domain of BoNT/B.

A BoNT/AB chimera may comprise an $LH_N$ domain from BoNT/A covalently linked to a $H_C$ domain from BoNT/B, wherein the C-terminal amino acid residue of the $LH_N$ domain corresponds to the C-terminal amino acid residue of the α-helix located at the end (C-terminus) of the $LH_N$ domain of BoNT/A, and wherein the N-terminal amino acid residue of the $H_C$ domain corresponds to the amino acid residue immediately C-terminal to the C-terminal amino acid residue of the α-helix located at the end (C-terminus) of the $LH_N$ domain of BoNT/B.

The rationale of the design process of the BoNT/AB chimera is to try to ensure that the secondary structure was not compromised and thereby minimise any changes to the tertiary structure and to the function of each domain. Without wishing to be bound by theory, it is hypothesized that by not disrupting the four central amino acid residues of the $3_{10}$ helix in the BoNT/AB chimera ensures an optimal conformation for the chimeric neurotoxin, thereby allowing for the chimeric neurotoxin to exert its functions to their full capacity. In fact, surprisingly, retaining solely the first amino acid residue of the $3_{10}$ helix of the BoNT/A and the second amino acid residue of the $3_{10}$ helix onwards of BoNT/B not only allows the production of soluble and functional BoNT/AB chimera, but further leads to improved properties over other BoNT/AB chimeras, in particular an increased potency, an increased Safety Ratio and/or a longer duration of action (as well as increased Safety Ratio and/or duration of action when compared to unmodified BoNT/A).

The BoNT/A light-chain, BoNT/A translocation domain, and/or BoNT/B $H_C$ domain may be a modified BoNT/A light-chain, BoNT/A translocation domain, and/or BoNT/B $H_C$ domain or a derivative thereof, including but not limited to those described below. A modified BoNT/A light-chain, BoNT/A translocation domain, and/or BoNT/B $H_C$ domain or derivative may contain one or more amino acids that has been modified as compared to the native (unmodified) form of the BoNT/A light-chain, BoNT/A translocation domain, and/or BoNT/B $H_C$ domain, or may contain one or more inserted amino acids that are not present in the native (unmodified) form of the BoNT/A light-chain, BoNT/A translocation domain, and/or BoNT/B $H_C$ domain. By way of example, a modified BoNT/A light-chain, BoNT/A translocation domain, and/or BoNT/B $H_C$ domain may have modified amino acid sequences in one or more domains relative to the native (unmodified) BoNT/A light-chain, BoNT/A translocation domain, and/or BoNT/B $H_C$ domain sequence. Such modifications may modify functional aspects thereof, for example biological activity or persistence. Thus, in one embodiment, the BoNT/A light-chain, BoNT/A translocation domain, and/or BoNT/B $H_C$ domain is a modified BoNT/A light-chain, BoNT/A translocation domain, and/or BoNT/B $H_C$ domain, or modified BoNT/A light-chain, BoNT/A translocation domain, and/or BoNT/B $H_C$ domain derivative.

A modified BoNT/B $H_C$ domain may have one or more modifications modifying binding to target nerve cells, for example providing higher or lower affinity binding when compared to the native (unmodified) BoNT/B $H_C$ domain. Such modifications in the BoNT/B $H_C$ domain may include modifying residues in the ganglioside binding site of the $H_C$ domain or in the protein (e.g. synaptotagmin) binding site that alter binding to the ganglioside receptor and/or the protein receptor of the target nerve cell. Examples of such modified neurotoxins are described in WO 2006/027207 and WO 2006/114308.

A modified light-chain may have one or more modifications in the amino acid sequence thereof, for example modifications in the substrate binding or catalytic domain which may alter or modify the SNARE protein specificity of the modified light-chain, preferably with the proviso that said modifications do not catalytically inactivate said light-chain.

Examples of such modified neurotoxins are described in WO 2010/120766 and US 2011/0318385 .

The LH$_N$ domain from BoNT/A may correspond to amino acid residues 1 to 872 of SEQ ID NO: 2, or a polypeptide sequence having at least 70% sequence identity thereto. The LH$_N$ domain from BoNT/A may correspond to amino acid residues 1 to 872 of SEQ ID NO: 2, or a polypeptide sequence having at least 80%, 90% or 95% sequence identity thereto. Preferably, the LH$_N$ domain from BoNT/A corresponds to amino acid residues 1 to 872 of SEQ ID NO: 2.

The H$_C$ domain from BoNT/B may correspond to amino acid residues 860 to 1291 of SEQ ID NO: 16, or a polypeptide sequence having at least 70% sequence identity thereto. The H$_C$ domain from BoNT/B may correspond to amino acid residues 860 to 1291 of SEQ ID NO: 16, or a polypeptide sequence having at least 80%, 90% or 95% sequence identity thereto. Preferably, the H$_C$ domain from BoNT/B corresponds to amino acid residues 860 to 1291 of SEQ ID NO: 16.

Preferably, the BoNT/AB chimera comprises a BoNT/A1 LH$_N$ domain and a BoNT/B1 H$_C$ domain. More preferably, the LH$_N$ domain corresponds to amino acid residues 1 to 872 of BoNT/A1 (SEQ ID NO: 2) and the H$_C$ domain corresponds to amino acid residues 860 to 1291 of BoNT/B1 (SEQ ID NO: 16).

Preferably, a BoNT/B H$_C$ domain further comprises at least one amino acid residue substitution, addition or deletion in the H$_{CC}$ subdomain which has the effect of increasing the binding affinity of BoNT/B neurotoxin for human Syt II as compared to the natural BoNT/B sequence. Suitable amino acid residue substitution, addition or deletion in the BoNT/B H$_{CC}$ subdomain have been disclosed in WO 2013/180799 and in WO 2016/154534 ·

A suitable amino acid residue substitution, addition or deletion in the BoNT/B H$_{CC}$ subdomain may include a substitution mutation selected from the group consisting of: V1118M; Y1183M; E1191M; E1191I; E1191Q; E1191T; S1199Y; S1199F; S1199L; S1201V; E1191C, E1191V, E1191L, E1191Y, S1199W, S1199E, S1199H, W1178Y, W1178Q, W1178A, W1178S, Y1183C, Y1183P and combinations thereof.

A suitable amino acid residue substitution, addition or deletion in the BoNT/B H$_{CC}$ subdomain may further include combinations of two substitution mutations selected from the group consisting of: E1191M and S1199L, E1191M and S1199Y, E1191M and S1199F, E1191Q and S1199L, E1191Q and S1199Y, E1191Q and S1199F, E1191M and S1199W, E1191M and W1178Q, E1191C and S1199W, E1191C and S1199Y, E1191C and W1178Q, E1191Q and S1199W, E1191V and S1199W, E1191V and S1199Y, or E1191V and W1178Q.

A suitable amino acid residue substitution, addition or deletion in the BoNT/B H$_{CC}$ subdomain may also include a combination of three substitution mutations which are E1191M, S1199W and W1178Q.

Most preferably, the suitable amino acid residue substitution, addition or deletion in the BoNT/B H$_{CC}$ subdomain includes a combination of two substitution mutations which are E1191M and S1199Y. Such modifications are present in BoNT/AB chimeras SEQ ID NO: 13 and SEQ ID NO: 14, for example.

The modification may be a modification when compared to unmodified BoNT/B shown as SEQ ID NO: 16, wherein the amino acid residue numbering is determined by alignment with SEQ ID NO: 16. As the presence of a methionine residue at position 1 of SEQ ID NO: 16 (as well as the SEQ ID NOs corresponding to modified BoNT/A polypeptides described herein) is optional, the skilled person will take the presence/absence of the methionine residue into account when determining amino acid residue numbering. For example, where SEQ ID NO: 16 includes a methionine, the position numbering will be as defined above (e.g. E1191 will be E1191 of SEQ ID NO: 16). Alternatively, where the methionine is absent from SEQ ID NO: 16 the amino acid residue numbering should be modified by -1 (e.g. E1191 will be E1190 of SEQ ID NO: 16). Similar considerations apply when the methionine at position 1 of the other polypeptide sequences described herein is present/absent, and the skilled person will readily determine the correct amino acid residue numbering using techniques routine in the art.

A modified BoNT/A for use in the invention may comprise a polypeptide sequence having at least 70% sequence identity to a polypeptide sequence selected from SEQ ID NOs: 11-15. For example, a polypeptide sequence having at least 80%, 90%, 95% or 99.9% sequence identity to a polypeptide sequence selected from SEQ ID NOs: 11-15. Preferably, a modified BoNT/A for use in the invention may comprise (more preferably consist of) a polypeptide sequence selected from SEQ ID NOs: 11-15.

When a modified BoNT/A is a BoNT/AB chimera, it is preferred that the modified BoNT/A comprises a polypeptide sequence having at least 70% sequence identity to SEQ ID NO: 14. For example, a polypeptide sequence having at least 80%, 90%, 95% or 99.9% sequence identity to SEQ ID NO: 14. Most preferably, a modified BoNT/A for use in the invention may comprise (more preferably consist of) SEQ ID NO: 14.

Methods for modifying proteins by substitution, insertion or deletion of amino acid residues are known in the art. By way of example, amino acid modifications may be introduced by modification of a DNA sequence encoding a BoNT/A (e.g. encoding unmodified BoNT/A). This can be achieved using standard molecular cloning techniques, for example by site-directed mutagenesis where short strands of DNA (oligonucleotides) coding for the desired amino acid(s) are used to replace the original coding sequence using a polymerase enzyme, or by inserting/deleting parts of the gene with various enzymes (e.g., ligases and restriction endonucleases). Alternatively, a modified gene sequence can be chemically synthesised.

Where a polypeptide sequence of a modified BoNT/A described herein comprises a tag, e.g. for purification, such as a His-tag, said tag is optional. Preferably, said tag is removed prior to use of the modified BoNT/A according to the invention.

As discussed above, a modified BoNT/A described herein has increased tissue retention properties that also provide increased potency and/or duration of action and can allow for increased dosages without any additional negative effects. One way in which these advantageous properties may be defined is in terms of the Safety Ratio of the modified BoNT/A. In this regard, undesired effects of a clostridial toxin (caused by diffusion of the toxin away from the site of administration) can be assessed experimentally by measuring percentage bodyweight loss in a relevant animal model (e.g. a mouse, where loss of bodyweight is detected within seven days of administration). Conversely, desired on-target effects of a clostridial toxin can be assessed experimentally by Digital Abduction Score (DAS) assay, a measurement of muscle paralysis. The DAS assay may be performed by injection of 20μl of clostridial toxin, formulated in Gelatin Phosphate Buffer, into the mouse gastrocnemius/soleus complex, followed by assessment of Digital Abduction Score using the method of Aoki (Aoki KR, Toxicon 39: 1815-1820; 2001). In the DAS assay, mice are suspended briefly by the tail in order to elicit a characteristic startle response in which the mouse extends its hind limbs and abducts its hind digits. Following clostridial toxin injection, the varying degrees of digit abduction are scored on a five-point scale (0=normal to 4=maximal reduction in digit abduction and leg extension).

The Safety Ratio of a modified BoNT/A of the invention (or unmodified BoNT/A for comparison) may then be expressed as the ratio between the amount of toxin required for a 10% drop in a bodyweight (measured at peak effect within the first seven days after dosing in a mouse) and the amount of toxin required for a DAS score of 2. High Safety Ratio scores are therefore desired and indicate a toxin that is able to effectively paralyse a target muscle with little undesired off-target effects. A modified BoNT/A of the present invention has a Safety Ratio that is higher than the Safety Ratio of an equivalent unmodified (native) BoNT/A.

A high Safety Ratio is particularly advantageous in therapy because it represents an increase in the therapeutic index. In other words, this means that reduced dosages can be used compared to alternative clostridial neurotoxin therapeutics and/or that increased dosages can be used without any additional (e.g. deleterious) effects. Deleterious effects include systemic toxicity and undesired spread to adjacent muscles. The possibility to use higher doses of neurotoxin without additional effects is particularly advantageous as higher doses usually lead to a longer duration of action of the neurotoxin.

The potency of a modified BoNT/A may be expressed as the minimal dose of neurotoxin which leads to a given DAS score when administered to a mouse gastrocnemius/soleus complex, for example a DAS score of 2 ($ED_{50}$ dose) or a DAS score of 4. The Potency of a modified BoNT/A may be also expressed as the $EC_{50}$ dose in a cellular assay measuring SNARE cleavage by the neurotoxin, for example the $EC_{50}$ dose in a cellular assay measuring SNAP25 cleavage by a modified BoNT/A.

The duration of action of a modified BoNT/A may be expressed as the time required for retrieving a DAS score of 0 after administration of a given dose of neurotoxin, for example the minimal dose of neurotoxin leading to a DAS score of 4, to a mouse gastrocnemius/soleus complex.

Thus, in one embodiment, a modified BoNT/A of the present invention has a Safety Ratio that is greater than 7 (for example, at least 8, 9, 10, 15, 20, 25, 30, 35, 40, 45 or 50), wherein Safety Ratio is calculated as: dose of toxin required for -10% bodyweight change (pg/mouse) divided by DAS $ED_{50}$ (pg/mouse) [$ED_{50}$ = dose required to produce a DAS score of 2].

In one embodiment, a modified BoNT/A of the present invention has a Safety Ratio of at least 10. In one embodiment, a modified BoNT/A of the present invention has a Safety Ratio of at least 15.

Preferably, where a modified BoNT/A is one comprising one or more amino acid residue(s) selected from: ASN 886, ASN 930, ASN 954, SER 955, GLN 991, ASN 1025, ASN 1026, ASN 1052, ASN 1188, ASP 1213, GLY 1215, ASN 1216, GLN 1229, ASN 1242, ASN 1243, SER 1274 and THR 1277 as described herein, said modified BoNT/A has a Safety Ratio of at least 20, more preferably at least 22 (e.g. 23-25).

Preferably, where a modified BoNT/A is one that comprises a BoNT/A light-chain and translocation domain, and a BoNT/B $H_C$ domain, the modified BoNT/A has a Safety Ratio of at least 10, more preferably at least 12 (e.g. 14-15).

The modified BoNT/A is preferably in a non-complexed form (i.e. free from complexing proteins that are present in naturally occurring BoNT/A). Examples of such complexing proteins include a neurotoxin-associated proteins (NAP) and a nontoxic-nonhemagglutinin component (NTNH). However, it is preferred that the modified BoNT/A is a recombinant modified BoNT/A. Such a modified BoNT/A of the present invention can be produced using recombinant nucleic acid technologies.

In one embodiment a nucleic acid (for example, DNA) comprising a nucleic acid sequence encoding a modified BoNT/A is provided. In one embodiment, the nucleic acid sequence is prepared as part of a DNA vector comprising a promoter and a terminator. The nucleic acid sequence may be selected from any of the nucleic acid sequences described herein.

In a preferred embodiment, the vector has a promoter selected from:

| Promoter | Induction Agent | Typical Induction Condition |
|---|---|---|
| Tac (hybrid) | IPTG | 0.2 mM (0.05-2.0mM) |
| AraBAD | L-arabinose | 0.2% (0.002-0.4%) |
| T7-lac operator | IPTG | 0.2 mM (0.05-2.0mM) |

In another preferred embodiment, the vector has a promoter selected from:

| Promoter | Induction Agent | Typical Induction Condition |
|---|---|---|
| Tac (hybrid) | IPTG | 0.2 mM (0.05-2.0mM) |
| AraBAD | L-arabinose | 0.2% (0.002-0.4%) |
| T7-lac operator | IPTG | 0.2 mM (0.05-2.0mM) |
| T5-lac operator | IPTG | 0.2 mM (0.05-2.0mM) |

The nucleic acid molecules may be made using any suitable process known in the art. Thus, the nucleic acid molecules may be made using chemical synthesis techniques. Alternatively, the nucleic acid molecules of the invention may be made using molecular biology techniques.

The DNA construct of the present invention is preferably designed *in silico,* and then synthesised by conventional DNA synthesis techniques.

The above-mentioned nucleic acid sequence information is optionally modified for codon-biasing according to the ultimate host cell (e.g. *E. coli*) expression system that is to be employed.

The terms "nucleotide sequence" and "nucleic acid" are used synonymously herein. Preferably the nucleotide sequence is a DNA sequence.

A modified BoNT/A of the invention may be present as a single-chain or as a di-chain. However, it is preferred that the modified BoNT/A is present as a di-chain in which the L-chain is linked to the H-chain (or component thereof, e.g. the $H_N$ domain) via a di-sulphide bond.

Production of a single-chain modified BoNT/A having a light-chain and a heavy-chain may be achieved using a method comprising expressing a nucleic acid encoding a modified BoNT/A in an expression host, lysing the host cell to provide a host cell homogenate containing the single-chain modified BoNT/A, and isolating the single-chain modified BoNT/A. The single-chain modified BoNT/A described herein may be proteolytically processed using a method comprising contacting a single-chain modified BoNT/A with a protease that hydrolyses a peptide bond in the activation loop of the modified BoNT/A, thereby converting the single-chain modified BoNT/A into a corresponding di-chain modified BoNT/A (e.g. wherein the light-chain and heavy-chain are joined together by a disulphide bond). A di-chain modified BoNT/A is preferably obtainable by such a method.

Thus, a modified BoNT/A used in the invention is preferably a di-chain modified BoNT/A that has been produced from a single-chain BoNT/A, wherein the single-chain BoNT/A comprises or consists of a polypeptide sequence described herein. For example, it is preferred that the modified BoNT/A used in the invention is a di-chain modified BoNT/A that has been produced from a polypeptide comprising a polypeptide sequence having at least 70% (e.g. at least 80%, 90%, 95% or 99.9%) sequence identity to SEQ ID NO: 14. Most preferably, the modified BoNT/A used in the invention is a di-chain modified BoNT/A that has been produced from a polypeptide comprising (even more preferably consisting of) SEQ ID NO: 14. Accordingly, in some embodiments, the modified BoNT/A is a di-chain modified BoNT/A in which the light-chain (L-chain) is linked to the heavy-chain (H-chain) via a di-sulphide bond obtainable by a method comprising contacting a single-chain modified BoNT/A comprising SEQ ID NO: 14 with a protease that hydrolyses a peptide bond in the activation loop thereof, thereby converting the single-chain modified BoNT/A into the corresponding di-chain modified BoNT/A. In some embodiments, the modified BoNT/A is a di-chain modified BoNT/A in which the L-chain is linked to the H-chain via a di-sulphide bond obtainable by a method comprising contacting a single-chain modified BoNT/A consisting of SEQ ID NO: 14 with a protease that hydrolyses a peptide bond in the activation loop thereof, thereby converting the single-chain modified BoNT/A into the corresponding di-chain modified BoNT/A.

In one embodiment, the modified BoNT/A used in the invention is a di-chain modified BoNT/A that has been produced from a polypeptide comprising a polypeptide sequence having at least 70% (e.g. at least 80%, 90%, 95% or 99.9%) sequence identity to SEQ ID NO: 4. Preferably, the modified BoNT/A used in the invention is a di-chain modified BoNT/A that has been produced from a polypeptide comprising (more preferably consisting of) SEQ ID NO: 4. Accordingly, in some embodiments, the modified BoNT/A is a di-chain modified BoNT/A in which the light-chain (L-chain) is linked to the heavy-chain (H-chain) via a di-sulphide bond obtainable by a method comprising contacting a single-chain modified BoNT/A comprising SEQ ID NO: 4 with a protease that hydrolyses a peptide bond in the activation loop thereof, thereby converting the single-chain modified BoNT/A into the corresponding di-chain modified BoNT/A. In some embodiments, the modified BoNT/A is a di-chain modified BoNT/A in which the L-chain is linked to the H-chain via a di-sulphide bond obtainable by a method comprising contacting a single-chain modified BoNT/A consisting of SEQ ID NO: 4 with a protease that hydrolyses a peptide bond in the activation loop thereof, thereby converting the single-chain modified BoNT/A into the corresponding di-chain modified BoNT/A.

The term "obtainable" as used herein also encompasses the term "obtained". In one embodiment the term "obtainable" means obtained.

The protease used to cleave the activation loop is preferably Lys-C. Suitable proteases and method for cleaving activation loops to produce di-chain clostridial neurotoxins are taught in WO 2014/080206, WO2014/079495, and EP2677029A2 .

Lys-C may cleave an activation loop C-terminal to one or more of the lysine residues present therein. Where Lys-C cleaves the activation loop more than once, the skilled person will appreciate that a small peptide of the activation loop of a di-chain modified BoNT/A may be absent when compared to a SEQ ID NO shown herein.

The modified BoNT/A of the invention may be formulated in any suitable manner for administration to a subject, for example as part of a pharmaceutical composition. Thus, in one aspect, the invention provides a pharmaceutical composition comprising a modified BoNT/A of the invention and a pharmaceutically acceptable carrier, excipient, adjuvant, and/or salt.

Fluid dosage forms are typically prepared utilising the modified BoNT/A and a pyrogen-free sterile vehicle. The modified BoNT/A, depending on the vehicle and concentration used, can be either dissolved or suspended in the vehicle. In preparing solutions the modified BoNT/A can be dissolved in the vehicle, the solution being made isotonic if necessary by addition of sodium chloride and sterilised by filtration through a sterile filter using aseptic techniques before filling into suitable sterile vials or ampoules and sealing. Alternatively, if solution stability is adequate, the solution in its sealed containers may be sterilised by autoclaving. Advantageously additives such as buffering, solubilising, stabilising, preservative or bactericidal, suspending or emulsifying agents and or local anaesthetic agents may be dissolved in the vehicle.

Dry powders, which are dissolved or suspended in a suitable vehicle prior to use, may be prepared by filling pre-sterilised ingredients into a sterile container using aseptic technique in a sterile area. Alternatively the ingredients may be dissolved into suitable containers using aseptic technique in a sterile area. The product is then freeze dried and the containers are sealed aseptically.

Parenteral suspensions, suitable for an administration route described herein, are prepared in substantially the same manner, except that the sterile components are suspended in the sterile vehicle, instead of being dissolved and sterilisation cannot be accomplished by filtration. The components may be isolated in a sterile state or alternatively it may be sterilised after isolation, e.g. by gamma irradiation.

Advantageously, a suspending agent for example polyvinylpyrrolidone is included in the composition(s) to facilitate uniform distribution of the components.

In another aspect, the invention provides a unit dosage form of modified botulinum neurotoxin A (BoNT/A) for use in treating cervical dystonia, the unit dosage form comprising:

a. 750 pg to 17,000 pg of modified BoNT/A; and
b. optionally a pharmaceutically acceptable carrier, excipient, adjuvant, and/or salt,

wherein the modified BoNT/A comprises a BoNT/A light-chain and translocation domain, and a BoNT/B receptor binding domain ($H_C$ domain).

It is preferred that the modified BoNT/A of the unit dosage form comprises a polypeptide sequence having at least 70% sequence identity to SEQ ID NO: 14. For example, a polypeptide sequence having at least 80%, 90%, 95% or 99.9% sequence identity to SEQ ID NO: 14. Most preferably, a modified BoNT/A may comprise (more preferably consist of) SEQ ID NO: 14.

A unit dosage form for treating cervical dystonia may comprise 750 pg to 17,000 pg of modified BoNT/A, wherein the 750 pg to 17,000 pg of modified BoNT/A, wherein the modified BoNT/A comprises a BoNT/A light-chain and translocation domain, and a BoNT/B receptor binding domain ($H_C$ domain). An upper limit of the unit dose range may be 16,500, 15,500, 14,500, 13,500, 12,500, 11,500, 10,500, 9,500, 8,500, 7,500, 6,500, 5,500, 4,500, 3,500, 2,500, 2,250, 2,000, 1,500, 1,250, 1,000 or 750 pg of modified BoNT/A, preferably the upper limit is 16,000 pg. A lower limit of the unit dose range may be 800, 850, 950, 1,000, 1,500, 1,750, 2,000, 2,500, 3,000, 3,500, 4,000, 4,500 or 5,000 pg of modified BoNT/A, preferably the lower limit is 1,000 pg. Preferably, the unit dose of modified BoNT/A is 1,000 pg to 16,000 pg of modified BoNT/A, e.g. 950 pg to 1,250 pg, 1,750 pg to 2,250 pg, or 8,000 pg to 12,000 pg. Preferably, a unit dose of modified BoNT/A may be 1,000, 2,000, 3,000, 8,000 or 16,000 pg.

A unit dosage form for treating cervical dystonia may comprise 31 Units to 707 Units of modified BoNT/A wherein the modified BoNT/A comprises a BoNT/A light-chain and translocation domain, and a BoNT/B receptor binding domain ($H_C$ domain). An upper limit of the unit dose range may be 700, 650, 600, 550, 500, 450, 400, 350, 300, 250, 200, 150 100, 95, 90, 85, 65, 60, 55, 50, or 31 Units of modified BoNT/A, preferably the upper limit is 666 Units. A lower limit of the unit dose range may be 35, 40, 45, 50, 60, 65, 70, 75, 80, 85, 90, 100, 150, 200, 250, 300, 350, 400, 450, 500, 550, 600, 650, or 700 Units of modified BoNT/A, preferably the lower limit is 42 Units. Preferably, the unit dose of modified BoNT/A is 42 Units to 666 Units of modified BoNT/A, for example 40 Units to 50 Units, 70 Units to 95 Units, or 333 Units to 499 Units. Preferably, a unit dose of modified BoNT/A may be 41.6 Units, 83.2 Units, 124.8 Units, 332.8 Units or 665.6 Units.

Described herein is a unit dosage form of modified botulinum neurotoxin A (BoNT/A) for treating cervical dystonia, the unit dosage form comprising:

a. 53 U to 948 U Units of modified BoNT/A, wherein 1 Unit is an amount of the modified BoNT/A that corresponds to the calculated median lethal dose ($LD_{50}$) in mice; or

b. 450 pg to 8,000 pg of modified BoNT/A; and

c. optionally a pharmaceutically acceptable carrier, excipient, adjuvant, and/or salt,

wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from:

i. substitution of an acidic surface exposed amino acid residue with a basic amino acid residue;

ii. substitution of an acidic surface exposed amino acid residue with an uncharged amino acid residue;

iii. substitution of an uncharged surface exposed amino acid residue with a basic amino acid residue;

iv. insertion of a basic amino acid residue; and

v. deletion of an acidic surface exposed amino acid residue.

A unit dosage form for treating cervical dystonia may comprise 450 pg to 8,000 pg of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. An upper limit of the range may be 7,750, 7,500, 7,000, 6,000, 5,000, 4,000, 3,000, 2,000 or 1,000, pg of modified BoNT/A, preferably the upper limit is 7,500 pg. A lower limit of the range may be 475, 500, 600, 700, 800, 900, 1,000, 1,500, 2,000, 3,000, 4,000, 5,000, 6,000 or 7,000 pg of modified BoNT/A, preferably the lower limit is 500 pg. Preferably, the unit dosage form comprises 500 pg to 7,500 pg of modified BoNT/A, e.g. 4,000 pg to 6,000 pg. Most preferably the unit dosage form comprises 2,000 pg to 3,000 pg, such as 2,400 pg to 2,600 pg.

A unit dosage form for treating cervical dystonia may comprise 53 Units to 948 Units of modified BoNT/A, wherein the modified BoNT/A comprises a modification at one or more amino acid residue(s) selected from: ASN 886, ASN 905, GLN 915, ASN 918, GLU 920, ASN 930, ASN 954, SER 955, GLN 991, GLU 992, GLN 995, ASN 1006, ASN 1025, ASN 1026, ASN 1032, ASN 1043, ASN 1046, ASN 1052, ASP 1058, HIS 1064, ASN 1080, GLU 1081, GLU 1083, ASP 1086, ASN 1188, ASP 1213, GLY 1215, ASN 1216, GLN 1229, ASN 1242, ASN 1243, SER 1274, and THR 1277, wherein the modification is selected from: (i) substitution of an acidic surface exposed amino acid residue with a basic amino acid residue; (ii) substitution of an acidic surface exposed amino acid residue with an uncharged amino acid residue; (iii) substitution of an uncharged surface exposed amino acid residue with a basic amino acid residue; (iv) insertion of a basic amino acid residue; and (v) deletion of an acidic surface exposed amino acid residue. An upper limit of the range may be 925, 900, 850, 800, 750, 700, 650, 600, 550, 500, 450, 400, 350, 300, 250, 200, 150 or 100 Units of modified BoNT/A, preferably the upper limit is 889 Units. A lower limit of the range may be 55, 60, 65, 70, 75, 80, 85, 90, 100, 150, 200, 250, 300, 350, 400, 450, 500, 550, 600, 650, 700, 750, 800, 850 or 900 Units of modified BoNT/A, preferably the lower limit is 59 Units. Preferably, the unit dosage form comprises 59 Units to 889 Units of modified BoNT/A, for example 200 Units to 600 Units. Most preferably the unit dosage form comprises 237 Units to 355 Units, such as 284 Units to 308 Units.

In another aspect, the disclosure provides a kit comprising:

a. the unit dosage form according to the present disclosure; and

b. instructions for use of the same in treating cervical dystonia; and

c. optionally a diluent.

The unit dosage form for use according to the invention may be comprised within a kit, wherein the kit comprises instructions for use of the same in treating cervical dystonia and optionally a diluent.

Embodiments related to the various therapeutic uses of the disclosure can be applied to the methods, compositions (e.g. unit dosage forms), and kits of the disclosure and *vice versa.*

## SEQUENCE HOMOLOGY

Any of a variety of sequence alignment methods can be used to determine percent identity, including, without limitation, global methods, local methods and hybrid methods, such as, e.g., segment approach methods. Protocols to determine percent identity are routine procedures within the scope of one skilled in the art. Global methods align sequences from the beginning to the end of the molecule and determine the best alignment by adding up scores of individual residue pairs and by imposing gap penalties. Non-limiting methods include, e.g., CLUSTAL W, see, e.g., Julie D. Thompson et al., CLUSTAL W: Improving the Sensitivity of Progressive Multiple Sequence Alignment Through Sequence Weighting, Position- Specific Gap Penalties and Weight Matrix Choice, 22(22) Nucleic Acids Research 4673-4680 (1994); and iterative refinement, see, e.g., Osamu Gotoh, Significant Improvement in Accuracy of Multiple Protein. Sequence Alignments by Iterative Refinement as Assessed by Reference to Structural Alignments, 264(4) J. Mol. Biol. 823-838 (1996). Local methods align sequences by identifying one or more conserved motifs shared by all of the input sequences. Non-limiting methods include, e.g., Match-box, see, e.g., Eric Depiereux and Ernest Feytmans, Match-Box: A Fundamentally New Algorithm for the Simultaneous Alignment of Several Protein Sequences, 8(5) CABIOS 501 -509 (1992); Gibbs sampling, see, e.g., C. E. Lawrence et al., Detecting Subtle Sequence Signals: A Gibbs Sampling Strategy for Multiple Alignment, 262(5131) Science 208-214 (1993); Align-M, see, e.g., Ivo Van Walle et al., Align-M - A New Algorithm for Multiple Alignment of Highly Divergent Sequences, 20(9) Bioinformatics:1428-1435 (2004).

Thus, percent sequence identity is determined by conventional methods. See, for example, Altschul et al., Bull. Math. Bio. 48: 603-16, 1986 and Henikoff and Henikoff, Proc. Natl. Acad. Sci. USA 89:10915-19, 1992. Briefly, two amino acid sequences are aligned to optimize the alignment scores using a gap opening penalty of 10, a gap extension penalty of 1, and the "blosum 62" scoring matrix of Henikoff and Henikoff (ibid.) as shown below (amino acids are indicated by the standard one-letter codes); preferably this method is used to align a sequence with a subject sequence herein (e.g. SEQ ID NO: 2) to define amino acid position numbering as described herein.

The "percent sequence identity" between two or more nucleic acid or amino acid sequences is a function of the number of identical positions shared by the sequences. Thus, % identity may be calculated as the number of identical nucleotides / amino acids divided by the total number of nucleotides / amino acids, multiplied by 100. Calculations of % sequence identity may also take into account the number of gaps, and the length of each gap that needs to be introduced to optimize alignment of two or more sequences. Sequence comparisons and the determination of percent identity between two or more sequences can be carried out using specific mathematical algorithms, such as BLAST, which will be familiar to a skilled person.

## ALIGNMENT SCORES FOR DETERMINING SEQUENCE IDENTITY

```
        A  R  N  D  C  Q  E  G  H  I  L  K  M  F  P  S  T  W  Y  V
A  4
R -1  5
N -2  0  6
D -2 -2  1  6
C  0 -3 -3 -3  9
Q -1  1  0  0 -3  5
E -1  0  0  2 -4  2  5
G  0 -2  0 -1 -3 -2 -2  6
H -2  0  1 -1 -3  0  0 -2  8
I -1 -3 -3 -3 -1 -3 -3 -4 -3  4
L -1 -2 -3 -4 -1 -2 -3 -4 -3  2  4
K -1  2  0 -1 -3  1  1 -2 -1 -3 -2  5
M -1 -1 -2 -3 -1  0 -2 -3 -2  1  2 -1  5
F -2 -3 -3 -3 -2 -3 -3 -3 -1  0  0 -3  0  6
P -1 -2 -2 -1 -3 -1 -1 -2 -2 -3 -3 -1 -2 -4  7
S  1 -1  1  0 -1  0  0  0 -1 -2 -2  0 -1 -2 -1  4
T  0 -1  0 -1 -1 -1 -1 -2 -2 -1 -1 -1 -1 -2 -1  1  5
W -3 -3 -4 -4 -2 -2 -3 -2 -2 -3 -2 -3 -1  1 -4 -3 -2 11
Y -2 -2 -2 -3 -2 -1 -2 -3  2 -1 -1 -2 -1  3 -3 -2 -2  2  7
V  0 -3 -3 -3 -1 -2 -2 -3 -3  3  1 -2  1 -1 -2 -2  0 -3 -1  4
```

The percent identity is then calculated as:

$$\frac{\text{Total number of identical matches}}{[\text{length of the longer sequence plus the number of gaps introduced into the longer sequence in order to align the two sequences}]} \times 100$$

Substantially homologous polypeptides are characterized as having one or more amino acid substitutions, deletions or additions. These changes are preferably of a minor nature, that is conservative amino acid substitutions (see below) and other substitutions that do not significantly affect the folding or activity of the polypeptide; small deletions, typically of one to about 30 amino acids; and small amino- or carboxyl-terminal extensions, such as an amino-terminal methionine residue, a small linker peptide of up to about 20-25 residues, or an affinity tag.

CONSERVATIVE AMINO ACID SUBSTITUTIONS

| | |
|---|---|
| Basic: | arginine |
| | lysine |

(continued)

| | |
|---|---|
| | histidine |
| Acidic: | glutamic acid |
| | aspartic acid |
| Polar: | glutamine |
| | asparagine |
| Hydrophobic: | leucine |
| | isoleucine |
| | valine |
| Aromatic: | phenylalanine |
| | tryptophan |
| | tyrosine |
| Small: | glycine |
| | alanine |
| | serine |
| | threonine |
| | methionine |

In addition to the 20 standard amino acids, non-standard amino acids (such as 4-hydroxyproline, 6-N-methyl lysine, 2-aminoisobutyric acid, isovaline and $\alpha$ -methyl serine) may be substituted for amino acid residues of the polypeptides of the present invention. A limited number of non-conservative amino acids, amino acids that are not encoded by the genetic code, and unnatural amino acids may be substituted for polypeptide amino acid residues. The polypeptides of the present invention can also comprise non-naturally occurring amino acid residues.

Non-naturally occurring amino acids include, without limitation, trans-3-methylproline, 2,4-methano-proline, cis-4-hydroxyproline, trans-4-hydroxy-proline, N-methylglycine, allo-threonine, methyl-threonine, hydroxy-ethylcysteine, hydroxyethylhomo-cysteine, nitro-glutamine, homoglutamine, pipecolic acid, tert-leucine, norvaline, 2-azaphenylalanine, 3-azaphenyl-alanine, 4-azaphenyl-alanine, and 4-fluorophenylalanine. Several methods are known in the art for incorporating non-naturally occurring amino acid residues into proteins. For example, an in vitro system can be employed wherein nonsense mutations are suppressed using chemically aminoacylated suppressor tRNAs. Methods for synthesizing amino acids and aminoacylating tRNA are known in the art. Transcription and translation of plasmids containing nonsense mutations is carried out in a cell free system comprising an E. coli S30 extract and commercially available enzymes and other reagents. Proteins are purified by chromatography. See, for example, Robertson et al., J. Am. Chem. Soc. 113:2722, 1991; Ellman et al., Methods Enzymol. 202:301, 1991; Chung et al., Science 259:806-9, 1993; and Chung et al., Proc. Natl. Acad. Sci. USA 90:10145-9, 1993). In a second method, translation is carried out in Xenopus oocytes by microinjection of mutated mRNA and chemically aminoacylated suppressor tRNAs (Turcatti et al., J. Biol. Chem. 271:19991-8, 1996). Within a third method, E. coli cells are cultured in the absence of a natural amino acid that is to be replaced (e.g., phenylalanine) and in the presence of the desired non-naturally occurring amino acid(s) (e.g., 2-azaphenylalanine, 3-azaphenylalanine, 4-azaphenylalanine, or 4-fluorophenylalanine). The non-naturally occurring amino acid is incorporated into the polypeptide in place of its natural counterpart. See, Koide et al., Biochem. 33:7470-6, 1994. Naturally occurring amino acid residues can be converted to non-naturally occurring species by in vitro chemical modification. Chemical modification can be combined with site-directed mutagenesis to further expand the range of substitutions (Wynn and Richards, Protein Sci. 2:395-403, 1993).

A limited number of non-conservative amino acids, amino acids that are not encoded by the genetic code, non-naturally occurring amino acids, and unnatural amino acids may be substituted for amino acid residues of polypeptides of the present invention.

Essential amino acids in the polypeptides of the present invention can be identified according to procedures known in the art, such as site-directed mutagenesis or alanine-scanning mutagenesis (Cunningham and Wells, Science 244: 1081-5, 1989). Sites of biological interaction can also be determined by physical analysis of structure, as determined by such techniques as nuclear magnetic resonance, crystallography, electron diffraction or photoaffinity labeling, in conjunction with mutation of putative contact site amino acids. See, for example, de Vos et al., Science 255:306-12, 1992; Smith et al., J. Mol. Biol. 224:899-904, 1992; Wlodaver et al., FEBS Lett. 309:59-64, 1992. The identities of essential amino acids can also be inferred from analysis of homologies with related components (e.g. the translocation or protease components) of the polypeptides of the present invention.

Multiple amino acid substitutions can be made and tested using known methods of mutagenesis and screening, such as those disclosed by Reidhaar-Olson and Sauer (Science 241:53-7, 1988) or Bowie and Sauer (Proc. Natl. Acad. Sci. USA 86:2152-6, 1989). Briefly, these authors disclose methods for simultaneously randomizing two or more positions in a polypeptide, selecting for functional polypeptide, and then sequencing the mutagenized polypeptides to determine the spectrum of allowable substitutions at each position. Other methods that can be used include phage display (e.g., Lowman et al., Biochem. 30:10832-7, 1991; Ladner et al., U.S. Patent No. 5,223,409; Huse, WIPO Publication WO 92/06204) and region-directed mutagenesis (Derbyshire et al., Gene 46:145, 1986; Ner et al., DNA 7:127, 1988).

Unless defined otherwise, all technical and scientific terms used herein have the same meaning as commonly understood by one of ordinary skill in the art to which this disclosure belongs. Singleton, et al., DICTIONARY OF MICROBIOLOGY AND MOLECULAR BIOLOGY, 20 ED., John Wiley and Sons, New York (1994), and Hale & Marham, THE HARPER COLLINS DICTIONARY OF BIOLOGY, Harper Perennial, NY (1991) provide the skilled person with a general dictionary of many of the terms used in this disclosure.

This disclosure is not limited by the exemplary methods and materials disclosed herein, and any methods and materials similar or equivalent to those described herein can be used in the practice or testing of embodiments of this disclosure. Numeric ranges are inclusive of the numbers defining the range. Unless otherwise indicated, any nucleic acid sequences are written left to right in 5' to 3' orientation; amino acid sequences are written left to right in amino to carboxy orientation, respectively.

The headings provided herein are not limitations of the various aspects or embodiments of this disclosure.

Amino acids are referred to herein using the name of the amino acid, the three letter abbreviation or the single letter abbreviation. The term "protein", as used herein, includes proteins, polypeptides, and peptides. As used herein, the term "amino acid sequence" is synonymous with the term "polypeptide" and/or the term "protein". In some instances, the term "amino acid sequence" is synonymous with the term "peptide". In some instances, the term "amino acid sequence" is synonymous with the term "enzyme". The terms "protein" and "polypeptide" are used interchangeably herein. In the present disclosure and claims, the conventional one-letter and three-letter codes for amino acid residues may be used. The 3-letter code for amino acids as defined in conformity with the IUPACIUB Joint Commission on Biochemical Nomenclature (JCBN). It is also understood that a polypeptide may be coded for by more than one nucleotide sequence due to the degeneracy of the genetic code.

Other definitions of terms may appear throughout the specification. Before the exemplary embodiments are described in more detail, it is to be understood that this disclosure is not limited to particular embodiments described, and as such may vary. It is also to be understood that the terminology used herein is for the purpose of describing particular embodiments only, and is not intended to be limiting, since the scope of the present disclosure will be defined only by the appended claims.

Where a range of values is provided, it is understood that each intervening value, to the tenth of the unit of the lower limit unless the context clearly dictates otherwise, between the upper and lower limits of that range is also specifically disclosed. Each smaller range between any stated value or intervening value in a stated range and any other stated or intervening value in that stated range is encompassed within this disclosure. The upper and lower limits of these smaller ranges may independently be included or excluded in the range, and each range where either, neither or both limits are included in the smaller ranges is also encompassed within this disclosure, subject to any specifically excluded limit in the stated range. Where the stated range includes one or both of the limits, ranges excluding either or both of those included limits are also included in this disclosure.

It must be noted that as used herein and in the appended claims, the singular forms "a", "an", and "the" include plural referents unless the context clearly dictates otherwise. Thus, for example, reference to "a modified botulinum neurotoxin A" includes a plurality of such candidate agents and reference to "the modified botulinum neurotoxin A" includes reference to one or more modified botulinum neurotoxin As and equivalents thereof known to those skilled in the art, and so forth.

The publications discussed herein are provided solely for their disclosure prior to the filing date of the present application. Nothing herein is to be construed as an admission that such publications constitute prior art to the claims appended hereto.

## BRIEF DESCRIPTION OF THE DRAWINGS

Embodiments of the invention will now be described, by way of example only, with reference to the following Figures and Examples.

**Figure 1** shows the FDA approved dosages of Dysport® for treating cervical dystonia.

**Figure 2** shows the isoelectric focusing (IEF) gel of cationic constructs.

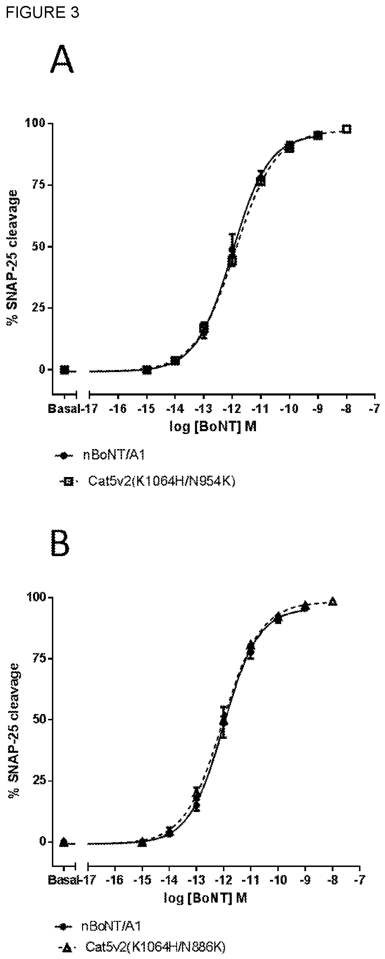

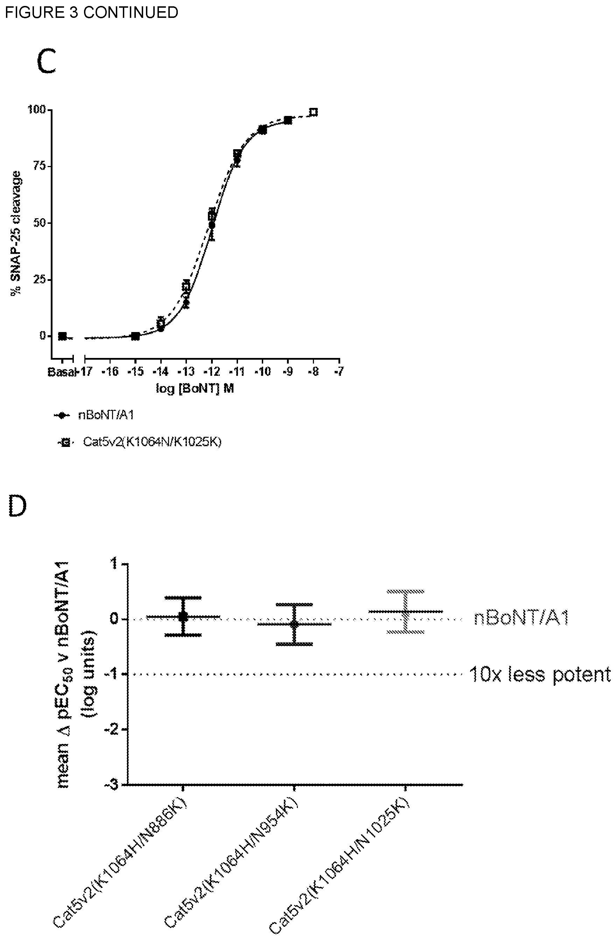

**Figure 3** shows the percentage SNAP-25 cleavage in rat embryonic spinal cord neurons (eSCN) for Cat5v2(K1064H/N954K) (A), Cat5v2(K1064H/N886K) (B) and Cat5v2(K1064H/ N1025K) (C), and summary of pEC50 relative to nBoNT/A1. (A, B, C) Rat embryonic spinal cord neurons were cultured for three weeks and treated with Cat5v4 for 24 h, before Western blotting with SNAP-25 specific antibody. Data is mean $\pm$SEM from three independent experiments in triplicate. (D) Relative potency of Cat5v2(K1064H/N886K), Cat5v2(K1064H/N954K) and Cat5v2(K1064H/ N1025K) to nBoNT/A1 (List Biological Laboratories) in the rat eSCN SNAP-25 cleavage potency assay. Each point corresponds to an individual batch and is a mean of 3 independent pEC50 determinations based on an 8-point concentration response curve (CRC). Each concentration in the CRC was assessed in triplicate. Potency comparisons are made to a mean of List batches, pooled data n=24. Data are mean $\pm$SEM of n=3 batches per Cat5v4.

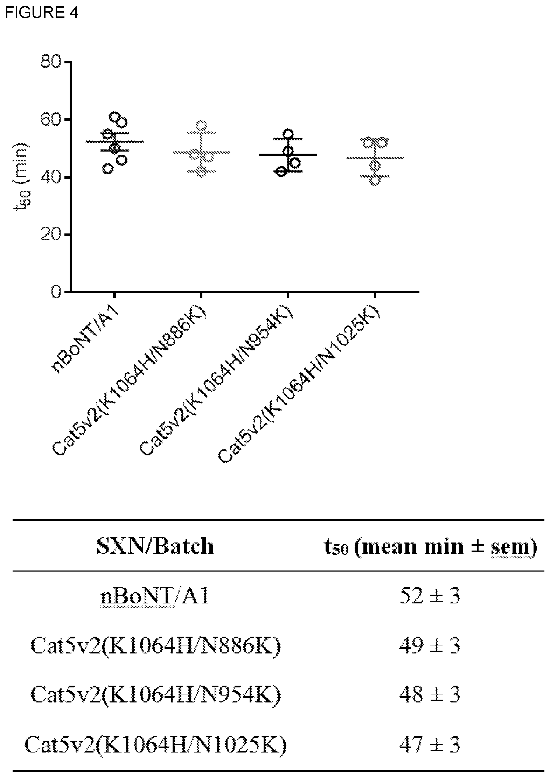

**Figure 4** shows the potency ($t_{50}$) of nBoNT/A1 and Cat5v4 in the mouse phrenic nerve hemi-diaphragm assay (mPNHD). Mouse phrenic nerve hemi-diaphragm tissue was incubated with Cat5v4 or native BoNT/A1 as indicated. Diaphragm contractile force was recorded until the contraction was no longer detectable or after 140 minutes. Each point corresponds to independent determinations. The $t_{50}$ value is the time required to inhibit the contractile force of the mouse hemi-diaphragm by 50%.

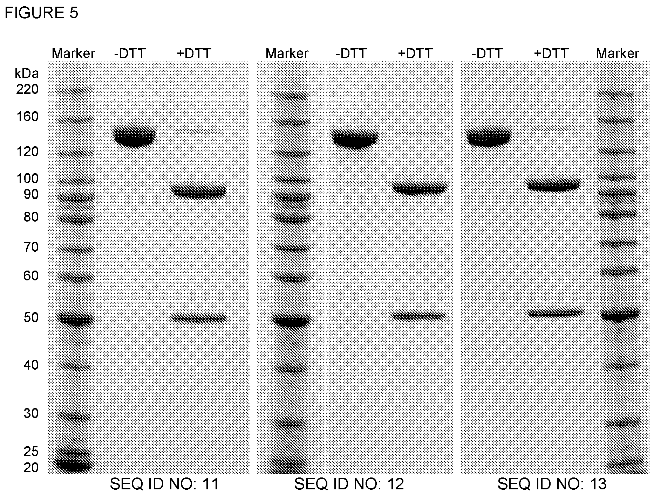

**Figure 5** shows SDS-PAGE of purified recombinant BoNT/AB chimera 1, 2 and 3A (SEQ ID NO: 11, 12 and 13 respectively). Lanes are labelled "Marker" (molecular weight marker), "-DTT" (oxidised BoNT/AB chimera sample), and "+DTT" (reduced BoNT/AB chimera sample).

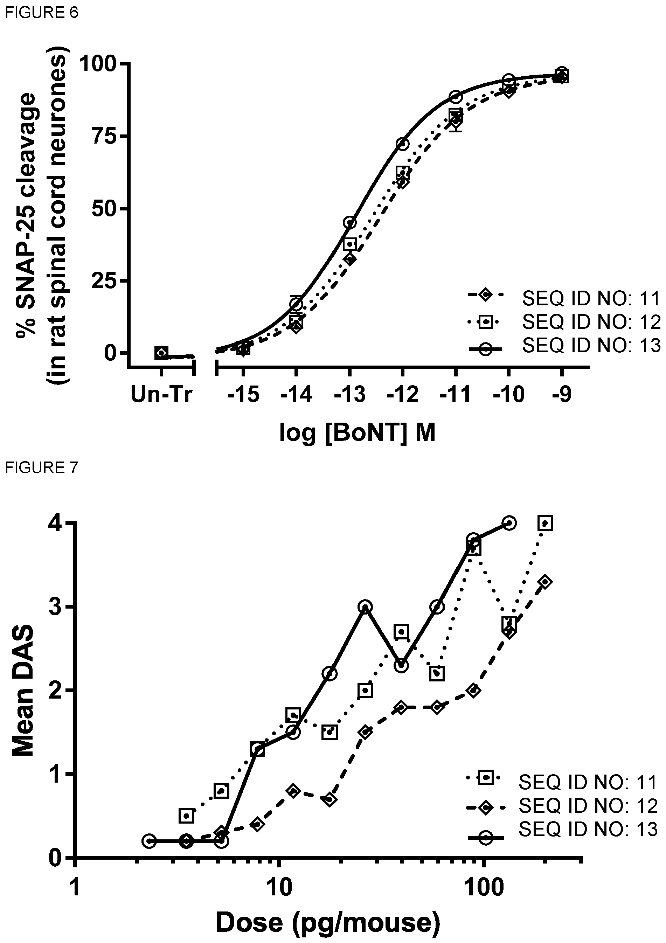

**Figure 6** shows cleavage of SNAP-25 in rat spinal cord neurones by recombinant BoNT/AB chimera 1, 2 and 3A (SEQ ID NO: 11, 12 and 13 respectively, converted into a di-chain form). Cultured rat primary spinal cord neurons (SCN) were exposed to various concentrations of recombinant BoNT/AB chimera 1, 2 or 3A for 24 hours, at 37 °C in a humidified atmosphere with 10% $CO_2$. Cells were then lysed with 1x NuPAGE buffer supplemented with DTT and Benzonase. The samples were transferred to microcentrifuge tubes, heated for 5 min at 90 °C on heat block and stored at -20°C, before analysis of SNAP-25 cleavage by Western blot. SNAP-25 was detected using a polyclonal antibody, that detects both the full length and cleaved forms of SNAP-25 (Sigma #S9684). Anti-rabbit HRP (Sigma #A6154) was used as the secondary antibody.

**Figure 7** shows mouse digit abduction scoring assay. Mice were injected into the gastrocnemius-soleus complex muscles of one hind limb, under short general anaesthesia; muscle weakening was measured on a 0-4 scale using the digit abduction score (DAS). DAS max values were determined for each dose and plotted against dose and the data were fitted to a 4-parameter logistic equation, ED50 and dose leading to DAS 4 (DAS 4 dose) values were determined.

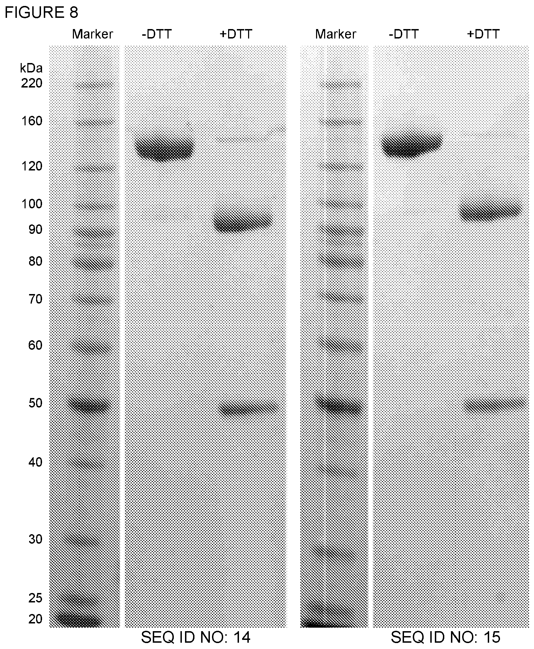

**Figure 8** shows SDS-PAGE of purified recombinant BoNT/AB chimera 3B and 3C (SEQ ID NO: 14 and 15 respectively). Lanes are labelled "Marker" (molecular weight marker), "-DTT" (oxidised BoNT/AB chimera sample), and "+DTT" (reduced BoNT/AB chimera sample).

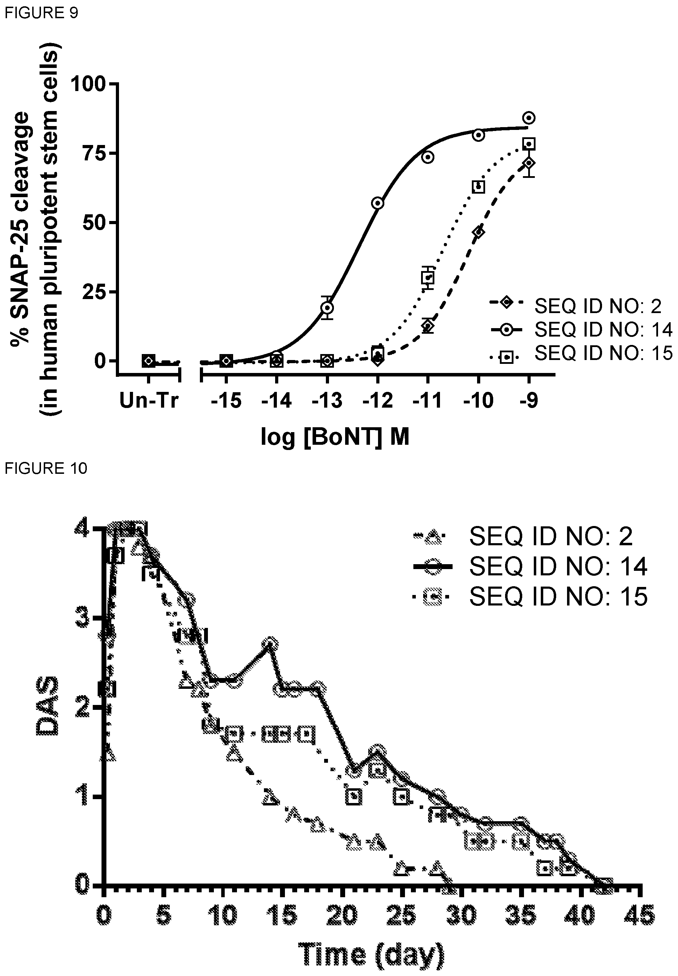

**Figure 9** shows cleavage of SNAP-25 by unmodified BoNT/A and BoNT/AB chimera 3B and 3C (SEQ ID NO: 2, 14 and 15 respectively, converted into a di-chain form) in human induced pluripotent stem cell derived peripheral neurons (PERI.4U - Axiogenesis, Germany). PERI.4U cells were exposed to various concentrations of recombinant BoNT/A, or BoNT/AB chimera 3B or 3C for 24 hours, at 37 °C in a humidified $CO_2$ atmosphere containing 5% $CO_2$. Cells were then lysed with 1x NuPAGE buffer supplemented with DTT and Benzonase. The samples were transferred to microcentrifuge tubes, heated for 5 min at 90 °C on heat block and stored at -20 °C, before analysis of SNAP-25 cleavage by Western blot. SNAP-25 was detected using a polyclonal antibody, that detects both the full length and cleaved forms of SNAP-25 (Sigma #S9684). Anti-rabbit HRP (Sigma #A6154) was used as the secondary antibody.

**Figure 10** shows duration of muscle weakening over time in the mouse digit abduction scoring assay. Mice were injected into the gastrocnemius-soleus complex muscles of one hind limb, under short general anaesthesia; muscle weakening was measured on a 0-4 scale using the digit abduction score (DAS). Animals of the group injected with the lowest dose that induced during the first four days of injection a DAS of 4 were monitored until complete recovery of the muscle weakness to a DAS of 0 (no observed muscle weakness).

## SEQUENCE LISTING

Where an initial Met amino acid residue or a corresponding initial codon is indicated in any of the following SEQ ID NOs, said residue/codon is optional.

SEQ ID NO: 1 (Nucleotide Sequence of Unmodified BoNT/A)

```
ATGCCATTCGTCAACAAGCAATTCAACTACAAAGACCCAGTCAACGGCGTCGACATCGCATACATCAAGATTCCG
AACGCCGGTCAAATGCAGCCGGTTAAGGCTTTTAAGATCCACAACAAGATTTGGGTTATCCCGGAGCGTGACACC
TTCACGAACCCGGAAGAAGGCGATCTGAACCCGCCACCGGAAGCGAAGCAAGTCCCTGTCAGCTACTACGATTCG
ACGTACCTGAGCACGGATAACGAAAAAGATAACTACCTGAAAGGTGTGACCAAGCTGTTCGAACGTATCTACAGC
ACGGATCTGGGTCGCATGCTGCTGACTAGCATTGTTCGCGGTATCCCGTTCTGGGGTGGTAGCACGATTGACACC
GAACTGAAGGTTATCGACACTAACTGCATTAACGTTATTCAACCGGATGGTAGCTATCGTAGCGAAGAGCTGAAT
CTGGTCATCATTGGCCCGAGCGCAGACATTATCCAATTCGAGTGCAAGAGCTTTGGTCACGAGGTTCTGAATCTG
ACCCGCAATGGCTATGGTAGCACCCAGTACATTCGTTTTTCGCCGGATTTTACCTTCGGCTTTGAAGAGAGCCTG
GAGGTTGATACCAATCCGTTGCTGGGTGCGGGCAAATCGCTACCGATCCGGCTGTCACGCTGGCCCATGAACTG
ATCCACGCAGGCCACCGCCTGTACGGCATTGCCATCAACCCAAACCGTGTGTTCAAGGTTAATACGAATGCATAC
TACGAGATGAGCGGCCTGGAAGTCAGCTTCGAAGAACTGCGCACCTTCGGTGGCCATGACGCTAAATTCATTGAC
AGCTTGCAAGAGAATGAGTTCCGTCTGTACTACTATAACAAATTCAAAGACATTGCAAGCACGTTGAACAAGGCC
AAAAGCATCGTTGGTACTACCGCGTCGTTGCAGTATATGAAGAATGTGTTTAAAGAGAAGTACCTGCTGTCCGAG
GATACCTCCGGCAAGTTTAGCGTTGATAAGCTGAAGTTTGACAAACTGTACAAGATGCTGACCGAGATTTACACC
GAGGACAACTTTGTGAAATTCTTCAAAGTGTTGAATCGTAAAACCTATCTGAATTTTGACAAAGCGGTTTTCAAG
ATTAACATCGTGCCGAAGGTGAACTACACCATCTATGACGGTTTTAACCTGCGTAACACCAACCTGGCGGCGAAC
TTTAACGGTCAGAATACGGAAATCAACAACATGAATTTCACGAAGTTGAAGAACTTCACGGGTCTGTTCGAGTTC
TATAAGCTGCTGTGCGTGCGCGGTATCATCACCAGCAAAACCAAAGCCTGGACAAAGGCTACAACAAGGCGCTG
AATGACCTGTGCATTAAGGTAAACAATTGGGATCTGTTCTTTTCGCCATCCGAAGATAATTTTACCAACGACCTG
AACAAGGGTGAAGAAATCACCAGCGATACGAATATTGAAGCAGCGGAAGAGAATATCAGCCTGGATCTGATCCAG
CAGTACTATCTGACCTTTAACTTCGACAATGAACCGGAGAACATTAGCATTGAGAATCTGAGCAGCGACATTATC
GGTCAGCTGGAACTGATGCCGAATATCGAACGTTTCCCGAACGGCAAAAAGTACGAGCTGGACAAGTACACTATG
TTCCATTACCTGCGTGCACAGGAGTTTGAACACGGTAAAAGCCGTATCGCGCTGACCAACAGCGTTAACGAGGCC
CTGCTGAACCCGAGCCGTGTCTATACCTTCTTCAGCAGCGACTATGTTAAGAAAGTGAACAAAGCCACTGAGGCC
GCGATGTTCCTGGGCTGGGTGGAACAGCTGGTATATGACTTCACGGACGAGACGAGCGAAGTGAGCACTACCGAC
AAAATTGCTGATATTACCATCATTATCCCGTATATTGGTCCGGCACTGAACATTGGCAACATGCTGTACAAAGAC
GATTTTGTGGGTGCCCTGATCTTCTCCGGTGCCGTGATTCTGCTGGAGTTCATTCCGGAGATTGCGATCCCGGTG
TTGGGTACCTTCGCGCTGGTGTCCTACATCGCGAATAAGGTTCTGACGGTTCAGACCATCGATAACGCGCTGTCG
AAACGTAATGAAAAATGGGACGAGGTTTACAAATACATTGTTACGAATTGGCTGGCGAAAGTCAATACCCAGATC
GACCTGATCCGTAAGAAAATGAAAGAGGCGCTGGAGAATCAGGCGGAGGCCACCAAAGCAATTATCAACTACCAA
TACAACCAGTACACGGAAGAAGAGAAGAATAACATTAACTTCAATATCGATGATTTGAGCAGCAAGCTGAATGAA
TCTATCAACAAAGCGATGATCAATATCAACAAGTTTTTGAATCAGTGTAGCGTTTCGTACCTGATGAATAGCATG
ATTCCGTATGGCGTCAAACGTCTGGAGGACTTCGACGCCAGCCTGAAAGATGCGTTGCTGAAATACATTTACGAC
AATCGTGGTACGCTGATTGGCCAAGTTGACCGCTTGAAAGACAAAGTTAACAATACCCTGAGCACCGACATCCCA
TTTCAACTGAGCAAGTATGTTGATAATCAACGTCTGTTGAGCACTTTCACCGAGTATATCAAAAACATCATCAAT
ACTAGCATTCTGAACCTGCGTTACGAGAGCAATCATCTGATTGATCTGAGCCGTTATGCAAGCAAGATCAACATC
GGTAGCAAGGTCAATTTTGACCCGATCGATAAGAACCAGATCCAGCTGTTTAATCTGGAATCGAGCAAAATTGAG
GTTATCCTGAAAAACGCCATTGTCTACAACTCCATGTACGAGAATTTCTCCACCAGCTTCTGGATTCGCATCCCG
AAATACTTCAACAGCATTAGCCTGAACAACGAGTATACTATCATCAACTGTATGGAGAACAACAGCGGTTGGAAG
GTGTCTCTGAACTATGGTGAGATCATTTGGACCTTGCAGGACACCCAAGAGATCAAGCAGCGCGTCGTGTTCAAG
TACTCTCAAATGATCAACATTTCCGATTACATTAATCGTTGGATCTTCGTGACCATTACGAATAACCGTCTGAAT
AACAGCAAGATTTACATCAATGGTCGCTTGATCGATCAGAAACCGATTAGCAACCTGGGTAATATCCACGCAAGC
AACAACATTATGTTCAAATTGGACGGTTGCCGCGATACCCATCGTTATATCTGGATCAAGTATTTCAACCTGTTT
GATAAGAACTGAATGAGAAGGAGATCAAAGATTTGTATGACAACCAATCTAACAGCGGCATTTTGAAGGACTTC
TGGGGCGATTATCTGCAATACGATAAGCCGTACTATATGCTGAACCTGTATGATCCGAACAAATATGTGGATGTC
AATAATGTGGGTATTCGTGGTTACATGTATTTGAAGGGTCCGCGTGGCAGCGTTATGACGACCAACATTTACCTG
AACTCTAGCCTGTACCGTGGTACGAAATTCATCATTAAGAAATATGCCAGCGGCAACAAAGATAACATTGTGCGT
AATAACGATCGTGTCTACATCAACGTGGTCGTGAAGAATAAAGAGTACCGTCTGGCGACCAACGCTTCGCAGGCG
GGTGTTGAGAAAATTCTGAGCGCGTTGGAGATCCCTGATGTCGGTAATCTGAGCCAAGTCGTGGTTATGAAGAGC
AAGAACGACCAGGGTATCACTAACAAGTGCAAGATGAACCTGCAAGACAACAATGGTAACGACATCGGCTTTATT
GGTTTCCACCAGTTCAACAATATTGCTAAACTGGTAGCGAGCAATTGGTACAATCGTCAGATTGAGCGCAGCAGC
CGTACTTTGGGCTGTAGCTGGGAGTTTATCCCGGTCGATGATGGTTGGGGCGAACGTCCGCTG
```

SEQ ID NO: 2 (Polypeptide Sequence of Unmodified BoNT/A)

MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTNIEAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPYIGPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNIINTSILNLRYESNHLIDLSRYASKINI
GSKVNFDPIDKNQIQLFNLESSKIEVILKNAIVYNSMYENFSTSFWIRIPKYFNSISLNNEYTIINCMENNSGWK
VSLNYGEIIWTLQDTQEIKQRVVFKYSQMINISDYINRWIFVTITNNRLNNSKIYINGRLIDQKPISNLGNIHAS
NNIMFKLDGCRDTHRYIWIKYFNLFDKELNEKEIKDLYDNQSNSGILKDFWGDYLQYDKPYYMLNLYDPNKYVDV
NNVGIRGYMYLKGPRGSVMTTNIYLNSSLYRGTKFIIKKYASGNKDNIVRNNDRVYINVVVKNKEYRLATNASQA
GVEKILSALEIPDVGNLSQVVVMKSKNDQGITNKCKMNLQDNNGNDIGFIGFHQFNNIAKLVASNWYNRQIERSS
RTLGCSWEFIPVDDGWGERPL

SEQ ID NO: 3 (Nucleotide Sequence of Modified BoNT/A "Cat-A")

ATGCCATTCGTCAACAAGCAATTCAACTACAAAGACCCAGTCAACGGCGTCGACATCGCATACATCAAGATTCCG
AACGCCGGTCAAATGCAGCCGGTTAAGGCTTTTAAGATCCACAACAAGATTTGGGTTATCCCCGGAGCGTGACACC
TTCACGAACCCGGAAGAAGGCGATCTGAACCCGCCACCGGAAGCGAAGCAAGTCCCTGTCAGCTACTACGATTCG
ACGTACCTGAGCACGGATAACGAAAAAGATAACTACCTGAAAGGTGTGACCAAGCTGTTCGAACGTATCTACAGC
ACGGATCTGGGTCGCATGCTGCTGACTAGCATTGTTCGCGGTATCCCGTTCTGGGGTGGTAGCACGATTGACACC
GAACTGAAGGTTATCGACACTAACTGCATTAACGTTATTCAACCGGATGGTAGCTATCGTAGCGAAGAGCTGAAT
CTGGTCATCATTGGCCCGAGCGCAGACATTATCCAAITCGAGTGCAAGAGCTTTGGTCACGAGGTTCTGAATCTG
ACCCGCAATGGCTATGGTAGCACCCAGTACATTCGTITTTCGCCGGATTTTACCTTCGGCTTTGAAGAGAGCCTG
GAGGTTGATACCAATCCGTTGCTGGGTGCGGGCAAAITCGCTACCGATCCGGCTGTCACGCTGGCCCATGAACTG
ATCCACGCAGGCCACCGCCTGTACGGCATTGCCATCAACCCAAACCGTGTGTTCAAGGTTAATACGAATGCATAC
TACGAGATGAGCGGCCTGGAAGTCAGCTTCGAAGAACTGCGCACCTTCGGTGGCCATGACGCTAAATTCATTGAC
AGCTTGCAAGAGAATGAGTTCCGTCTGTACTACTATAACAAATTCAAAGACATTGCAAGCACGTTGAACAAGGCC
AAAAGCATCGTTGGTACTACCGCGTCGTTGCAGTATATGAAGAATGTGTTTAAAGAGAAGTACCTGCTGTCCGAG
GATACCTCCGGCAAGTTTAGCGTTGATAAGCTGAAGITTGACAAACTGTACAAGATGCTGACCGAGATTTACACC
GAGGACAACTTTGTGAAATTCTTCAAAGTGTTGAATCGTAAAACCTATCTGAATTTTGACAAAGCGGTTTTCAAG
ATTAACATCGTGCCGAAGGTGAACTACACCATCTATGACGGTTTTAACCTGCGTAACACCAACCTGGCGGCGAAC
TTTAACGGTCAGAATACGGAAATCAACAACATGAATITCACGAAGTTGAAGAACTTCACGGGTCTGTTCGAGTTC
TATAAGCTGCTGTGCGTGCGCGGTATCATCACCAGCAAAACCAAAAGCCTGGACAAAGGCTACAACAAGGCGCTG
AATGACCTGTGCATTAAGGTAAACAATTGGGATCTGITCTTTTCGCCATCCGAAGATAATTTTACCAACGACCTG
AACAAGGGTGAAGAAATCACCAGCGATACGAATATTGAAGCAGCGGAAGAGAATATCAGCCTGGATCTGATCCAG
CAGTACTATCTGACCTTTAACTTCGACAATGAACCGGAGAACATTAGCATTGAGAATCTGAGCAGCGACATTATC
GGTCAGCTGGAACTGATGCCGAATATCGAACGTTTCCCGAACGGCAAAAAGTACGAGCTGGACAAGTACACTATG
TTCCATTACCTGCGTGCACAGGAGTTTGAACACGGTAAAAGCCGTATCGCGCTGACCAACAGCGTTAACGAGGCC
CTGCTGAACCCCAGCCGTGTCTATACCTTCTTCAGCAGCGACTATGTTAAGAAACTGAACAAGCCACTCAGGCC
GCGATGTTCCTGGGCTGGGTGGAACAGCTGGTATATGACTTCACGGACGAGACGAGCGAAGTGAGCACTACCGAC
AAAATTGCTGATATTACCATCATTATCCCGTATATTGGTCCGGCACTGAACATTGGCAACATGCTGTACAAAGAC
GATTTTGTGGGTGCCCTGATCTTCTCCGGTGCCGTGATTCTGCTGGAGTTCATTCCGGAGATTGCGATCCCGGTG
TTGGGTACCTTCGCGCTGGTGTCCTACATCGCGAATAAGGTTCTGACGGTTCAGACCATCGATAACGCGCTGTCG
AAACGTAATGAAAAATGGGACGAGGTTTACAAATACATTGTTACGAATTGGCTGGCGAAAGTCAATACCCAGATC
GACCTGATCCGTAAGAAAATGAAAGAGGCGCTGGAGAATCAGGCGGAGGCCACCAAAGCAATTATCAACTACCAA
TACAACCAGTACACGGAAGAAGAGAAGAATAACATTAACTTCAATATCGATGATTTGAGCAGCAAGCTGAATGAA
TCTATCAACAAAGCGATGATCAATATCAACAAGTTTITGAATCAGTGTAGCGTTTCGTACCTGATGAATAGCATG
ATTCCGTATGGCGTCAAACGTCTGGAGGACTTCGACGCCAGCCTGAAAGATGCGTTGCTGAAATACATTTACGAC
AATCGTGGTACGCTGATTGGCCAAGTTGACCGCTTGAAAGACAAAGTTAACAATACCCTGAGCACCGACATCCCA
TTTCAACTGAGCAAGTATGTTGATAATCAACGTCTGITGAGCACTTTCACCGAGTATATCAAAAACATCATCAAT
ACTAGCATTCTGAACCTGCGTTACGAGAGCAAGCATCTGATTGATCTGAGCCGTTATGCTAGCAAGATCAACATC
GGTAGCAAGGTCAATTTTGACCCGATCGATAAGAACCAGATCCAGCTGTTTAATCTGGAATCGAGCAAAATTGAG
GTTATCCTGAAAAAGGCCATTGTCTACAACTCCATGITACGAGAATTTCTCCACCAGCTTCTGGATTCGCATCCCG
AAATACTTCAACAAGATTAGCCTGAACAACGAGTATACTATCATCAACTGTATGGAGAACAACAGCGGTTGGAAG

```
GTGTCTCTGAACTATGGTGAGATCATTTGGACCTTGCAGGACACCAAAGAGATCAAGCAGCGCGTCGTGTTCAAG
TACTCTCAAATGATCAACATTTCCGATTACATTAATCGTTGGATCTTCGTGACCATTACGAATAACCGTCTGAAT
AAGAGCAAGATTTACATCAATGGTCGCTTGATCGATCAGAAACCGATTAGCAACCTGGGTAATATCCACGCAAGC
AACAAGATTATGTTCAAATTGGACGGTTGCCGCGATACCCATCGTTATATCTGGATCAAGTATTTCAACCTGTTT
GATAAAGAACTGAATGAGAAGGAGATCAAAGATTTGTATGACAACCAATCTAACAGCGGCATTTTGAAGGACTTC
TGGGGCGATTATCTGCAATACGATAAGCCGTACTATATGCTGAACCTGTATGATCCGAACAAATATGTGGATGTC
AATAATGTGGGTATTCGTGGTTACATGTATTTGAAGGGTCCGCGTGGCAGCGTTATGACGACCAACATTTACCTG
AACTCTAGCCTGTACCGTGGTACGAAATTCATCATTAAGAAATATGCCAGCGGCAACAAAGATAACATTGTGCGT
AATAACGATCGTGTCTACATCAACGTGGTCGTGAAGAATAAAGAGTACCGTCTGGCGACCAACGCTTCGCAGGCG
GGTGTTGAGAAAATTCTGAGCGCGTTGGAGATCCCTGATGTCGGTAATCTGAGCCAAGTCGTGGTTATGAAGAGC
AAGAACGACAAGGGTATCACTAACAAGTGCAAGATGAACCTGCAAGACAACAATGGTAACGACATCGGCTTTATT
GGTTTCCACCAGTTCAACAATATTGCTAAACTGGTAGCGAGCAATTGGTACAATCGTCAGATTGAGCGCAGCAGC
cGTACTTTGGGCTGTAGCTGGGAGTTTATCCCGGTCGATGATGGTTGGGGCGAACGTCCGCTG
```

SEQ ID NO: 4 (Polypeptide Sequence of Modified BoNT/A "Cat-A")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTNIEAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPYIGPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNIINTSILNLRYESKHLIDLSRYASKINI
GSKVNFDPIDKNQIQLFNLESSKIEVILKKAIVYNSMYENFSTSFWIRIPKYFNKISLNNEYTIINCMENNSGWK
VSLNYGEIIWTLQDTKEIKQRVVFKYSQMINISDYINRWIFVTITNNRLNKSKIYINGRLIDQKPISNLGNIHAS
NKIMFKLDGCRDTHRYIWIKYFNLFDKELNEKEIKDLYDNQSNSGILKDFWGDYLQYDKPYYMLNLYDPNKYVDV
NNVGIRGYMYLKGPRGSVMTTNIYLNSSLYRGTKFIIKKYASGNKDNIVRNNDRVYINVVVKNKEYRLATNASQA
GVEKILSALEIPDVGNLSQVVVMKSKNDKGITNKCKMNLQDNNGNDIGFIGFHQFNNIAKLVASNWYNRQIERSS
RTLGCSWEFIPVDDGWGERPL
```

SEQ ID NO: 5 (Nucleotide Sequence of Modified BoNT/A "Cat-B")

```
ATGCCATTCGTCAACAAGCAATTCAACTACAAAGACCCAGTCAACGGCGTCGACATCGCATACATCAAGATTCCG
AACGCCGGTCAAATGCAGCCGGTTAAGGCTTTTAAGATCCACAACAAGATTTGGGTTATCCCGGAGCGTGACACC
TTCACGAACCCGGAAGAAGGCGATCTGAACCCGCCACCGGAAGCGAAGCAAGTCCCTGTCAGCTACTACGATTCG
ACGTACCTGAGCACGGATAACGAAAAGATAACTACCTGAAAGGTGTGACCAAGCTGTTCGAACGTATCTACAGC
ACGGATCTGGGTCGCATGCTGCTGACTAGCATTGTTCGCGGTATCCCGTTCTGGGGTGGTAGCACGATTGACACC
GAACTGAAGGTTATCGACACTAACTGCATTAACGTTATTCAACCGGATGGTAGCTATCGTAGCGAAGAGCTGAAT
CTGGTCATCATTGGCCCGAGCGCAGACATTATCCAATCGAGTGCAAGAGCTTTGGTCACGAGGTTCTGAATCTG
ACCCGCAATGGCTATGGTAGCACCCAGTACATTCGTTTTCGCCGGATTTTACCTTCGGCTTTGAAGAGAGCCTG
GAGGTTGATACCAATCCGTTGCTGGGTGCGGGCAAATCGCTACCGATCCGGCTGTCACGCTGGCCCATGAACTG
ATCCACGCAGGCCACCGCCTGTACGGCATTGCCATCAACCCAAACCGTGTGTTCAAGGTTAATACGAATGCATAC
TACGAGATGAGCGGCCTGGAAGTCAGCTTCGAAGAACTGCGCACCTTCGGTGGCCATGACGCTAAATTCATTGAC
AGCTTGCAAGAGAATGAGTTCCGTCTGTACTACTATAACAAATTCAAAGACATTGCAAGCACGTTGAACAAGGCC
AAAAGCATCGTTGGTACTACCGCGTCGTTGCAGTATATGAAGAATGTGTTTAAAGAGAAGTACCTGCTGTCCGAG
GATACCTCCGGCAAGTTTAGCGTTGATAAGCTGAAGTTGACAAACTGTACaAGATGCTGACCGAGATTTACACC
GAGGACAACTTTGTGAAATTCTTCAAAGTGTTGAATCGTAAAACCTATCTGAATTTTGACAAAGCGGTTTTCAAG
ATTAACATCGTGCCGAAGGTGAACTACACCATCTATGACGGTTTTAACCTGCGTAACACCAACCTGGCGGCGAAC
TTTAACGGTCAGAATACGGAAATCAACAACATGAATTTCACGAAGTTGAAGAACTTCACGGGTCTGTTCGAGTTC
TATAAGCTGCTGTGCGTGCGCGGTATCATCACCAGCAAAACCAAAGCCTGGACAAAGGCTACAACAAGGCGCTG
AATGACCTGTGCATTAAGGTAAACAATTGGGATCTGTTCTTTTCGCCATCCGAAGATAATTTTACCAACGACCTG
AACAAGGGTGAAGAAATCACCAGCGATACGAATATTGAAGCAGCGGAAGAGAATATCAGCCTGGATCTGATCCAG
CAGTACTATCTGACCTTTAACTTCGACAATGAACCGGAGAACATTAGCATTGAGAATCTGAGCAGCGACATTATC
GGTCAGCTGGAACTGATGCCGAATATCGAACGTTTCCCGAACGGCAAAAAGTACGAGCTGGACAAGTACACTATG
TTCCATTACCTGCGTGCACAGGAGTTTGAACACGGTAAAAGCCGTATCGCGCTGACCAACAGCGTTAACGAGGCC
CTGCTGAACCCGAGCCGTGTCTATACCTTCTTCAGCAGCGACTATGTTAAGAAAGTGAACAAAGCCACTGAGGCC
GCGATGTTCCTGGGCTGGGTGGAACAGCTGGTATATGACTTCACGGACGAGACGAGCGAAGTGAGCACTACCGAC
```

```
AAAATTGCTGATATTACCATCATTATCCCGTATATTGGTCCGGCACTGAACATTGGCAACATGCTGTACAAAGAC
GATTTTGTGGGTGCCCTGATCTTCTCCGGTGCCGTGATTCTGCTGGAGTTCATTCCGGAGATTGCGATCCCGGTG
TTGGGTACCTTCGCGCTGGTGTCCTACATCGCGAATAAGGTTCTGACGGTTCAGACCATCGATAACGCGCTGTCG
AAACGTAATGAAAAATGGGACGAGGTTTACAAATACATTGTTACGAATTGGCTGGCGAAAGTCAATACCCAGATC
GACCTGATCCGTAAGAAAATGAAAGAGGCGCTGGAGAATCAGGCGGAGGCCACCAAAGCAATTATCAACTACCAA
TACAACCAGTACACGGAAGAAGAGAAGAATAACATTAACTTCAATATCGATGATTTGAGCAGCAAGCTGAATGAA
TCTATCAACAAAGCGATGATCAATATCAACAAGTTTTTGAATCAGTGTAGCGTTTCGTACCTGATGAATAGCATG
ATTCCGTATGGCGTCAAACGTCTGGAGGACTTCGACGCCAGCCTGAAAGATGCGTTGCTGAAATACATTTACGAC
AaTCGTGGTACGCTGATTGGCCAAGTTGACCGCTTGAAAGACAAAGTTAACAATACCCTGAGCACCGACATCCCA
TTTCAACTGAGCAAGTATGTTGATAATCAACGTCTGTTGAGCACTTTCACCGAGTATATCAAAAACATCATCAAT
ACTAGCATTCTGAACCTGCGTTACGAGAGCAATCATCTGATTGATCTGAGCCGTTATGCTAGCAAGATCAACATC
GGTAGCAAGGTCAATTTTGACCCGATCGATAAGAACCAGATCCAGCTGTTTAATCTGGAATCGAGCAAAATTGAG
GTTATCCTGAAAAAGGCCATTGTCTACAACTCCATGTACGAGAATTTCTCCACCAGCTTCTGGATTCGCATCCCG
AAATACTTCAAGAAGATTAGCCTGAACAACGAGTATACTATCATCAACTGTATGGAGAACAACAGCGGTTGGAAG
GTGTCTCTGAACTATGGTGAGATCATTTGGACCTTGCAGGACACCAAAGAGATCAAGCAGCGCGTCGTGTTCAAG
TACTCTCAAATGATCAACATTTCCGATTACATTAATCGTTGGATCTTCGTGACCATTACGAATAACCGTCTGAAT
AAGAGCAAGATTTACATCAATGGTCGCTTGATCGATCAGAAACCGATTAGCAACCTGGGTAATATCCACGCAAGC
AACAAGATTATGTTCAAATTGGACGGTTGCCGCGATACCCATCGTTATATCTGGATCAAGTATTTCAACCTGTTT
GATAAAGAACTGAATGAGAAGGAGATCAAAGATTTGTATGACAACCAATCTAACAGCGGCATTTTGAAGGACTTC
TGGGGCGATTATCTGCAATACGATAAGCCGTACTATATGCTGAACCTGTATGATCCGAACAAATATGTGGATGTC
AATAATGTGGGTATTCGTGGTTACATGTATTTGAAGGGTCCGCGTGGCAGCGTTATGACGACCAACATTTACCTG
AACTCTAGCCTGTACCGTGGTACGAAATTCATCATTAAGAAATATGCCAGCGGCAACAAAGATAACATTGTGCGT
AATAACGATCGTGTCTACATCAACGTGGTCGTGAAGAATAAAGAGTACCGTCTGGCGACCAACGCTTCGCAGGCG
GGTGTTGAGAAAATTCTGAGCGCGTTGGAGATCCCTGATGTCGGTAATCTGAGCCAAGTCGTGGTTATGAAGAGC
AAGAACGACAAGGGTATCACTAACAAGTGCAAGATGAACCTGCAAGACAACAATGGTAACGACATCGGCTTTATT
GGTTTCCACCAGTTCAACAATATTGCTAAACTGGTAGCGAGCAATTGGTACAATCGTCAGATTGAGCGCAGCAGC
CGTACTTTGGGCTGTAGCTGGGAGTTTATCCCGGTCGATGATGGTTGGGGCGAACGTCCGCTG
```

SEQ ID NO: 6 (Polypeptide Sequence of Modified BoNT/A "Cat-B")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTN_EAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPY_GPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNIINTSILNLRYESNHLIDLSRYASKINI
GSKVNFDPIDKNQIQLFNLESSKIEVILKKAIVYNSMYENFSTSFWIRIPKYFKKISLNNEYTIINCMENNSGWK
VSLNYGEIIWTLQDTKEIKQRVVFKYSQMINISDYINRWIFVTITNNRLNKSKIYINGRLIDQKPISNLGNIHAS
NKIMFKLDGCRDTHRYIWIKYFNLFDKELNEKEIKDLYDNQSNSGILKDFWGDYLQYDKPYYMLNLYDPNKYVDV
NNVGIRGYMYLKGPRGSVMTTNIYLNSSLYRGTKFI_KKYASGNKDNIVRNNDRVYINVVVKNKEYRLATNASQA
GVEKILSALEIPDVGNLSQVVVMKSKNDKGITNKCKMNLQDNNGNDIGFIGFHQFNNIAKLVASNWYNRQIERSS
RTLGCSWEFIPVDDGWGERPL
```

SEQ ID NO: 7 (Nucleotide Sequence of Modified BoNT/A "Cat-C")

```
ATGCCATTCGTCAACAAGCAATTCAACTACAAAGACCCAGTCAACGGCGTCGACATCGCATACATCAAGATTCCG
AACGCCGGTCAAATGCAGCCGGTTAAGGCTTTTAAGATCCACAACAAGATTGGGTTATCCCGGAGCGTGACACC
TTCACGAACCCGGAAGAAGGCGATCTGAACCCGCCACCGGAAGCGAAGCAAGTCCCTGTCAGCTACTACGATTCG
ACGTACCTGAGCACGGATAACGAAAAGATAACTACCTGAAAGGTGTGACCAAGCTGTTCGAACGTATCTACAGC
ACGGATCTGGGTCGCATGCTGCTGACTAGCATTGTTCGCGGTATCCCGTTCTGGGGTGGTAGCACGATTGACACC
GAACTGAAGGTTATCGACACTAACTGCATTAACGTTATTCAACCGGATGGTAGCATCGTAGCGAAGAGCTGAAT
CTGGTCATCATTGGCCCGAGCGCAGACATTATCCAA_TCGAGTGCAAGAGCTTTGGTCACGAGGTTCTGAATCTG
ACCCGCAATGGCTATGGTAGCACCCAGTACATTCGT_TTTCGCCGGATTTTACCTTCGGCTTTGAAGAGAGCCTG
GAGGTTGATACCAATCCGTTGCTGGGTGCGGGCAAA_TCGCTACCGATCCGGCTGTCACGCTGGCCCATGAACTG
ATCCACGCAGGCCACCGCCTGTACGGCATTGCCATCAACCCAAACCGTGTGTTCAAGGTTAATACGAATGCATAC
TACGAGATGAGCGGCCTGGAAGTCAGCTTCGAAGAACTGCGCACCTTCGGTGGCCATGACGCTAAATTCATTGAC
```

```
AGCTTGCAAGAGAATGAGTTCCGTCTGTACTACTATAACAAATTCAAAGACATTGCAAGCACGTTGAACAAGGCC
AAAAGCATCGTTGGTACTACCGCGTCGTTGCAGTATATGAAGAATGTGTTTAAAGAGAAGTACCTGCTGTCCGAG
GATACCTCCGGCAAGTTTAGCGTTGATAAGCTGAAGTTTGACAAACTGTACAAGATGCTGACCGAGATTTACACC
GAGGACAACTTTGTGAAATTCTTCAAAGTGTTGAATCGTAAAACCTATCTGAATTTTGACAAAGCGGTTTTCAAG
ATTAACATCGTGCCGAAGGTGAACTACACCATCTATGACGGTTTTAACCTGCGTAACACCAACCTGGCGGCGAAC
TTTAACGGTCAGAATACGGAAATCAACAACATGAATTTCACGAAGTTGAAGAACTTCACGGGTCTGTTCGAGTTC
TATAAGCTGCTGTGCGTGCGCGGTATCATCACCAGCAAAACCAAAAGCCTGGACAAAGGCTACAACAAGGCGCTG
AATGACCTGTGCATTAAGGTAAACAATTGGGATCTGTTCTTTTCGCCATCCGAAGATAATTTTACCAACGACCTG
AACAAGGGTGAAGAAATCACCAGCGATACGAATATTGAAGCAGCGGAAGAGAATATCAGCCTGGATCTGATCCAG
CAGTACTATCTGACCTTTAACTTCGACAATGAACCGGAGAACATTAGCATTGAGAATCTGAGCAGCGACATTATC
GGTCAGCTGGAACTGATGCCGAATATCGAACGTTTCCCGAACGGCAAAAAGTACGAGCTGGACAAGTACACTATG
TTCCATTACCTGCGTGCACAGGAGTTTGAACACGGTAAAAGCCGTATCGCGCTGACCAACAGCGTTAACGAGGCC
CTGCTGAACCCGAGCCGTGTCTATACCTTCTTCAGCAGCGACTATGTTAAGAAAGTGAACAAAGCCACTGAGGCC
GCGATGTTCCTGGGCTGGGTGGAACAGCTGGTATATGACTTCACGGACGAGACGAGCGAAGTGAGCACTACCGAC
AAAATTGCTGATATTACCATCATTATCCCGTATATTGGTCCGGCACTGAACATTGGCAACATGCTGTACAAAGAC
GATTTTGTGGGTGCCCTGATCTTCTCCGGTGCCGTGATTCTGCTGGAGTTCATTCCGGAGATTGCGATCCCGGTG
TTGGGTACCTTCGCGCTGGTGTCCTACATCGCGAATAAGGTTCTGACGGTTCAGACCATCGATAACGCGCTGTCG
AAACGTAATGAAAAATGGGACGAGGTTTACAAATACATTGTTACGAATTGGCTGGCGAAAGTCAATACCCAGATC
GACCTGATCCGTAAGAAATGAAAGAGGCGCTGGAGAATCAGGCGGAGGCCACCAAAGCAATTATCAACTACCAA
TACAACCAGTACACGGAAGAAGAGAAGAATAACATTAACTTCAATATCGATGATTTGAGCAGCAAGCTGAATGAA
TCTATCAACAAAGCGATGATCAATATCAACAAGTTTTTGAATCAGTGTAGCGTTTCGTACCTGATGAATAGCATG
ATTCCGTATGGCGTCAAACGTCTGGAGGACTTCGACGCCAGCCTGAAAGATGCGTTGCTGAAATACATTTACGAC
AATCGTGGTACGCTGATTGGCCAAGTTGACCGCTTGAAAGACAAAGTTAACAATACCCTGAGCACCGACATCCCA
TTTCAACTGAGCAAGTATGTTGATAATCAACGTCTGTTGAGCACTTTCACCGAGTATATCAAAAACATCATCAAT
ACTAGCATTCTGAACCTGCGTTACGAGAGCAATCATCTGATTGATCTGAGCCGTTATGCTAGCAAGATCAACATC
GGTAGCAAGGTCAATTTTGACCCGATCGATAAGAACCAGATCCAGCTGTTTAATCTGGAATCGAGCAAAATTGAG
GTTATCCTGAAAAAGGCCATTGTCTACAACTCCATGTACGAGAATTTCTCCACCAGCTTCTGGATTCGCATCCCG
AAATACTTCAACAAGATTAGCCTGAACAACGAGTATACTATCATCAACTGTATGGAGAACAACAGCGGTTGGAAG
GTGTCTCTGAACTATGGTGAGATCATTTGGACCTTGCAGGACACCAAAGAGATCAAGCAGCGCGTCGTGTTCAAG
TACTCTCAAATGATCAACATTTCCGATTACATTAATCGTTGGATCTTCGTGACCATTACGAATAACCGTCTGAAG
AAGAGCAAGATTTACATCAATGGTCGCTTGATCGATCAGAAACCGATTAGCAACCTGGGTAATATCCACGCAAGC
AACAAGATTATGTTCAAATTGGACGGTTGCCGCGATACCCATCGTTATATCTGGATCAAGTATTTCAACCTGTTT
GATAAGAACTGAATGAGAAGGAGATCAAAGATTTGTATGACAACCAATCTAACAGCGGCATTTTGAAGGACTTC
TGGGGCGATTATCTGCAATACGATAAGCCGTACTATATGCTGAACCTGTATGATCCGAACAAATATGTGGATGTC
AATAATGTGGGTATTCGTGGTTACATGTATTTGAAGGGTCCGCGTGGCAGCGTTATGACGACCAACATTTACCTG
AACTCTAGCCTGTACCGTGGTACGAAATTCATCATTAAGAAATATGCCAGCGGCAACAAAGATAACATTGTGCGT
AATAACGATCGTGTCTACATCAACGTGGTCGTGAAGAATAAAGAGTACCGTCTGGCGACCAACGCTTCGCAGGCG
GGTGTTGAGAAATTCTGAGCGCGTTGGAGATCCCTGATGTCGGTAATCTGAGCCAAGTCGTGGTTATGAAGAGC
AAGAACGACAAGGGTATCACTAACAAGTGCAAGATGAACCTGCAAGACAACAATGGTAACGACATCGGCTTTATT
GGTTTCCACCAGTTCAACAATATTGCTAAACTGGTAGCGAGCAATTGGTACAATCGTCAGATTGAGCGCAGCAGC
CGTACTTTGGGCTGTAGCTGGGAGTTTATCCCGGTCGATGATGGTTGGGGCGAACGTCCGCTG
```

SEQ ID NO: 8 (Polypeptide Sequence of Modified BoNT/A "Cat-C")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTNIEAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPYIGPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNIINTSILNLRYESNHLIDLSRYASKINI
GSKVNFDPIDKNQIQLFNLESSKIEVILKKAIVYNSMYENFSTSFWIRIPKYFNKISLNNEYTIINCMENNSGWK
VSLNYGEIIWTLQDTKEIKQRVVFKYSQMINISDYINRWIFVTITNNRLKKSKIYINGRLIDQKPISNLGNIHAS
NKIMFKLDGCRDTHRYIWIKYFNLFDKELNEKEIKDLYDNQSNSGILKDFWGDYLQYDKPYYMLNLYDPNKYVDV
NNVGIRGYMYLKGPRGSVMTTNIYLNSSLYRGTKFIIKKYASGNKDNIVRNNDRVYINVVVKNKEYRLATNASQA
```

GVEKILSALEIPDVGNLSQVVVMKSKNDKGITNKCKMNLQDNNGNDIGFIGFHQFNNIAKLVASNWYNRQIERSS
RTLGCSWEFIPVDDGWGERPL

SEQ ID NO: 9 (Nucleotide Sequence of Modified BoNT/A "Cat-D")

```
ATGCCATTCGTCAACAAGCAATTCAACTACAAAGACCCAGTCAACGGCGTCGACATCGCATACATCAAGATTCCG
AACGCCGGTCAAATGCAGCCGGTTAAGGCTTTTAAGATCCACAACAAGATTTGGGTTATCCCGGAGCGTGACACC
TTCACGAACCCGGAAGAAGGCGATCTGAACCCGCCACCGGAAGCGAAGCAAGTCCCTGTCAGCTACTACGATTCG
ACGTACCTGAGCACGGATAACGAAAAGATAACTACCTGAAAGGTGTGACCAAGCTGTTCGAACGTATCTACAGC
ACGGATCTGGGTCGCATGCTGCTGACTAGCATTGTTCGCGGTATCCCGTTCTGGGGTGGTAGCACGATTGACACC
GAACTGAAGGTTATCGACACTAACTGCATTAACGTTATTCAACCGGATGGTAGCTATCGTAGCGAAGAGCTGAAT
CTGGTCATCATTGGCCCGAGCGCAGACATTATCCAATCGAGTGCAAGAGCTTTGGTCACGAGGTTCTGAATCTG
ACCCGCAATGGCTATGGTAGCACCCAGTACATTCGTTTTTCGCCGGATTTTACCTTCGGCTTTGAAGAGAGCCTG
GAGGTTGATACCAATCCGTTGCTGGGTGCGGGCAAATCGCTACCGATCCGGCTGTCACGCTGGCCCATGAACTG
ATCCACGCAGGCCACCGCCTGTACGGCATTGCCATCAACCCAAACCGTGTGTTCAAGGTTAATACGAATGCATAC
TACGAGATGAGCGGCCTgGAAGTCAGCTTCGAAGAACTGCGCACCTTCGGTGGCCATGACGCTAAATTCATTGAC
AGCTTGCAAGAGAATGAGTTCCGTCTGTACTACTATAACAAATTCAAAGACATTGCAAGCACGTTGAACAAGGCC
AAAAGCATCGTTGGTACTACCGCGTCGTTGCAGTATATGAAGAATGTGTTTAAAGAGAAGTACCTGCTGTCCGAG
GATACCTCCGGCAAGTTTAGCGTTGATAAGCTGAAGTTTGACAAACTGTACAAGATGCTGACCGAGATTTACACC
GAGGACAACTTTGTGAAATTCTTCAAaGTGTTGAATCGTAAAACCTATCTGAATTTTGACAAAGCGGTTTTCaAG
ATTAACATCGTGCCGAAGGTGAACTACACCATCTATGACGGTTTTAACCTGCGTAACACCAACCTGGCGGCGAAC
TTTAACGGTCAGAATACGGAAATCAACAACATGAATTTCACGAAGTTGAAGAACTTCACGGGTCTGTTCGAGTTC
TATAAGCTGCTGTGCGTGCGCGGTATCATCACCAGCAAAACCAAAGCCTGGACAAAGGCTACAACAAGGCGCTG
AATGACCTGTGCATTAAGGTAAACAATTGGGATCTGTTCTTTTCGCCATCCGAAGATAATTTTACCAACGACCTG
AACAAGGGTGAAGAAATCACCAGCGATACGAATATTGAAGCAGCGGAAGAGAATATCAGCCTGGATCTGATCCAG
CAGTACTATCTGACCTTTAACTTCGACAATGAACCGGAGAACATTAGCATTGAGAATCTGAGCAGCGACATTATC
GGTCAGCTGGAACTGATGCCGAATATCGAACGTTTCCCGAACGGCAAAAAGTACGAGCTGGACAAGTACACTATG
TTCCATTACCTGCGTGCACAGGAGTTTGAACACGGTAAAAGCCGTATCGCGCTGACCAACAGCGTTAACGAGGCC
CTGCTGAACCCGAGCCGTGTCTATACCTTCTTCAGCAGCGACTATGTTAAGAAAGTGAACAAAGCCACTGAGGCC
GCGATGTTCCTGGGCTGGGTGGAACAGCTGGTATATGACTTCACGGACGAGACGAGCGAAGTGAGCACTACCGAC
AAAaTTGCTGATaTTACCATCATTATCCCGTATATTGGTCCGGCACTGAACATTGGCAACATGCTGTACAAAGAC
GATTTTGTGGGTGCCCTGATCTTCTCCGGTGCCGTGATTCTGCTGGAGTTCATTCCGGAGATTGCGATCCCGGTG
TTGGGTACCTTCGCGCTGGTGTCCTACATCGCGAATAAGGTTCTGACGGTTCAGACCATCGATAACGCGCTGTCG
AAACGTAATGAAAAATGGGACGAGGTTTACAAATACATTGTTACGAATTGGCTGGCGAAAGTCaATACCCAGATC
GACCTGATCCGTAAGAAAATGAAAGAGGCGCTGGAGAATCAGGCGGAGGCCACCAAAGCAATTATCAACTACCAA
TACAACCAGTACACGGAAGAAGAGAAGAATAACATTAACTTCAATATCGATGATTTGAGCAGCAAGCTGAATGAA
TCTATCAACAAAGCGATGATCAATATCAACAAGTTTTTGAATCAGTGTAGCGTTTCGTACCTGATGAATAGCATG
ATTCCGTATGGCGTCAAACGTCTGGAGGACTTCGACGCCAGCCTGAAAGATGCGTTGCTGAAATACATTTACGAC
AATCGTGGTACGCTGATTGGCCAAGTTGACCGCTTGAAAGACAAAGTTAACAATACCCTGAGCACCGACATCCCA
TTTCAACTGAGCAAGTATGTTGATAATCAACGTCTGTTGAGCACTTTCACCGAGTATATCAAAAACATCATCAAT
ACTAGCATTCTGAACCTGCGTTACGAGAGCAATCATCTGATtGATCTGAGCCGTTATGCAAGCAAGATCAACATC
GGTAGCAAGGTCAATTTTGACCCGATCGATAAGAACCAGATCCAGCTGTTTAATCTGGAATCGAGCAAAATTGAG
GTTATCCTGAAAAACGCCATTGTCTACAACTCCATGTACGAGAATTTCTCCACCAGCTTCTGGATTCGCATCCCG
AAATACTTCAACAGCATTAGCCTGAACAACGAGTATACTATCATCAACTGTATGGAGAACAACAGCGGTTGGAAG
GTGTCTCTGAACTATGGTGAGATCATTTGGACCTTGCAGGACACCCAAGAGATCAAGCAGCGCGTCGTGTTCAAG
TACTCTCAAATGATCAACATTTCCGATTACATTAATCGTTGGATCTTCGTGACCATTACGAATAACCGTCTGAAT
AACAGCAAGATTTACATCAATGGTCGCTTGATCGATCAGAAACCGATTAGCAACCTGGGTAATATCCACGCAAGC
AACAACATTATGTTCAAATTGGACGGTTGCCGCGATACCCATCGTTATATCTGGATCAAGTATTTCAACCTGTTT
GATAAAGAACTGAATGAGAAGGAGATCAAAGATTTGTATGACAACCAATCTAACAGCGGCATTTTGAAGGACTTC
TGGGGCGATTATCTGCAATACGATAAGCCGTACTATATGCTGAACCTGTATGATCCGAACAAATATGTGGATGTC
AATAATGTGGGTATTCGTGGTTACATGTATTTGAAGGGTCCGCGTGGCAGCGTTATGACGACCAACATTTACCTG
AACTCTAGCCTGTACCGTGGTACGAAATTCATCATTAAGAAATATGCCAGCGGCAACAAAGATAACATTGTGCGT
AATAACGATCGTGTCTACATCAACGTGGTCGTGAAGCGTAAAGAGTACCGTCTGGCGACCAACGCTTCGCAGGCG
GGTGTTGAGAAAATTCTGAGCGCGTTGGAGATCCCTCGTGTCCGTCGTCTGAGCCAAGTCGTGGTTATGAAGAGC
AAGAACGACCAGGGTATCACTAACAAGTGCAAGATGAACCTGCAAGACCGTCGTGGTAACGACATCGGCTTTATT
GGTTTCCACCAGTTCAACAATATTGCTAAACTGGTAGCGAGCAATTGGTACAATCGTCAGATTGAGCGCCGTAGC
CGTCGTTTGGGCTGTAGCTGGGAGTTTATCCCGGTCGATGATGGTTGGGGCGAACGTCCGCTG
```

SEQ ID NO: 10 (Polypeptide Sequence of Modified BoNT/A "Cat-D")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
```

```
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTN_EAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPY_GPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNIINTSILNLRYESNHLIDLSRYASKINI
GSKVNFDPIDKNQIQLFNLESSKIEVILKNAIVYNSMYENFSTSFWIRIPKYFNSISLNNEYTIINCMENNSGWK
VSLNYGEIIWTLQDTQEIKQRVVFKYSQMINISDYINRWIFVTITNNRLNNSKIYINGRLIDQKPISNLGNIHAS
NNIMFKLDGCRDTHRYIWIKYFNLFDKELNEKEIKDLYDNQSNSGILKDFWGDYLQYDKPYYMLNLYDPNKYVDV
NNVGIRGYMYLKGPRGSVMTTNIYLNSSLYRGTKFI_KKYASGNKDNIVRNNDRVYINVVVKRKEYRLATNASQA
GVEKILSALEIPRVRRLSQVVVMKSKNDQGITNKCKMNLQDRRGNDIGFIGFHQFNNIAKLVASNWYNRQIERRS
RRLGCSWEFIPVDDGWGERPL
```

SEQ ID NO: 11 (Polypeptide Sequence of Modified BoNT/A "Chimera 1")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTN_EAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPY_GPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKSEILNNIILNLRYKDNNLIDLSGYGAKVE
VYDGVELNDKNQFKLTSSANSKIRVTQNQNIIFNSVFLDFSVSFWIRIPKYKNDGIQNYIHNEYTIINCMKNNSG
WKISIRGNRIIWTLIDINGKTKSVFFEYNIREDISEYINRWFFVTITNNLNNAKIYINGKLESNTDIKDIREVIA
NGEIIFKLDGDIDRTQFIWMKYFSIFNTELSQSNIEERYKIQSYSEYLKDFWGNPLMYNKEYYMFNAGNKNSYIK
LKKDSPVGEILTRSKYNQNSKYINYRDLYIGEKFIIRRKSNSQSINDDIVRKEDYIYLDFFNLNQEWRVYTYKYF
KKEEMKLFLAPIYDSDEFYNTIQIKEYDEQPTYSCQLLFKKDEESTDEIGLIGIHRFYESGIVFEEYKDYFCISK
WYLKEVKRKPYNLKLGCNWQFIPKDEGWTEHHHHHHHHHH
```

SEQ ID NO: 12 (Polypeptide Sequence of Modified BoNT/A "Chimera 2")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTN_EAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPY_GPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNIIELGGGGSELSEILNNIILNLRYKDNN
LIDLSGYGAKVEVYDGVELNDKNQFKLTSSANSKIRVTQNQNIIFNSVFLDFSVSFWIRIPKYKNDGIQNYIHNE
YTIINCMKNNSGWKISIRGNRIIWTLIDINGKTKSVFFEYNIREDISEYINRWFFVTITNNLNNAKIYINGKLES
NTDIKDIREVIANGEIIFKLDGDIDRTQFIWMKYFS_FNTELSQSNIEERYKIQSYSEYLKDFWGNPLMYNKEYY
MFNAGNKNSYIKLKKDSPVGEILTRSKYNQNSKYINYRDLYIGEKFIIRRKSNSQSINDDIVRKEDYIYLDFFNL
NQEWRVYTYKYFKKEEMKLFLAPIYDSDEFYNTIQIKEYDEQPTYSCQLLFKKDEESTDEIGLIGIHRFYESGIV
FEEYKDYFCISKWYLKEVKRKPYNLKLGCNWQFIPKDEGWTEHHHHHHHHHH
```

SEQ ID NO: 13 (Polypeptide Sequence of Modified BoNT/A "Chimera 3A")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTN_EAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPY_GPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNILNNIILNLRYKDNNLIDLSGYGAKVEV
YDGVELNDKNQFKLTSSANSKIRVTQNQNIIFNSVFLDFSVSFWIRIPKYKNDGIQNYIHNEYTIINCMKNNSGW
KISIRGNRIIWTLIDINGKTKSVFFEYNIREDISEY_NRWFFVTITNNLNNAKIYINGKLESNTDIKDIREVIAN
GEIIFKLDGDIDRTQFIWMKYFSIFNTELSQSNIEERYKIQSYSEYLKDFWGNPLMYNKEYYMFNAGNKNSYIKL
KKDSPVGEILTRSKYNQNSKYINYRDLYIGEKFIIRRKSNSQSINDDIVRKEDYIYLDFFNLNQEWRVYTYKYFK
KEEMKLFLAPIYDSDEFYNTIQIKEYDEQPTYSCQLLFKKDEESTDEIGLIGIHRFYESGIVFEEYKDYFCISKW
YLKEVKRKPYNLKLGCNWQFIPKDEGWTEHHHHHHHHHH
```

SEQ ID NO: 14 (Polypeptide Sequence of Modified BoNT/A "Chimera 3B")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTN_EAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDETSEVSTTDKIADITIIIPY_GPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNILNNIILNLRYKDNNLIDLSGYGAKVEV
YDGVELNDKNQFKLTSSANSKIRVTQNQNIIFNSVFLDFSVSFWIRIPKYKNDGIQNYIHNEYTIINCMKNNSGW
KISIRGNRIIWTLIDINGKTKSVFFEYNIREDISEY_NRWFFVTITNNLNNAKIYINGKLESNTDIKDIREVIAN
GEIIFKLDGDIDRTQFIWMKYFSIFNTELSQSNIEERYKIQSYSEYLKDFWGNPLMYNKEYYMFNAGNKNSYIKL
KKDSPVGEILTRSKYNQNSKYINYRDLYIGEKFIIRRKSNSQSINDDIVRKEDYIYLDFFNLNQEWRVYTYKYFK
KEEMKLFLAPIYDSDEFYNTIQIKEYDEQPTYSCQLLFKKDEESTDEIGLIGIHRFYESGIVFEEYKDYFCISKW
YLKEVKRKPYNLKLGCNWQFIPKDEGWTE
```

SEQ ID NO: 15 (Polypeptide Sequence of Modified BoNT/A "Chimera 3C")

```
MPFVNKQFNYKDPVNGVDIAYIKIPNAGQMQPVKAFKIHNKIWVIPERDTFTNPEEGDLNPPPEAKQVPVSYYDS
TYLSTDNEKDNYLKGVTKLFERIYSTDLGRMLLTSIVRGIPFWGGSTIDTELKVIDTNCINVIQPDGSYRSEELN
LVIIGPSADIIQFECKSFGHEVLNLTRNGYGSTQYIRFSPDFTFGFEESLEVDTNPLLGAGKFATDPAVTLAHEL
IHAGHRLYGIAINPNRVFKVNTNAYYEMSGLEVSFEELRTFGGHDAKFIDSLQENEFRLYYYNKFKDIASTLNKA
KSIVGTTASLQYMKNVFKEKYLLSEDTSGKFSVDKLKFDKLYKMLTEIYTEDNFVKFFKVLNRKTYLNFDKAVFK
INIVPKVNYTIYDGFNLRNTNLAANFNGQNTEINNMNFTKLKNFTGLFEFYKLLCVRGIITSKTKSLDKGYNKAL
NDLCIKVNNWDLFFSPSEDNFTNDLNKGEEITSDTNIEAAEENISLDLIQQYYLTFNFDNEPENISIENLSSDII
GQLELMPNIERFPNGKKYELDKYTMFHYLRAQEFEHGKSRIALTNSVNEALLNPSRVYTFFSSDYVKKVNKATEA
AMFLGWVEQLVYDFTDTETSEVSTTDKIADITIIIPYIGPALNIGNMLYKDDFVGALIFSGAVILLEFIPEIAIPV
LGTFALVSYIANKVLTVQTIDNALSKRNEKWDEVYKYIVTNWLAKVNTQIDLIRKKMKEALENQAEATKAIINYQ
YNQYTEEEKNNINFNIDDLSSKLNESINKAMININKFLNQCSVSYLMNSMIPYGVKRLEDFDASLKDALLKYIYD
NRGTLIGQVDRLKDKVNNTLSTDIPFQLSKYVDNQRLLSTFTEYIKNILNNIILNLRYKDNNLIDLSCYGAKVEV
YDGVELNDKNQFKLTSSANSKIRVTQNQNIIFNSVFLDFSVSFWIRIPKYKNDGIQNYIHNEYTIINCMKNNSGW
KISIRGNRIIWTLIDINGKTKSVFFEYNIREDISEYINRWFFVTITNNLNNAKIYINGKLESNTDIKDIREVIAN
GEIIFKLDGDIDRTQFIWMKYFSIFNTELSQSNIEERYKIQSYSEYLKDFWGNPLMYNKEYYMFNAGNKNSYIKL
KKDSPVGEILTRSKYNQNSKYINYRDLYIGEKFIIRRKSNSQSINDDIVRKEDYIYLDFFNLNQEWRVYTYKYFK
KEEEKLFLAPISDSDEFYNTIQIKEYDEQPTYSCQLLFKKDEESTDEIGLIGIHRFYESGIVFEEYKDYFCISKW
YLKEVKRKPYNLKLGCNWQFIPKDEGWTE
```

SEQ ID NO: 16 (Polypeptide Sequence of BoNT/B)

```
MPVTINNFNYNDPIDNNNIIMMEPPFARGTGRYYKAFKITDRIWIIPERYTFGYKPEDFNKSSGIFNRDVCEYYD
PDYLNTNDKKNIFLQTMIKLFNRIKSKPLGEKLLEMIINGIPYLGDRRVPLEEFNTNIASVTVNKLISNPGEVER
KKGIFANLIIFGPGPVLNENETIDIGIQNHFASREGFGGIMQMKFCPEYVSVFNNVQENKGASIFNRRGYFSDPA
LILMHELIHVLHGLYGIKVDDLPIVPNEKKFFMQSTDAIQAEELYTFGGQDPSIITPSTDKSIYDKVLQNFRGIV
DRLNKVLVCISDPNININIYKNKFKDKYKFVEDSEGKYSIDVESFDKLYKSLMFGFTETNIAENYKIKTRASYFS
DSLPPVKIKNLLDNEIYTIEEGFNISDKDMEKEYRGQNKAINKQAYEEISKEHLAVYKIQMCKSVKAPGICIDVD
NEDLFFIADKNSFSDDLSKNERIEYNTQSNYIENDFPINELILDTDLISKIELPSENTESLTDFNVDVPVYEKQP
AIKKIFTDENTIFQYLYSQTFPLDIRDISLTSSFDDALLFSNKVYSFFSMDYIKTANKVVEAGLFAGWVKQIVND
FVIEANKSNTMDKIADISLIVPYIGLALNVGNETAKGNFENAFEIAGASILLEFIPELLIPVVGAFLLESYIDNK
NKIIKTIDNALTKRNEKWSDMYGLIVAQWLSTVNTQFYTIKEGMYKALNYQAQALEEIIKYRYNIYSEKEKSNIN
IDFNDINSKLNEGINQAIDNINNFINGCSVSYLMKKMIPLAVEKLLDFDNTLKKNLLNYIDENKLYLIGSAEYEK
SKVNKYLKTIMPFDLSIYTNDTILIEMFNKYNSEILNNIILNLRYKDNNLIDLSGYGAKVEVYDGVELNDKNQFK
LTSSANSKIRVTQNQNIIFNSVFLDFSVSFWIRIPKYKNDGIQNYIHNEYTIINCMKNNSGWKISIRGNRIIWTL
IDINGKTKSVFFEYNIREDISEYINRWFFVTITNNLNNAKIYINGKLESNTDIKDIREVIANGEIIFKLDGDIDR
TQFIWMKYFSIFNTELSQSNIEERYKIQSYSEYLKDFWGNPLMYNKEYYMFNAGNKNSYIKLKKDSPVGEILTRS
KYNQNSKYINYRDLYIGEKFIIRRKSNSQSINDDIVRKEDYIYLDFFNLNQEWRVYTYKYFKKEEEKLFLAPISD
SDEFYNTIQIKEYDEQPTYSCQLLFKKDEESTDEIGLIGIHRFYESGIVFEEYKDYFCISKWYLKEVKRKPYNLK
LGCNWQFIPKDEGWTE
```

## EXAMPLES

## EXAMPLE 1

### Cloning, Expression and Purification

The nucleotide sequence SEQ ID NO: 1, which encodes wild-type BoNT/A (SEQ ID NO: 2) was mutated to introduce the following substitutions to form the four constructs shown in Table 1 below:

Table 1. Constructs.

| Construct | Mutations | Nucleotide Sequence | Polypeptide Sequence |
|---|---|---|---|
| Cat-A | N930K, S955K, Q991K, N1026K, N1052K, Q1229K, N886K | 3 | 4 |
| Cat-B | N930K, S955K, Q991K, N1026K, N1052K, Q1229K, N954K | 5 | 6 |

(continued)

| Construct | Mutations | Nucleotide Sequence | Polypeptide Sequence |
|---|---|---|---|
| Cat-C | N930K, S955K, Q991K, N1026K, N1052K, Q1229K, N1025K | 7 | 8 |
| Cat-D* | N1188R, D1213R, G1215R, N1216R, N1242R, N1243R, S1274R, T1277R | 9 | 10 |
| *Cat-D had a calculated pI of 7.45, and a molecular weight of 149,859. | | | |

DNA constructs encoding the modified BoNT/A molecules above were synthesised, cloned into the pJ401 expression vector and then transformed into BL21 (DE3) *E. coli.* This allowed for soluble over-expression of the recombinant Cat-A, Cat-B, Cat-C, and Cat-D proteins in BL21(DE3) *E. coli.*

The recombinant modified BoNTs were purified using classical chromatography techniques from the *E. coli* lysates. An initial purification step using a cation-exchange resin was employed, followed by an intermediate purification step using a hydrophobic interaction resin. The recombinant modified BoNT single-chain was then cleaved by proteolysis, resulting in the activated di-chain modified BoNT. A final purification step was then employed to remove remaining contaminants. Suitable techniques are taught in WO2015/166242, WO2017055274A1, EP2524963B1, EP2677029B1, and US10087432B2.

## EXAMPLE 2

## Characterization of Purified Modified BoNT/A

The modified BoNTs described in Example 1 above were characterised experimentally as follows.

Measurement of the pI showed that the modified BoNTs had an isoelectric point greater than that of unmodified (native) BoNT/A1 - see Figure 2 and Table 2 below.

Table 2. Modified BoNT/A pI values.

| BoNT/A1 molecule | pI (calculated) | pI (observed) |
|---|---|---|
| Modified, "Cat-A" [Cat5v2(K1064H/N886K] (SEQ ID NO: 4 converted into a di-chain form) | 6.9 | ~8.0 |
| Modified, "Cat-B" [Cat5v2(K1064/N954K)] (SEQ ID NO: 6 converted into a di-chain form) | 6.9 | ~8.0 |
| Modified, "Cat-C" [Cat5v2(K1064H/N 1025K)] (SEQ ID NO: 8 converted into a di-chain form) | 6.9 | 7.8-8.0 |
| Native BoNT/A1 [rBoNT/A1] (SEQ ID NO: 2 converted into a di-chain form) | 6.05 | ~7.4 |

The ability of the modified BoNTs to enter neurons and cleave SNAP-25 (the target of BoNT/A1) was assessed using rat embryonic spinal cord neurons (eSCN). Figure 3 shows that the modified BoNTs retained the same ability to enter the neuron and cleave SNAP-25 as native BoNT/A1.

Potency of the modified BoNTs was further assessed using the mouse phrenic nerve hemi-diaphragm assay (mPNHD). Figure 4 shows that the modified BoNTs retained the same ability to inhibit the contractile abilities of the mouse hemi-diaphragm as native BoNT/A1.

The *in vivo* mouse Digital Abduction Score (DAS) assay was used to assess potency as well as safety relative to native BoNT/A1. Both molecules (Cat-A [SEQ ID NO: 4 converted into a di-chain form] and Cat-B [SEQ ID NO: 6 converted into a di-chain form]) displayed a higher safety ratio relative to native BoNT/A1 and were slightly more potent. These data are presented in Table 3 below:

Table 3. DAS assay and safety ratio.

| Molecule | DAS ED$_{50}$ (pg/mouse) | Dose DAS 4 (pg/mouse) | Dose for -10% ∆BW (pg/mouse) | Safety Ratio |
|---|---|---|---|---|
| Native BoNT/A1 (n=5) (SEQ ID NO: 2 converted into a di-chain form) | 2 | 10-20 | 9.9-14.5 | 7 |
| Modified, "Cat-A" (SEQ ID NO: 4 converted into a di-chain form) | 1.16 | 10-20 | 27.4 | 24 |
| Modified, "Cat-B" (SEQ ID NO: 6 converted into a di-chain form) | 1.79 | 25 | 47.6 | 27 |

- DAS ED$_{50}$: Calculated dose inducing a DAS 2
- Dose DAS 4: Experimental dose inducing a DAS 4
- BW: Body weight
- Dose for -10% ∆BW: Calculated dose inducing a decrease of 10% on BW in comparison to BW at D0
- Safety Ratio: Dose for -10% ∆BW / DAS ED$_{50}$

The Safety Ratio is a measure of a negative effect of BoNT treatment (weight loss) with respect to potency (half maximal digital abduction score (DAS)). It is calculated as the ratio between - 10% Body Weight (BW) and the DAS ED$_{50}$, where -10%BW refers to the amount of BoNT (pg/animal) required for a 10% decrease in body weight, and ED$_{50}$ refers to the amount of BoNT (pg/animal) that will produce a DAS of 2.

The DAS assay is performed by injection of 20 µl of modified BoNT/A, formulated in Gelatin Phosphate Buffer, into the mouse gastrocnemius/soleus complex, followed by assessment of Digit Abduction as previously reported by Aoki (Aoki KR, Toxicon 39: 1815-1820; 2001).

## EXAMPLE 3

### Cloning, Expression and Purification of Modified BoNT/A (BoNT/AB Chimeras)

BoNT/AB chimeric constructs 1, 2, 3A, 3B, and 3C (SEQ ID NO: 11 to 15, respectively) were constructed from DNA encoding the parent serotype molecule and appropriate oligonucleotides using standard molecular biology techniques. These were then cloned into the pJ401 expression vector with or without a C-terminal His$_{10}$-tag and transformed into BLR (DE3) *E. coli* cells for over-expression. These cells were grown at 37 °C and 225 RPM shaking in 2 L baffled conical flasks containing 1 L modified Terrific Broth (mTB) supplemented with the appropriate antibiotic. Once the A$_{600}$ reached >0.5, the incubator temperature was decreased to 16 °C, and then induced with 1 mM IPTG an hour later for 20 h at 225 RPM shaking, to express the recombinant BoNT/AB construct.

Harvested cells were lysed by ultrasonication and clarified by centrifugation at 4500 RPM for 1 h at 4 °C. The recombinant BoNT/AB chimeric molecules were then extracted in ammonium sulphate and purified by standard fast protein liquid chromatography (FPLC) techniques. This involved using a hydrophobic interaction resin for capture and an anion-exchange resin for the intermediate purification step. The partially purified molecules were then proteolytically cleaved with endoproteinase Lys-C to yield the active di-chain. This was further purified with a second hydrophobic interaction resin to obtain the final BoNT/AB chimera.

For BoNT/AB chimeric molecules with a decahistadine tag (H$_{10}$) (chimera 1, 2, 3A), the capture step employed the use of an immobilised nickel resin instead of the hydrophobic interaction resin.

The sequence of each chimera is presented in Table 4.

Table 4 - chimeric BoNT/AB constructs

| Molecule | SEQ ID NO | Sequence |
|---|---|---|
| Chimera 1 | 11 | A1:1-871 + B1:858-1291 (E1191M/S1199Y) + His$_{10}$-tag |
| Chimera 2 | 12 | A1:1-874 + ELGGGGSEL + B1:858-1291 (E1191M/S1199Y) + His$_{10}$-tag |
| Chimera 3A | 13 | A1:1-872 + B1: 860-1291 (E1191M/S1199Y) + His$_{10}$-tag |
| Chimera 3B | 14 | A1:1-872 + B1: 860-1291 (E1191M/S1199Y) |
| Chimera 3C | 15 | A1:1-872 + B1: 860-1291 |

## EXAMPLE 4

### Comparison of BoNT/AB chimera 1, 2 and 3A

BoNT/AB chimera 1, 2 and 3A which have a C-terminal $His_{10}$ tag and E1191M/S1199Y double mutation were purified as described in Example 3 (Figure 5) and tested for functional activity.

RAT SPINAL CORD NEURONS SNAP-25 CLEAVAGE ASSAY

Primary cultures of rat spinal cord neurons (SCN) were prepared and grown, for 3 weeks, in 96 well tissue culture plates (as described in: Masuyer *et al.,* 2011, J. Struct. Biol. Structure and activity of a functional derivative of Clostridium botulinum neurotoxin B; and in: Chaddock *et al.,* 2002, Protein Expr. Purif. Expression and purification of catalytically active, non-toxic endopeptidase derivatives of Clostridium botulinum toxin type A). Serial dilutions of BoNT/AB were prepared in SCN feeding medium. The growth medium from the wells to be treated was collected and filtered (0.2 μm filter). 125 μL of the filtered medium was added back to each test well. 125 μL of diluted toxin was then added to the plate (triplicate wells). The treated cells were incubated at 37 °C, 10% $CO_2$, for 24 $\pm$ 1 h).

Analysis of BoNT activity using the SNAP-25 cleavage assay

Following treatment, BoNT was removed and cells were washed once in PBS (Gibco, UK). Cells were lysed in 1x NuPAGE lysis buffer (Life Technologies) supplemented with 0.1 M dithiothreitol (DTT) and 250 units/mL benzonase (Sigma). Lysate proteins were separated by SDS-PAGE and transferred to nitrocellulose membranes. Membranes were probed with a primary antibody specific for SNAP-25 (Sigma #S9684) which recognizes uncleaved SNAP-25 as well as SNAP-25 cleaved by the BoNT/A endopeptidase. The secondary antibody used was an HRP-conjugated anti-rabbit IgG (Sigma #A6154). Bands were detected by enhanced chemiluminescence and imaged using a pXi6 Access (Synoptics, UK). The intensity of bands was determined using GeneTools software (Syngene, Cambridge, UK) and the percentage of SNAP-25 cleaved at each concentration of BoNT calculated. Data were fitted to a 4-parameter logistic equation and $pEC_{50}$ calculated using GraphPad Prism version 6 (GraphPad).

Table 5 below provides the $pEC_{50}$ values determined for Chimera 1, 2 and 3A in the rat SCN SNAP-25 cleavage assay. These results show that the three BoNT/AB chimeras retained the ability to enter rat spinal cord neurons and cleave their target substrate. However, chimera 3A was more potent than chimera 1 and 2 in this assay (see also Figure 6).

Table 5. $pEC_{50}$ values.

|  | $pEC_{50}$ $\pm$SEM |
|---|---|
| Chimera 1 | 12.42 $\pm$0.04 |
| Chimera 2 | 12.57 $\pm$0.01 |
| Chimera 3A | 12.89 $\pm$0.04 |

DIGIT ABDUCTION SCORING (DAS) ASSAY

The method to measure the activity of BoNT/AB chimera 1, 2 and 3A in the DAS assay is based on the startled response toe spreading reflex of mice, when suspended briefly by the tail. This reflex is scored as Digit Abduction Score (DAS) and is inhibited after administration of BoNT into the gastrocnemius-soleus muscles of the hind paw. Mice are suspended briefly by the tail to elicit a characteristic startled response in which the animal extends its hind limb and abducts its hind digits. (Aoki et al. 1999, Eur. J. Neurol.; 6 (suppl. 4) S3-S10).

On the day of injection, mice were anaesthetized in an induction chamber receiving isoflurane 3% in oxygen. Each mouse received an intramuscular injection of BoNT/AB chimera or vehicle (phosphate buffer containing 0.2 % gelatine) in the gastrocnemius-soleus muscles of the right hind paw.

Following neurotoxin injection, the varying degrees of digit abduction were scored on a scale from zero to four, where 0= normal and 4= maximal reduction in digit abduction and leg extension. $ED_{50}$ was determined by nonlinear adjustment analysis using average of maximal effect at each dose. The mathematical model used was the 4 parameters logistic model.

DAS was performed every 2 hours during the first day after dosing; thereafter it was performed 3 times a day for 4 days.

Figure 7 shows the fitted curves for chimera 1, 2 and 3A (SEQ ID NO: 11, 12 and 13 converted into a di-chain form, respectively). The chimera 3A curve is shifted to the left, meaning lower doses of chimera 3A achieved a similar DAS response compared to chimera 1 and 2, therefore showing that chimera 3A is more potent than the others in the mouse DAS assay; see also the table below (Table 6) that provides the values for the calculated $ED_{50}$ and the dose leading to DAS 4 (highest score) for each chimera.

Table 6 below provides the $ED_{50}$ and DAS 4 doses determined for unmodified recombinant BoNT/A1 (rBoNT/A1 - SEQ ID NO: 2 converted into a di-chain form) and chimeras 1, 2 and 3A in the mouse DAS assay. These results show that of the three chimeras, chimera 3A has the highest in vivo potency in inducing muscle weakening. Studies shown in Figure 7 and Table 6 were performed in mice obtained from Charles River laboratories.

Table 6. $ED_{50}$ values.

|  | $ED_{50}$ (pg/mouse) | DAS 4 dose (pg/mouse) |
|---|---|---|
| rBoNT/A1 | 1 | 5 |
| Chimera 1 | 23 | 200 |
| Chimera 2 | 89 | >300 |
| Chimera 3A | 18 | 133 |

## EXAMPLE 5

## Comparison of BoNT/AB Chimera 3B, 3C and Unmodified BoNT/A1

Untagged BoNT/AB chimera 3B and 3C, respectively with and without the presence of the E1191M/S1199Y double mutation (SEQ ID NO: 14 and 15) were purified as described in Example 3 (Figure 8), and tested for functional activity using unmodified BoNT/A (SEQ ID NO: 2 converted into a di-chain form) as a reference.

HUMAN PLURIPOTENT STEM CELLS SNAP-25 CLEAVAGE ASSAY

Cryopreserved PERI.4U-cells were purchased from Axiogenesis (Cologne, Germany). Thawing and plating of the cells were performed as recommended by the manufacturer. Briefly, cryovials containing the cells were thawed in a water bath at 37° C for 2 minutes. After gentle resuspension the cells were transferred to a 50 mL tube. The cryovial was washed with 1 mL of Peri.4U® thawing medium supplied by the manufacturer and the medium was transferred drop-wise to the cell suspension to the 50 mL tube, prior to adding a further 2 mL of Peri.4U® thawing medium drop-wise to the 50 mL tube. Cells were then counted using a hemocytometer. After this, a further 6 mL of Peri.4U® thawing medium was added to the cell suspension. A cell pellet was obtained by centrifugation at 260 xg (e.g. 1,100 RPM) for 6 minutes at room temperature. Cells were then resuspended in complete Peri.4U® culture medium supplied by the manufacturer. Cells were plated at a density of 50,000 to 150,000 cells per $cm^2$ on cell culture plates coated with poly-L-ornithine and laminin. Cells were cultured at 37 °C in a humidified $CO_2$ atmosphere, and medium was changed completely every 2-3 days during culture.

For toxin treatment, serial dilutions of BoNTs were prepared in Peri.4U® culture medium. The medium from the wells to be treated was collected and filtered (0.2 μm filter). 125 μL of the filtered medium was added back to each test well. 125 μL of diluted toxin was then added to the plate (triplicate wells). The treated cells were incubated at 37 °C, 10% $CO_2$, for 48 ± 1 h).

Analysis of BoNT activity using the SNAP-25 cleavage assay

Following treatment, BoNT was removed and cells were washed once in PBS (Gibco, UK). Cells were lysed in 1x NuPAGE lysis buffer (Life Technologies) supplemented with 0.1 M dithiothreitol (DTT) and 250 units/mL benzonase (Sigma). Lysate proteins were separated by SDS-PAGE and transferred to nitrocellulose membranes. Membranes were probed with a primary antibody specific for SNAP-25 (Sigma #S9684) which recognizes uncleaved SNAP-25 as well as SNAP-25 cleaved by the BoNT/A endopeptidase. The secondary antibody used was an HRP-conjugated anti-rabbit IgG (Sigma #A6154). Bands were detected by enhanced chemiluminescence and imaged using a pXi6 Access (Synoptics, UK). The intensity of bands was determined using GeneTools software (Syngene, Cambridge, UK) and the percentage of SNAP-25 cleaved at each concentration of BoNT calculated. Data were fitted to a 4-parameter logistic equation and $pEC_{50}$ calculated using GraphPad Prism version 6 (GraphPad).

Figure 9 shows that chimera 3B and 3C displayed greater potency than rBoNT/A1 in cleaving SNAP-25 in induced human pluripotent stem cells but the former significantly more so. This can be explained by the double mutation which increases the affinity of chimera 3B for the human synaptotagmin II protein receptor present in these cells (Figure 9, Table 7).

Table 7. pEC$_{50}$ values.

|  | pEC$_{50}$ ±SEM |
|---|---|
| rBoNT/A1 | 10.21 ±0.05 |
| Chimera 3B | 12.38 ±0.06 |
| Chimera 3C | 10.72 ±0.08 |

DIGIT ABDUCTION SCORING (DAS) ASSAY - SAFETY RATIO

The method to measure the activity of BoNTs in the DAS assay is based on the startled response toe spreading reflex of mice, when suspended briefly by the tail. This reflex is scored as Digit Abduction Score (DAS) and is inhibited after administration of BoNT into the gastrocnemius-soleus muscles of the hind paw. Mice are suspended briefly by the tail to elicit a characteristic startled response in which the animal extends its hind limb and abducts its hind digits. (Aoki et al. 1999, Eur. J. Neurol.; 6 (suppl. 4) S3-S10).

On the day of injection, mice were anaesthetized in an induction chamber receiving isoflurane 3% in oxygen. Each mouse received an intramuscular injection of BoNT or vehicle (phosphate buffer containing 0.2 % gelatine) in the gastrocnemius-soleus muscles of the right hind paw.

Following neurotoxin injection, the varying degrees of digit abduction were scored on a scale from zero to four, where 0= normal and 4= maximal reduction in digit abduction and leg extension. ED$_{50}$ was determined by nonlinear adjustment analysis using average of maximal effect at each dose. The mathematical model used was the 4 parameters logistic model.

DAS was performed every 2 hours during the first day after dosing; thereafter it was performed 3 times a day for 4 days for all doses. Animals of the groups injected with vehicle and the lowest dose that induced during the first four days of injection a DAS of 4 were thereafter monitored until complete recovery of the muscle weakness to a DAS of 0 (no observed muscle weakness).

For calculation of the safety ratio all animals were weighed the day before toxin injection (D0) and thereafter once daily throughout the duration of the study. The average body weight, its standard deviation, and the standard error mean were calculated daily for each dose-group. To obtain the safety ratio for a BoNT (-10%ΔBW/ED$_{50}$), the dose at which at any time during the study the average weight of a dose-group was lower than 10% of the average weight at D0 of that same dose-group was divided by the ED$_{50}$ for the BoNT studied. The lethal dose was defined as the dose at which one or more of the animals within that dose-group died.

Figure 10 shows the duration of muscle weakening over time in the mouse digit abduction scoring assay for unmodified BoNT/A, chimera 3B and chimera 3C (SEQ ID NO: 2, 14 and 15 converted into a di-chain form), showing that the chimera has longer duration of action.

Table 8 below provides the ED$_{50}$ and DAS 4 doses determined for rBoNT/A1 and chimeras 3B and 3C in the mouse DAS assay. The table also provide the total duration of action for the DAS 4 dose until complete recovery of the muscle weakness to a DAS of 0 (no observed muscle weakness). In addition, the table shows the mouse lethal dose and the safety ratio (-10%ΔBW/ED$_{50}$), as defined in the text above. In comparison to rBoNT/A1, chimeras 3B and 3C have longer duration of action, a better safety ratio, and a higher lethal dose. Studies shown in Figure 10 and Table 8 were performed in mice obtained from Janvier laboratories.

Table 8. DAS and Safety Ratios of the BoNT/AB chimeras.

|  | ED$_{50}$ (DAS 2) Dose (pg/mouse) | DAS 4 dose (pg/mouse) | Total duration of action (day) with lowest DAS 4 dose | Mouse lethal dose (pg) | Safety ratio (-10% ΔBW/ED$_{50}$) |
|---|---|---|---|---|---|
| rBoNT/A1 | 0.9 | 2.3 | 29 | 18 | 4.5 |
| Chimera 3B | 8.0 | 89 | 42 | 200 | 14.1 |
| Chimera 3C | 5.0 | 26 | 42 | 8.9 | 7.4 |

**EXAMPLE 6**

**Pre-Clinical Testing of Modified BoNT/A (SEQ ID NO: 4 converted into a di-chain form)**

The modified BoNT/A "Cat-A" (SEQ ID NO: 4 converted into a di-chain form) was subjected to additional pre-clinical testing.

**Materials & Methods**

Rat Digit Abduction Score (DAS) Assay

To assess the effects of modified BoNT/A (SEQ ID NO: 4 converted into a di-chain form) on *in vivo* muscular activity, dose-response studies were conducted using the rat DAS assay. The rat DAS assay is based on the toe spreading reflex, a characteristic startle response, when the animal is briefly grasped. Following a single neurotoxin injection into the left peroneus muscle complex, the muscular weakness results in a reduction in digit abduction. The varying degrees of digit abduction are scored on a 5-point scale: 0=normal to 4=maximal reduction in digit abduction and leg extension (Broide RS, Rubino J, Nicholson GS, et al. The rat Digit Abduction Score (DAS) assay: A physiological model for assessing botulinum neurotoxin-induced skeletal muscle paralysis. Toxicon 2013;71:18-24). DAS values were measured for the first five consecutive days after toxin injection and after this at intervals of two to three days until complete disappearance of the effect of modified BoNT/A (SEQ ID NO: 4 converted into a di-chain form) on the toe spreading reflex for lower doses and until recovery to DAS2 for doses resulting in DAS4. Transient BoNT-induced dose-dependent effects on body weight gain are considered evidence of a generalised toxin effect (Torii Y, Goto Y, Nakahira S, et al. Comparison of Systemic Toxicity between Botulinum Toxin Subtypes A1 and A2 in Mice and Rats. Basic Clin. Pharmacol. Toxicol. 2015;116:524-528.). At each evaluation time point rats were consequently weighed and side effects were noted. Dosing solutions of BoNT were masked (assigned random letters) before injection and until the end of the study. Potency was determined as the dose required to induce 50% of the effect ($ED_{50}$: dose leading to a DAS value of 2). To determine $ED_{50}$ and the 95% confidence intervals (CIs), doses ranging between 2.5 and 750 pg/kg were tested. Higher doses of 1, 1.5, 2, 2.4, 3, 4 and 5 ng/kg were also administered to assess possible side effects.

To evaluate the duration of action of modified BoNT/A (SEQ ID NO: 4 converted into a di-chain form) and compare it to the duration of action of unmodified BoNT/A (SEQ ID NO: 2 converted into a di-chain form), the median time necessary to return to a DAS2 reading of 2 was evaluated for the highest tolerated dose (no impact on body weight evolution compared to untreated rats) for both toxins in two independent, direct head-to-head studies.

Rat Single Dose Studies

Rats received a single intramuscular (i.m.) injection of modified BoNT/A (SEQ ID NO: 4 converted into a di-chain form) at doses of 0, 0.1, 1 and 3 ng/kg administered into the right gastrocnemius muscle. Control animals received SEQ ID NO: 4 diluent in the right gastrocnemius. Animals were euthanised 7 days after treatment (ten males and ten females per group) or after a 13 or 26-week observation period (five males and five females per dose). Irwin test observations, for assessment of central nervous system function, were performed pretest (Day -1), on Day 8 and during Weeks 13 and 27. Other clinical (adverse) signs assessed for were limping, small toxin injected muscle size, and soft distended abdomen.

Monkey Studies

Monkeys received single i.m. doses of 0, 0.1, 0.25 and 0.75 ng/kg modified BoNT/A (SEQ ID NO: 4 converted into a di-chain form) administered into the right gastrocnemius muscle. Animals were euthanised 7 days after treatment (three males and three females per group) or after a 13 or 26-week observation period (two males and two females per dose). Cardiovascular examinations, including haemodynamic, electrocardiogram and respiratory parameters, were performed by external telemetry pretest, on Days 8 and 15.

Preliminary Enhanced EFD in Pregnant Rat

The objective of the study was to provide initial information on the effects of modified BoNT/A (SEQ ID NO: 4 converted into a di-chain form) on embryonic and foetal development of the rat when administered by the i.m. route throughout the period of organogenesis. Modified BoNT/A (SEQ ID NO: 4 converted into a di-chain form) was administered by daily i.m. injection (gastrocnemius) at dose levels of 0.02, 0.05 and 0.1 ng/kg/day to groups of nine mated female Sprague-Dawley rats from days 6 (G6) to 17 (G17) of gestation, inclusive. Clinical condition, body weight and food consumption were monitored throughout the study. The females were submitted to a caesarean examination on G21 and litter parameters were recorded. At necropsy, the females were examined macroscopically, the gravid uteri were weighed and for those who presented a small injected gastrocnemius muscle, this muscle and the contralateral muscle were weighed. All foetuses were weighed. The foetuses were then examined for external and visceral abnormalities and sexed. The heads of approximately half of the foetuses were fixed for internal examination by serial sectioning. The eviscerated carcasses of all fetuses were processed for skeletal examination.

Preliminary Extended EFD in Pregnant Rabbit

The objective of the study was to provide initial information on the effects of modified BoNT/A (SEQ ID NO: 4 converted into a di-chain form) on embryonic and foetal development of the rabbit when administered by the i.m. route throughout the period of organogenesis. Modified BoNT/A (SEQ ID NO: 4 converted into a di-chain form) was administered by daily i.m. injection (gastrocnemius) at dose levels of 0.002, 0.005 and 0.01 ng/kg/day to groups of nine mated female New Zealand White rabbits from days 6 (G6) to 19 (G19) of gestation, inclusive. Clinical condition, body weight and food consumption were monitored throughout the study. The females were submitted to a caesarean examination on G29 and litter parameters were recorded. At necropsy, the females were examined macroscopically, the gravid uteri were weighed and for those who presented a small injected gastrocnemius muscle, this muscle and the contralateral muscle were weighed. All foetuses were weighed. The foetuses were then examined for external and visceral abnormalities and sexed. The heads of approximately half of the foetuses were fixed for internal examination by serial sectioning.

**Results**

By carrying out the studies as indicated above, the following pharmacological data (indicated in Table 9 below) were obtained for a number of different species administered the modified BoNT/A.

Table 9. Pre-clinical results.

| Animal | Study Type | Results |
|---|---|---|
| Mouse | LD50 IP | 0.422 ng/kg |
| Rat | DAS | ED50 0.013 ng/kg<br>DAS4 0.125 pg/kg |
| | CMAP Single Dose Distant Spread | 0.002 ng/kg: No spread<br>0.3 ng/kg: -25%<br>0.8 ng/kg: -56% |
| | Single Dose | Estimated NOAEL 1.5 ng/kg<br>Estimated Lethal 3 ng/kg |
| Monkey | Single Dose | Estimated NOAEL 0.125 ng/kg<br>Lethal 0.375 ng/kg |
| Rat (Pregnant Female) | pEFD | Maternal NOAEL and fetal NOEL 0.1 ng/kg/day |
| Rabbit (Pregnant Female) | pEFD | Maternal NOAEL 0.005 ng/kg/day<br>Fetal NOEL 0.01 ng/kg/day |

Additionally, modified BoNT/A (SEQ ID NO: 4 converted into a di-chain form) was tested in a rat DAS assay to determine the duration of action when compared to Dysport®. Results are presented in Table 10 below:

Table 10. Duration of action.

| | Dysport®<br>3 U/rat<br>15 U/kg | Modified BoNT/A<br>150 pg/rat<br>0.750 ng/kg |
|---|---|---|
| Duration of Action (median days) | 21.9 | 46.4 |

These data show that the modified BoNT/A has a duration of action that is more than double that of Dysport®.

**EXAMPLE 7**

**Determination of a Unit Dose of Modified BoNT/A (SEQ ID NO: 4 converted into a di-chain form) for Treating Cervical Dystonia**

In view of the pre-clinical pharmacology data obtained in Example 6 above, a suitable unit dose range (UD) for administration of modified BoNT/A in humans has been determined. The studies showed that modified BoNT/A provides a longer duration of action than unmodified BoNT/A while at the same time exhibiting an improved safety profile. This improved safety profile may be expressed by the high Safety Ratio described herein for the modified BoNT/A.

As modified BoNT/A shares the same mechanism of action as Dysport® (albeit with an increased Safety Ratio due to its modified properties), the lowest dose of modified BoNT/A for treating subjects has been positioned for context relative to the labelled doses of Dysport® in that same muscle group:

- In the Digit Abduction Score rat model, the $ED_{50}$ of modified BoNT/A is 13 pg/kg, and is more than 100-fold lower than the estimated no-observed-adverse-effect-level (NOAEL) of 1500 pg/kg in the same animal species. In the same rat model, the $ED_{50}$ of Dysport® is 0.5 U/kg. Based on these animal data, a dose of 2.6 ng of modified BoNT/A would estimate to a dose of 100 U Dysport®.
- The intraperitoneal mouse $LD_{50}$ was established at 8.44 pg. Under these conditions, a dose of 0.84 ng of modified BoNT/A corresponds to a dose of 100 U Dysport®.

The calculated lowest dose is thus 500 pg (0.5 ng). To provide some context and using the intraperitoneal mouse $LD_{50}$ data above, 0.5 ng modified BoNT/A equates to approximately 60 U Dysport®, and would thus be active when administered intramuscularly for treatment of cervical dystonia.

The estimated NOAEL of 1.5 ng/kg of modified BoNT/A in rats corresponds to a 90 ng dose for a human of 60 kg body weight. In monkeys, the more sensitive of the two nonclinical species tested, the estimated NOAEL of 0.125 ng/kg of modified BoNT/A corresponds to a 7.5 ng dose for a human of 60 kg body weight.

The upper limit of the unit dose is thus determined to be 7,500 pg (7.5 ng) as this remains below the rat NOAEL translated in human dose.

Thus, a suitable unit dose for treatment of cervical dystonia using modified BoNT/A has been determined to be 500-7,500 pg. Based on the pre-clinical data obtained, this is ~59-889 Units of modified BoNT/A (and also corresponds to ~59-889 Units of Dysport®) based on the calculated median lethal intraperitoneal dose ($LD_{50}$) in mice as determined using the mouse intraperitoneal Lethal Dose Assay.

In view of the improved safety profile when compared to Dysport® as determined by the pre-clinical data of Example 6, total dosages (in units) administered in cervical dystonia are expected to be almost 9x greater than that for Dysport®. The maximum total dose of Dysport® for treatment of cervical dystonia is 1,000 Units (see Figure 1).

Advantageously, more modified BoNT/A can be injected and/or can be injected at a greater number of neck muscles/sites in the treatment of cervical dystonia before reaching the maximum dose. This is a significant and advantageous finding leading to improved treatment of cervical dystonia while providing clinicians with a greater range of treatment options.

**EXAMPLE 8**

**Dosage Regimen for Treating Cervical Dystonia**

Modified BoNT/A (e.g. SEQ ID NO:4 converted into a di-chain form) is provided as a lyophilised powder in 2mL clear glass vials containing 15 ng of modified BoNT/A per vial. The lyophilised powder is reconstituted with a mixture of sterile sodium chloride 0.9% v/w preservative free solution and diluent (formulation buffer containing only the excipients of modified BoNT/A). After reconstitution, the solution is further diluted as necessary.

The unit dose (UD) is 500-7,500 pg (~59-889 Units).

Cervical dystonia is treated by intramuscular injection according to the following dosage regimen (Table 11):

Table 11. Dosage regimen.

| Neck Muscle | Dosage (Unit Dose) |
|---|---|
| Sternocleidomastoid | 1 x UD |
| Splenius capitis | 1 x UD |
| Splenius cervicis | 1 x UD |
| Trapezius | 1 x UD |

(continued)

| Neck Muscle | Dosage (Unit Dose) |
|---|---|
| Levator scapulae | 1 x UD |
| Scalenus medius | 1 x UD |
| Scalenus anterior | 1 x UD |
| Semispinalis capitis | 1 x UD |
| Longissimus | 1 x UD |

The administration may be unilateral or bilateral as required based on the specific presentation.

A maximum total dosage administered is 10x UD (e.g. in some cases 2x UD are administered to one or more of the neck muscles indicated). This corresponds to 75,000 pg/~8,890 Units. This is almost 9x greater than the maximum total dosage of Dysport® that can be administered during treatment of cervical dystonia without approaching toxic limits (a concern with conventional treatment regimens). Thus, the clinician is able to tailor treatment to the patient with the knowledge that 10x UD can be administered without any concern of toxicity, thereby allowing the treatment of additional neck muscles of the subject and/or ensuring each neck muscle receives a pharmaceutically effective dose.

## EXAMPLE 9

### Pre-Clinical Testing of Modified BoNT/A (BoNT/AB Chimera [SEQ ID NO: 14 converted into a di-chain form])

BoNT/AB chimera SEQ ID NO: 14 (converted into a di-chain form) was tested in a mouse $LD_{50}$ assay yielding a result of 1.202 ng/kg. 1 Unit of SEQ ID NO: 14 (converted into a di-chain form) therefore corresponds to 24.04 pg in this assay.

Additionally, said BoNT/AB chimera was tested in a rat DAS assay to determine the duration of action (as per Example 6) when compared to Dysport®. Results are presented in Table 12 below:

Table 12. Duration of action.

| | Dysport® 3 U/rat 15 U/kg | BoNT/AB 300 pg/rat 1.5 ng/kg |
|---|---|---|
| Duration of Action (median days) | 21.9 | 47.7 |

In conclusion, the duration of action of BoNT/AB was much higher than Dysport® and similar to that of SEQ ID NO: 4 (converted into a di-chain form). Thus, it is expected that the unit doses and dosage regimen for SEQ ID NO: 4 (converted into a di-chain form) could similarly be applied to BoNT/AB to provide an improved treatment for cervical dystonia.

## EXAMPLE 10

### Determination of a Unit Dose of Modified BoNT/A (SEQ ID NO: 14 converted into a di-chain form) for Treating Cervical Dystonia

In view of pre-clinical pharmacology data, a suitable unit dose range (UD) for administration of modified BoNT/A in humans has been determined.

A DAS $ED_{50}$ of 13 pg/kg was calculated for SEQ ID NO: 14 (converted into a di-chain form). $ED_{50}$ is considered as a minimal pharmacologically active dose, which is approximately 300-fold lower than the no observed adverse effect level (NOAEL) of 4 ng/kg in the same animal species. An $ED_{50}$ of 13 pg/kg of SEQ ID NO: 14 (converted into a di-chain form) in rats corresponds to a 0.8 ng dose for a human of 60 kg body weight.

Thus, the lower limit of a unit dose of 1,000 pg was selected. An upper limit of the unit dose of 16,000 pg was selected, which is lower than the NOAEL of 4 ng/kg from both nonclinical safety species (rat and monkey) converted into human dose for 60 kg body weight. Thus, a unit dose was determined to be 1,000 pg to 16,000 pg (~42 Units to ~666 Units).

In view of the improved safety profile the maximum total dose for the treatment of cervical dystonia was set at 160,000 pg (~7,070 Units), which is derived from the NOAEL of 4 ng/kg from both nonclinical safety species (rat and monkey) converted into human dose for 60 kg body weight.

In view of the improved safety profile when compared to Dysport® as determined by the pre-clinical data of Example 9, total dosages (in units) administered in cervical dystonia are expected to be almost 7x greater than that for Dysport®. The maximum total dose of Dysport® for treatment of cervical dystonia is 1,000 Units (see Figure 1).

Advantageously, more modified BoNT/A (SEQ ID NO: 14 converted into a di-chain form) can be injected and/or can be injected at a greater number of neck muscles/sites in the treatment of cervical dystonia before reaching the maximum dose. This is a significant and advantageous finding leading to improved treatment of cervical dystonia while providing clinicians with a greater range of treatment options.

### EXAMPLE 11

### Dosage Regimen for Treating Cervical Dystonia Using a Modified BoNT/A (SEQ ID NO: 14 converted into a di-chain form)

Modified BoNT/A (e.g. SEQ ID NO: 14 converted into a di-chain form) is provided as a lyophilised powder in a vial containing 36 ng of modified BoNT/A per vial. The lyophilised powder is reconstituted.

The unit dose (UD) is 1,000-16,000 pg (~42-666 Units [measured by mouse $LD_{50}$]).

Cervical dystonia is treated by intramuscular injection according to the following dosage regimen (Table 13):

Table 13. Dosage regimen.

| Neck Muscle | Dosage (Unit Dose) |
|---|---|
| Sternocleidomastoid | 1 x UD |
| Splenius capitis | 1 x UD |
| Splenius cervicis | 1 x UD |
| Trapezius | 1 x UD |
| Levator scapulae | 1 x UD |
| Scalenus medius | 1 x UD |
| Scalenus anterior | 1 x UD |
| Semispinalis capitis | 1 x UD |
| Longissimus | 1 x UD |

The administration may be unilateral or bilateral as required based on the specific presentation.

A maximum total dosage administered is 10x UD (e.g. in some cases 2x UD are administered to one or more of the neck muscles indicated). This corresponds to 160,000 pg/~6,660 Units. This is almost 7x greater than the maximum total dosage of Dysport® that can be administered during treatment of cervical dystonia without approaching toxic limits (a concern with conventional treatment regimens). Thus, the clinician is able to tailor treatment to the patient with the knowledge that 10x UD can be administered without any concern of toxicity, thereby allowing the treatment of additional neck muscles of the subject and/or ensuring each neck muscle receives a pharmaceutically effective dose.

### EXAMPLE 12

### Treatment of a Patient with Cervical Dystonia (Laterocollis)

Jane, aged 65, is diagnosed by her GP with cervical dystonia. The specific presentation is as laterocollis. A single unit dose (3,000 pg) of modified BoNT/A (SEQ ID NO: 4 converted into a di-chain form) is ipsilaterally administered to Jane's levator scapulae muscle and a single unit dose is also ipsilaterally administered to Jane's sternocleidomastoid muscle (resulting in a total dose at the treatment session of 6,000 pg). The laterocollis is alleviated and, owing to the long duration of the modified BoNT/A, Jane does not require further treatment for greater than 9 months. Thus, Jane receives less frequent injections (e.g. per year) when compared to an equivalent subject administered an unmodified BoNT/A. Additionally, Jane does not exhibit any side-effects owing to the improved safety profile of the modified BoNT/A.

### EXAMPLE 13

**Treatment of a Patient with Cervical Dystonia (Retrocollis)**

Brian, aged 48, is diagnosed by his GP with cervical dystonia. The specific presentation is as retrocollis. Modified BoNT/A (SEQ ID NO: 14 converted into a di-chain form) is administered bilaterally to each of the following of Brian's muscles:

- 1x unit dose (UD) of 10,000 pg to each levator scapulae;
- 1x UD of 10,000 pg to each trapezius;
- 1x UD of 10,000 pg to each longissimus;
- 1x UD of 10,000 pg to each splenius capitis; and
- 1x UD of 10,000 pg to each splenius cervicis.

The total dose administered is 10x UDs (100,000 pg), which is well-within the upper limit of 160,000 pg and is possible given the greater safety profile of the modified BoNT/A when compared to an unmodified BoNT/A. The retrocollis is alleviated and, owing to the long duration of the modified BoNT/A, Brian does not require further treatment for 12 months. Thus, Brian receives less frequent injections when compared to an equivalent subject administered an unmodified BoNT/A.

**EXAMPLE 14**

**Safety & Efficacy of Modified BoNT/A (SEQ ID NO: 14 converted into a di-chain form) in Humans**

SEQ ID NO: 14 (converted into a di-chain form) was administered to human subjects by way of a single unit dose of modified BoNT/A. 5 cohorts were administered different (increasing) amounts of modified BoNT/A (SEQ ID NO: 14 converted into a di-chain form). Cohort 1 was administered 2x 1,000 pg unit doses of modified BoNT/A (i.e. 2,000 pg maximum), while cohort 5 was administered 2x 16,000 pg unit doses of modified BoNT/A (i.e. 32,000 pg maximum).
Results showed that all unit doses of modified BoNT/A tested, (i.e. up to 16,000 pg unit doses), were effective at muscle paralysis, safely tolerated, and no adverse effects were observed, despite the exceptionally high dosage per muscle. This shows that the modified BoNT/A does not diffuse away from the injection site and highlights the exceptional safety profile of modified BoNT/A (SEQ ID NO: 14 converted into a di-chain form).

**EXAMPLE 15**

**Treatment of a Patient with Cervical Dystonia**

Sally 64 is diagnosed by her GP with cervical dystonia. She is treated by way of a unit dose (UD) of 1000 pg of SEQ ID NO: 14 (converted into a di-chain form) administered as follows:

| Neck Muscle | Dosage (Unit Dose) |
|---|---|
| Right Levator scapulae | 1 x UD |
| Left Levator scapulae | 1 x UD |
| Right Trapezius | 1 x UD |
| Left Trapezius | 1 x UD |
| Right Sternocleidomastoid | 1 x UD |
| Left Sternocleidomastoid | 1 x UD |
| Right Splenius capitis | 1 x UD |
| Left Splenius capitis | 1 x UD |
| Scalenus medius | 1 x UD |
| Scalenus anterior | 1 x UD |
| Right Semispinalis capitis | 1 x UD |
| Left Semispinalis capitis | 1 x UD |
| Right Longissimus capitis | 1 x UD |

(continued)

| Neck Muscle | Dosage (Unit Dose) |
|---|---|
| Left Longissimus capitis | 1 x UD |

She receives a total dose of 14,000 pg of SEQ ID NO: 14 (converted into a di-chain form). The treatment is successful and her symptoms are alleviated. She does not require treatment for greater than 9 months.

## EXAMPLE 16

### Treatment of a Patient with Cervical Dystonia

Francesco 43 is diagnosed by his GP with cervical dystonia. He is treated by way of a unit dose (UD) of 2000 pg of SEQ ID NO: 14 (converted into a di-chain form) administered as follows:

| Neck Muscle | Dosage (Unit Dose) |
|---|---|
| Right Levator scapulae | 1 x UD |
| Left Levator scapulae | 1 x UD |
| Right Trapezius | 1 x UD |
| Left Trapezius | 1 x UD |
| Right Sternocleidomastoid | 1 x UD |
| Left Sternocleidomastoid | 1 x UD |
| Right Splenius capitis | 1 x UD |
| Left Splenius capitis | 1 x UD |
| Scalenus medius | 1 x UD |
| Scalenus anterior | 1 x UD |
| Right Semispinalis capitis | 1 x UD |
| Left Semispinalis capitis | 1 x UD |
| Right Longissimus capitis | 1 x UD |
| Left Longissimus capitis | 1 x UD |

He receives a total dose of 28,000 pg of SEQ ID NO: 14 (converted into a di-chain form). The treatment is successful and his symptoms are alleviated. She does not require treatment for greater than 10 months.

## Claims

1. A modified botulinum neurotoxin A (BoNT/A) for use in treating cervical dystonia, wherein the modified BoNT/A is administered by intramuscular injection to an affected neck muscle of a subject,

   wherein the modified BoNT/A is administered by way of a unit dose of 750 pg to 17,000 pg of modified BoNT/A, wherein at least a single unit dose is administered to the affected neck muscle, wherein the total dose administered during the treatment is up to 170,000 pg of modified BoNT/A, and wherein the modified BoNT/A comprises a BoNT/A light-chain and translocation domain, and a BoNT/B receptor binding domain ($H_C$ domain).

2. The modified BoNT/A for use according to claim 1, wherein:

   (a) the total dose administered is 5,250 pg to 170,000 pg, preferably 7,000 pg to 160,000 pg;
   (b) the unit dose is 1,000 pg to 16,000 pg; and/or
   (c) the total dose administered during the treatment is up to 160,000 pg of modified BoNT/A.

3. The modified BoNT/A for use according to claim 1 or 2, wherein the modified BoNT/A is administered to an affected neck muscle at a single injection site, or at two or more injection sites (e.g. two injection sites).

4. The modified BoNT/A for use according to any one of the preceding claims, wherein cervical dystonia is treated for a longer duration than that treated by an unmodified BoNT/A (e.g. SEQ ID NO: 2 [such as a di-chain form of SEQ ID NO: 2]).

5. The modified BoNT/A for use according to any one of the preceding claims, wherein the modified BoNT/A has a Safety Ratio of greater than 7, wherein the Safety Ratio is calculated as: dose of toxin required for -10% bodyweight change measured as pg/mouse divided by DAS $ED_{50}$ measured as pg/mouse, wherein $ED_{50}$ = dose required to produce a DAS score of 2.

6. The modified BoNT/A for use according to any one of the preceding claims, wherein:
the affected neck muscle is selected from: the sternocleidomastoid, the splenius capitis, the splenius cervicis, the scalene complex (e.g. the scalenus anterior and/or the scalenus medius), the trapezius (e.g. the upper trapezius), the levator scapulae, the semispinalis capitis, and the longissimus (e.g. longissimus capitis and/or longissimus cervicis).

7. The modified BoNT/A for use according to any one of the preceding claims, wherein the modified BoNT/A is administered by intramuscular injection to a plurality of affected neck muscles of the subject, and wherein at least a single unit dose is administered to each affected neck muscle,
preferably wherein:
the plurality of affected neck muscles are selected from: the sternocleidomastoid, the splenius capitis, the splenius cervicis, the scalene complex (e.g. the scalenus anterior and/or the scalenus medius), the trapezius (e.g. the upper trapezius), the levator scapulae, the semispinalis capitis, and the longissimus (e.g. longissimus capitis and/or longissimus cervicis).

8. The modified BoNT/A for use according to any one of the preceding claims, wherein:

(a) the modified BoNT/A is administered by way of a unit dose per injection site at an affected neck muscle, or wherein the modified BoNT/A is administered by way of less than a unit dose per injection site;
(b) a single unit dose is administered at a plurality of injection sites at an affected neck muscle and/or two or more unit doses are administered at a plurality of injection sites at an affected neck muscle; and/or
(c) a single unit dose is administered at an affected neck muscle (e.g. a single unit dose is administered at each affected neck muscle).

9. The modified BoNT/A for use according to any one of the preceding claims, wherein the subject is a human subject.

10. A unit dosage form of modified BoNT/A for use in treating cervical dystonia, the unit dosage form comprising:

(a) 750 pg to 17,000 pg of modified BoNT/A; and
(b) optionally a pharmaceutically acceptable carrier, excipient, adjuvant, and/or salt,

wherein the modified BoNT/A comprises a BoNT/A light-chain and translocation domain, and a BoNT/B receptor binding domain ($H_C$ domain).

11. The modified BoNT/A for use according to any one of claims 1-9, or the unit dosage form for use according to claim 10, wherein:

(a) the modified BoNT/A comprises a combination of two substitution mutations which are E1191M and S1199Y; and/or
(b) the modified BoNT/A comprises a polypeptide sequence having at least 70% sequence identity to SEQ ID NO: 14.

12. The modified BoNT/A for use according to any one of claims 1-9 or 11, or the unit dosage form for use according to any one of claims 10 or 11, wherein:
the modified BoNT/A is a di-chain modified BoNT/A in which the light-chain (L-chain) and heavy-chain (H-chain) are joined together by a di-sulphide bond obtainable by a method comprising contacting a single-chain modified BoNT/A comprising a polypeptide sequence having at least 70%, 80%, 90%, 95%, or 99.9% sequence identity to SEQ ID NO:

14 with a protease that hydrolyses a peptide bond in the activation loop thereof, thereby converting the single-chain modified BoNT/A into the corresponding di-chain modified BoNT/A.

13. The modified BoNT/A for use according to any one of claims 1-9 or 11-12, or the unit dosage form for use according to any one of claims 10-12, wherein the C-terminal amino acid residue of the translocation domain corresponds to the first amino acid residue of the $3_{10}$ helix separating the $LH_N$ and $H_C$ domains of BoNT/A, and the N-terminal amino residue of the $H_C$ domain corresponds to the second amino acid residue of the $3_{10}$ helix separating the $LH_N$ and $H_C$ domains in BoNT/B.

## Patentansprüche

1. Ein modifiziertes Botulinumneurotoxin A (BoNT/A) zur Verwendung bei der Behandlung von zervikaler Dystonie, wobei das modifizierte BoNT/A durch intramuskuläre Injektion in einen betroffenen Halsmuskel eines Individuums verabreicht wird,

   wobei das modifizierte BoNT/A mittels einer Einzeldosis von 750 pg bis 17 000 pg des modifizierten BoNT/A verabreicht wird,
   wobei mindestens eine einzelne Einzeldosis in den betroffenen Halsmuskel verabreicht wird,
   wobei die während der Behandlung verabreichte Gesamtdosis bis zu 170 000 pg des modifizierten BoNT/A beträgt, und
   wobei das modifizierte BoNT/A eine BoNT/A-Leichtketten- und Translokationsdomäne und eine BoNT/B-Rezeptorbindungsdomäne ($H_C$-Domäne) umfasst.

2. Modifiziertes BoNT/A zur Verwendung nach Anspruch 1, wobei:

   (a) die verabreichte Gesamtdosis 5250 pg bis 170 000 pg, bevorzugt 7000 pg bis 160 000 pg, beträgt;
   (b) die Einzeldosis 1000 pg bis 16 000 pg beträgt;
   und/oder
   (c) die während der Behandlung verabreichte Gesamtdosis bis zu 160 000 pg des modifizierten BoNT/A beträgt.

3. Modifiziertes BoNT/A zur Verwendung nach Anspruch 1 oder 2, wobei das modifizierte BoNT/A an einem einzelnen Injektionsort oder an zwei oder mehr Injektionsorten (z. B. zwei Injektionsorten) in einen betroffenen Halsmuskel verabreicht wird.

4. Modifiziertes BoNT/A zur Verwendung nach einem der vorhergehenden Ansprüche, wobei die zervikale Dystonie über eine längere Zeitdauer behandelt wird als bei Behandlung mit einem nicht modifizierten BoNT/A (z. B. SEQ ID NO: 2 [wie etwa eine Zweikettenform von SEQ ID NO: 2]).

5. Modifiziertes BoNT/A zur Verwendung nach einem der vorhergehenden Ansprüche, wobei das modifizierte BoNT/A ein Sicherheitsverhältnis von mehr als 7 aufweist, wobei das Sicherheitsverhältnis berechnet wird als: Dosis des Toxins, die für eine Änderung des Körpergewichts von -10% erforderlich ist, gemessen als pg/Maus, dividiert durch DAS-$ED_{50}$, gemessen als pg/Maus, wobei $ED_{50}$ = die zum Produzieren eines DAS-Score von 2 erforderliche Dosis.

6. Modifiziertes BoNT/A zur Verwendung nach einem der vorhergehenden Ansprüche, wobei:
   der betroffene Halsmuskel aus den Folgenden ausgewählt ist: M. sternocleidomastoideus, M. splenius capitis, M. splenius cervicis, Scalenusgruppe (z. B. M. scalenus anterior und/oder M. scalenus medius), M. trapezius (z. B. oberer M. trapezius), M. levator scapulae, M. semispinalis capitis und M. longissimus (z. B. M. longissimus capitis und/oder M. longissimus cervicis).

7. Modifiziertes BoNT/A zur Verwendung nach einem der vorhergehenden Ansprüche, wobei das modifizierte BoNT/A durch intramuskuläre Injektion in eine Vielzahl von betroffenen Halsmuskeln des Individuums verabreicht wird und wobei mindestens eine einzelne Einzeldosis in jeden betroffenen Halsmuskel injiziert wird,
   wobei bevorzugt:
   die Vielzahl von betroffenen Halsmuskeln aus den Folgenden ausgewählt sind: M. sternocleidomastoideus, M. splenius capitis, M. splenius cervicis, Scalenusgruppe (z. B. M. scalenus anterior und/oder M. scalenus medius), M. trapezius (z. B. oberer M. trapezius), M. levator scapulae, M. semispinalis capitis und M. longissimus (z. B. M. longissimus capitis und/oder M. longissimus cervicis).

8. Modifiziertes BoNT/A zur Verwendung nach einem der vorhergehenden Ansprüche, wobei:

(a) das modifizierte BoNT/A mittels einer Einzeldosis pro Injektionsort an einem betroffenen Halsmuskel verabreicht wird oder wobei das modifizierte BoNT/A mittels weniger als einer Einzeldosis pro Injektionsort verabreicht wird;
(b) eine einzelne Einzeldosis an einer Vielzahl von Injektionsorten an einem betroffenen Halsmuskel verabreicht wird und/oder zwei oder mehr Einzeldosen an einer Vielzahl von Injektionsorten an einem betroffenen Halsmuskel verabreicht werden; und/oder
(c) eine einzelne Einzeldosis an einem betroffenen Halsmuskel verabreicht wird (z. B. eine einzelne Einzeldosis an jedem betroffenen Halsmuskel verabreicht wird).

9. Modifiziertes BoNT/A zur Verwendung nach einem der vorhergehenden Ansprüche, wobei das Individuum ein menschliches Individuum ist.

10. Eine Einzelarzneiform von modifiziertem BoNT/A zur Verwendung bei der Behandlung von zervikaler Dystonie, wobei die Einzelarzneiform Folgendes umfasst:

(a) 750 pg bis 17 000 pg des modifizierten BoNT/A;
und
(b) optional einen pharmazeutisch akzeptablen Träger, Hilfsstoff, Adjuvans und/oder Salz, wobei das modifizierte BoNT/A eine BoNT/A-Leichtketten- und Translokationsdomäne und eine BoNT/B-Rezeptorbindungsdomäne ($H_C$-Domäne) umfasst.

11. Modifiziertes BoNT/A zur Verwendung nach einem der Ansprüche 1-9 oder Einzelarzneiform zur Verwendung nach Anspruch 10, wobei:

(a) das modifizierte BoNT/A eine Kombination von zwei Substitutionsmutationen umfasst, die E1191M und S1199Y sind; und/oder
(b) das modifizierte BoNT/A eine Polypeptidsequenz umfasst, die eine mindestens 70%ige Sequenzidentität mit SEQ ID NO: 14 aufweist.

12. Modifiziertes BoNT/A zur Verwendung nach einem der Ansprüche 1-9 oder 11 oder Einzelarzneiform zur Verwendung nach einem der Ansprüche 10 oder 11, wobei:
das modifizierte BoNT/A ein modifiziertes Zweiketten-BoNT/A, in dem die leichte Kette (L-Kette) und die schwere Kette (H-Kette) durch eine Disulfidbindung miteinander verknüpft sind, die durch ein Verfahren erhalten werden kann, das Folgendes umfasst: In-Kontakt-Bringen eines modifizierten Einzelketten-BoNT/A, das eine Polypeptidsequenz umfasst, die eine mindestens 70%ige, 80%ige, 90%ige, 95%ige oder 99,9%ige Sequenzidentität mit SEQ ID NO: 14 aufweist, mit einer Protease, die eine Peptidbindung in dessen Aktivierungsschleife hydrolysiert, wodurch das modifizierte Einketten-BoNT/A in das entsprechende modifizierte Zweiketten-BoNT/A umgewandelt wird.

13. Modifiziertes BoNT/A zur Verwendung nach einem der Ansprüche 1-9 oder 11-12 oder Einzelarzneiform zur Verwendung nach einem der Ansprüche 10-12, wobei der C-terminale Aminosäurerest der Translokationsdomäne dem ersten Aminosäurerest der $3_{10}$-Helix entspricht, die die $LH_N$- und $H_C$-Domänen von BoNT/A trennt, und der N-terminale Aminosäurerest der $H_C$-Domäne dem zweiten Aminosäurerest der $3_{10}$-Helix entspricht, die die $LH_N$- und $H_C$-Domänen in BoNT/B trennt.

**Revendications**

1. Neurotoxine botulique A (BoNT/A) modifiée destinée à une utilisation dans le traitement de la dystonie cervicale, la BoNT/A modifiée étant administrée par injection intramusculaire dans un muscle cervical touché d'un sujet,

la BoNT/A modifiée étant administrée en une dose unitaire de 750 pg à 17 000 pg de BoNT/A modifiée,
au moins une dose unitaire individuelle étant administrée dans le muscle cervical touché,
la dose totale administrée durant le traitement étant de jusqu'à 170 000 pg de BoNT/A modifiée, et
la BoNT/A modifiée comprenant une chaîne légère de BoNT/A et un domaine de translocation, et un domaine de liaison au récepteur de BoNT/B (domaine $H_C$).

**2.** BoNT/A modifiée destinée à une utilisation selon la revendication 1,

(a) la dose totale administrée étant de 5 250 pg à 170 000 pg, préférablement de 7 000 pg à 160 000 pg ;
(b) la dose unitaire étant de 1 000 pg à 16 000 pg ;
et/ou
(c) la dose totale administrée durant le traitement étant de jusqu'à 160 000 pg de BoNT/A modifiée.

**3.** BoNT/A modifiée destinée à une utilisation selon la revendication 1 ou 2, la BoNT/A modifiée étant administrée dans un muscle cervical touché dans un seul site d'injection, ou dans deux sites d'injection ou plus (par exemple dans deux sites d'injection).

**4.** BoNT/A modifiée destinée à une utilisation selon l'une quelconque des revendications précédentes, la dystonie cervicale étant traitée pendant plus longtemps que celle traitée par une BoNT/A non modifiée (par exemple SÉQ. ID N° 2 [telle qu'une forme bicaténaire de SÉQ ID N° 2]).

**5.** BoNT/A modifiée destinée à une utilisation selon l'une quelconque des revendications précédentes, la BoNT/A modifiée ayant un rapport d'innocuité supérieur à 7, le rapport d'innocuité étant calculé ainsi : dose de toxine nécessaire pour produire un changement de poids corporel de -10 %, mesurée en pg/souris, divisée par la $DE_{50}$ établie en utilisant le score DAS et mesurée en pg/souris, avec $DE_{50}$ = dose requise pour produire un score DAS de 2.

**6.** BoNT/A modifiée destinée à une utilisation selon l'une quelconque des revendications précédentes,
le muscle cervical touché étant sélectionné parmi : le sterno-cléido-mastoïdien, le muscle splénius de la tête, le muscle splénius du cou, le complexe scalène (par exemple le muscle scalène antérieur et/ou le muscle scalène moyen), le muscle trapèze (par exemple le muscle trapèze supérieur), le muscle élévateur de la scapula, le muscle semi-épineux de la tête, et le muscle longissimus (par exemple le muscle longissimus de la tête et/ou le muscle longissimus du cou).

**7.** BoNT/A modifiée destinée à une utilisation selon l'une quelconque des revendications précédentes, la BoNT/A modifiée étant administrée par injection intramusculaire dans une pluralité de muscles cervicaux touchés du sujet, et au moins une dose unitaire individuelle étant administrée dans chaque muscle cervical touché,
préférablement :
la pluralité de muscles cervicaux touchés étant sélectionnée parmi : le sterno-cléido-mastoïdien, le muscle splénius de la tête, le muscle splénius du cou, le complexe scalène (par exemple le muscle scalène antérieur et/ou le muscle scalène moyen), le muscle trapèze (par exemple le muscle trapèze supérieur), le muscle élévateur de la scapula, le muscle semi-épineux de la tête et le muscle longissimus (par exemple le muscle longissimus de la tête et/ou le muscle longissimus du cou).

**8.** BoNT/A modifiée destinée à une utilisation selon l'une quelconque des revendications précédentes,

(a) la BoNT/A modifiée étant administrée en une dose unitaire par site d'injection dans un muscle cervical touché, ou la BoNT/A modifiée étant administrée en moins d'une dose unitaire par site d'injection ;
(b) une dose unitaire individuelle étant administrée dans une pluralité de sites d'injection dans un muscle cervical touché et/ou deux doses unitaires ou plus étant administrées dans une pluralité de sites d'injection dans un muscle cervical touché ; et/ou
(c) une dose unitaire individuelle étant administrée dans un muscle cervical touché (par exemple une dose unitaire individuelle étant administrée dans chaque muscle cervical touché).

**9.** BoNT/A modifiée destinée à une utilisation selon l'une quelconque des revendications précédentes, le sujet étant un sujet humain.

**10.** Forme galénique unitaire de BoNT/A modifiée destinée à une utilisation dans le traitement de la dystonie cervicale, la forme galénique unitaire comprenant :

(a) 750 pg à 17 000 pg de BoNT/A modifiée ; et
(b) optionnellement un support, un excipient, un adjuvant et/ou un sel pharmaceutiquement acceptable, la BoNT/A modifiée comprenant une chaîne légère de BoNT/A et un domaine de translocation, et un domaine de liaison au récepteur de BoNT/B (domaine HC).

11. BoNT/A modifiée destinée à une utilisation selon l'une quelconque des revendications 1 à 9, ou forme galénique unitaire destinée à une utilisation selon la revendication 10,

(a) la BoNT/A modifiée comprenant une combinaison de deux mutations de substitution qui sont E1191M et S1199Y ; et/ou
(b) la BoNT/A modifiée comprenant une séquence polypeptidique ayant une identité de séquence d'au moins 70 % avec la SÉQ. ID n° 14.

12. BoNT/A modifiée destinée à une utilisation selon l'une quelconque des revendications 1 à 9 ou 11, ou forme galénique unitaire destinée à une utilisation selon l'une quelconque des revendications 10 ou 11, la BoNT/A modifiée étant une BoNT/A modifiée bicaténaire dans laquelle la chaîne légère (chaîne L) et la chaîne lourde (chaîne H) sont reliées par un pont di-sulfure qui peut être obtenu par un procédé comprenant la mise en contact d'une BoNT/A modifiée monocaténaire comprenant une séquence polypeptidique ayant une identité de séquence d'au moins 70 %, 80 %, 90 %, 95 % ou 99,9 % avec la SÉQ. ID n° 14 avec une protéase qui hydrolyse une liaison peptidique dans la boucle d'activation de celle-ci, en convertissant ainsi la BoNT/A modifiée monocaténaire en la BoNT/A modifiée bicaténaire correspondante.

13. BoNT/A modifiée destinée à une utilisation selon l'une quelconque des revendications 1 à 9 ou 11 ou 12, ou forme galénique unitaire destinée à une utilisation selon l'une quelconque des revendications 10 à 12, le résidu acide aminé C-terminal du domaine de translocation correspondant au premier résidu acide aminé de l'hélice $3_{10}$ séparant les domaines $LH_N$ et $H_C$ de la BoNT/A, et le résidu aminé N-terminal du domaine $H_C$ correspondant au deuxième résidu acide aminé de l'hélice $3_{10}$ séparant les domaines $LH_N$ et $H_C$ dans la BoNT/B.

FIGURE 1

| Indication | Recommended Concentration | Recommended DYSPORT Dose |
|---|---|---|
| Cervical Dystonia, Adults | 50 Units/0.1 mL or 25 Units/0.1 mL | 500 Units to 1000 Units |

FIGURE 2

FIGURE 3

FIGURE 3 CONTINUED

FIGURE 4

| SXN/Batch | $t_{50}$ (mean min ± sem) |
|---|---|
| nBoNT/A1 | 52 ± 3 |
| Cat5v2(K1064H/N886K) | 49 ± 3 |
| Cat5v2(K1064H/N954K) | 48 ± 3 |
| Cat5v2(K1064H/N1025K) | 47 ± 3 |

FIGURE 5

FIGURE 6

FIGURE 7

FIGURE 8

SEQ ID NO: 14    SEQ ID NO: 15

FIGURE 9

FIGURE 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017191315 A
- WO 2015004461 A
- WO 2015004461 A1
- US 20070166332 A
- WO 2006027207 A
- WO 2006114308 A
- WO 2010120766 A
- US 20110318385 A
- WO 2013180799 A
- WO 2016154534 A

- WO 2014080206 A
- WO 2014079495 A
- EP 2677029 A2
- US 5223409 A, Ladner
- WO 9206204 A
- WO 2015166242 A
- WO 2017055274 A1
- EP 2524963 B1
- EP 2677029 B1
- US 10087432 B2

**Non-patent literature cited in the description**

- **JOST et al.** *J Neural Transm (Vienna)*, 2013, vol. 120 (3), 487-496
- **GERALD K**. Cell and Molecular Biology. John Wiley & Sons, Inc, 2002
- **HENDERSON et al.** The Clostridia: Molecular Biology and Pathogenesis. Academic press, 1997
- **UMLAND TC**. *Nat. Struct. Biol.*, 1997, vol. 4, 788-792
- **HERREROS J**. *Biochem. J.*, 2000, vol. 347, 199-204
- **HALPERN J**. *J. Biol. Chem.*, 1993, vol. 268 (15), 11188-11192
- **RUMMEL A**. *PNAS*, 2007, vol. 104, 359-364
- **LACEY DB**. *Nat. Struct. Biol.*, 1998, vol. 5, 898-902
- **KNAPP**. *Am. Cryst. Assoc. Abstract Papers*, 1998, vol. 25, 90
- **SWAMINATHAN ; ESWARAMOORTHY**. *Nat. Struct. Biol.*, 2000, vol. 7, 1751-1759
- **RUMMEL A**. *Mol. Microbiol.*, 2004, vol. 51 (3), 631-643
- **AOKI KR**. *Toxicon*, 2001, vol. 39, 1815-1820
- **JULIE D. THOMPSON et al.** CLUSTAL W: Improving the Sensitivity of Progressive Multiple Sequence Alignment Through Sequence Weighting. *Position-Specific Gap Penalties and Weight Matrix Choice*, 1994, 4673-4680
- **OSAMU GOTOH**. Significant Improvement in Accuracy of Multiple Protein. Sequence Alignments by Iterative Refinement as Assessed by Reference to Structural Alignments. *J. Mol. Biol.*, 1996, vol. 264 (4), 823-838

- **ERIC DEPIEREUX ; ERNEST FEYTMANS**. Match-Box: A Fundamentally New Algorithm for the Simultaneous Alignment of Several Protein Sequences. *CABIOS*, 1992, vol. 8 (5), 501-509
- **C. E. LAWRENCE et al.** Detecting Subtle Sequence Signals: A Gibbs Sampling Strategy for Multiple Alignment. *Science*, 1993, vol. 262 (5131), 208-214
- **IVO VAN WALLE et al.** Align-M - A New Algorithm for Multiple Alignment of Highly Divergent Sequences. *Bioinformatics*, 2004, vol. 20 (9), 1428-1435
- **ALTSCHUL et al.** *Bull. Math. Bio.*, 1986, vol. 48, 603-16
- **HENIKOFF ; HENIKOFF**. *Proc. Natl. Acad. Sci. USA*, 1992, vol. 89, 10915-19
- **ROBERTSON et al.** *J. Am. Chem. Soc.*, 1991, vol. 113, 2722
- **ELLMAN et al.** *Methods Enzymol.*, 1991, vol. 202, 301
- **CHUNG et al.** *Science*, 1993, vol. 259, 806-9
- **CHUNG et al.** *Proc. Natl. Acad. Sci. USA*, 1993, vol. 90, 10145-9
- **TURCATTI et al.** *J. Biol. Chem.*, 1996, vol. 271, 19991-8
- **KOIDE et al.** *Biochem.*, 1994, vol. 33, 7470-6
- **WYNN ; RICHARDS**. *Protein Sci.*, 1993, vol. 2, 395-403
- **CUNNINGHAM ; WELLS**. *Science*, 1989, vol. 244, 1081-5
- **DE VOS et al.** *Science*, 1992, vol. 255, 306-12
- **SMITH et al.** *J. Mol. Biol.*, 1992, vol. 224, 899-904
- **WLODAVER et al.** *FEBS Lett.*, 1992, vol. 309, 59-64

- **REIDHAAR-OLSON** ; **SAUER**. *Science*, 1988, vol. 241, 53-7
- **BOWIE** ; **SAUER**. *Proc. Natl. Acad. Sci. USA*, 1989, vol. 86, 2152-6
- **LOWMAN et al.** *Biochem.*, 1991, vol. 30, 10832-7
- **DERBYSHIRE et al.** *Gene*, 1986, vol. 46, 145
- **NER et al.** *DNA*, 1988, vol. 7, 127
- **SINGLETON et al.** DICTIONARY OF MICROBIOLOGY AND MOLECULAR BIOLOGY. John Wiley and Sons, 1994
- **HALE** ; **MARHAM**. THE HARPER COLLINS DICTIONARY OF BIOLOGY. Harper Perennial, 1991
- **AOKI et al.** *Eur. J. Neurol*, 1999, vol. 6 (4), S3-S10
- **BROIDE RS** ; **RUBINO J** ; **NICHOLSON GS et al.** The rat Digit Abduction Score (DAS) assay: A physiological model for assessing botulinum neurotoxin-induced skeletal muscle paralysis. *Toxicon*, 2013, vol. 71, 18-24
- **TORII Y** ; **GOTO Y** ; **NAKAHIRA S et al.** Comparison of Systemic Toxicity between Botulinum Toxin Subtypes A1 and A2 in Mice and Rats. *Basic Clin. Pharmacol. Toxicol.*, 2015, vol. 116, 524-528